# EUROPEAN PATENT APPLICATION

(11) **EP 4 431 489 A1**
(43) Date of publication of application: **18.09.2024**
(21) Application number: 22892797.6
(22) Date of filing: 09.11.2022
(51) Int. Cl.: C07C 229/12, A61K 9/16, A61K 31/7088, A61K 31/7105, A61K 31/711, A61K 31/713, A61K 47/18, A61K 48/00

(54) **CATIONIC LIPID**

(30) Priority: 10.11.2021 JP 2021183413
(71) Applicant: Takeda Pharmaceutical Company Limited, Osaka-shi, Osaka 541-0045 (JP)
(72) Inventor: MATSUMOTO Satoru, Fujisawa-shi, Kanagawa 251-0012 (JP); OMORI Yoshimasa, Fujisawa-shi, Kanagawa 251-0012 (JP); HOASHI Yasutaka, Fujisawa-shi, Kanagawa 251-0012 (JP)
(74) Representative: Jones, Nicholas Andrew
(86) International application number: PCT/JP2022/041651
(87) International publication number: WO 2023/085299

(57) **Abstract**

The present invention provides a technique capable of transferring an active ingredient, particularly, a nucleic acid, to a cell with excellent efficiency and/or to various cells and a cationic lipid for use in this technique, etc. The cationic lipid of the present invention is a compound represented by the following formula (I) or a salt thereof. In the formula (I), W represents -NR¹R² or -N⁺R¹¹R¹²R¹³ (Z⁻), R¹ and R² each independently represent H or an optionally substituted C₁₋₅ alkyl group, R¹¹, R¹² and R¹³ each independently represent an optionally substituted C₁₋₅ alkyl group, Z- represents an anion, X represents an optionally substituted C₂₋₆ alkylene group, R^{A} and R^{B} each independently represent an optionally substituted C₁₋₁₇ alkyl group, an optionally substituted C₃₋₁₇ alkenyl group, an optionally substituted C₁₅₋₁₇ alkadienyl group, -R³-C (O)O-R⁴ or - R³-OC(O)-R⁴, R^{C} represents -R³-C (O)O-R⁴ or -R³-OC(O)-R⁴, R³ represents an optionally substituted C₁₋₁₆ alkylene group, an optionally substituted C₄₋₁₆ alkenylene group or an optionally substituted C₇₋₁₆ alkadienylene group, and R⁴ represents H, an optionally substituted C₁₋₁₈ alkyl group, an optionally substituted C₃₋₁₈ alkenyl group or an optionally substituted C₁₅₋₁₈ alkadienyl group.

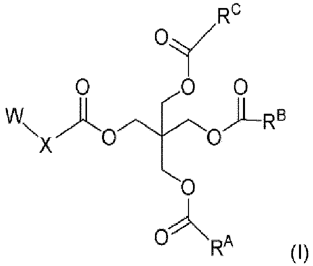

## Description

### Technical Field

The present invention relates to a cationic lipid capable of transferring a nucleic acid as an active ingredient to many types of cells, tissues or organs. The present invention further relates to a lipid particle containing the cationic lipid, and a composition containing the lipid particle and a nucleic acid.

### Background of Invention

In recent years, research and development have been actively made on nucleic acid medicaments containing a nucleic acid as an active ingredient. For example, many studies have been conducted on nucleic acid medicaments containing a nucleic acid such as siRNA, miRNA, miRNA mimic or antisense nucleic acid and having an effect of degrading or functionally suppressing target mRNA. Also, studies have been conducted on nucleic acid medicaments for the intracellular expression of a protein of interest, containing mRNA encoding the protein of interest and the like. In relation to such research and development, techniques for transferring a nucleic acid to cells, tissues or organs with high efficiency have been developed as drug delivery system (DDS) techniques.

Techniques of mixing a nucleic acid with a lipid to form a complex, followed by the cellular uptake of the nucleic acid via the complex have heretofore been known as the DDS techniques. Cationic lipids, hydrophilic polymer lipids, helper lipids, and the like have heretofore been known as lipids for use in the complex formation. For example, the following compounds described in the prior art documents are known as the cationic lipids.

Patent Literature 1 describes a compound represented by the following formula or a salt thereof, etc.

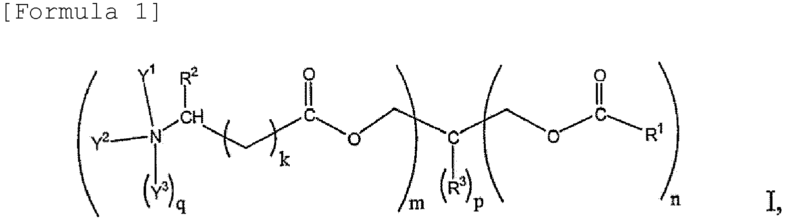

The formula is defined as follows: R¹ is each independently selected from the group consisting of optionally substituted C₈ to C₂₄ alkyl and optionally substituted C₈ to C₂₄ alkenyl; R² and R³ are each independently selected from the group consisting of hydrogen, optionally substituted C₁ to C₈ alkyl, optionally substituted arylalkyl, and the like; Y¹ and Y² are each independently selected from the group consisting of hydrogen, optionally substituted C₁ to C₆ alkyl, optionally substituted arylalkyl, and the like; Y³, if present, is each independently selected from the group consisting of hydrogen, optionally substituted C₁ to C₈ alkyl, optionally substituted arylalkyl, and the like; m is any integer of 1 to 4, n is any integer of 0 to 3, p is 0 or 1, and the total of m, n and p is 4; k is any integer of 1 to 5; q is 0 or 1.

Patent Literature 2 describes a compound represented by the following formula or a salt thereof, etc.

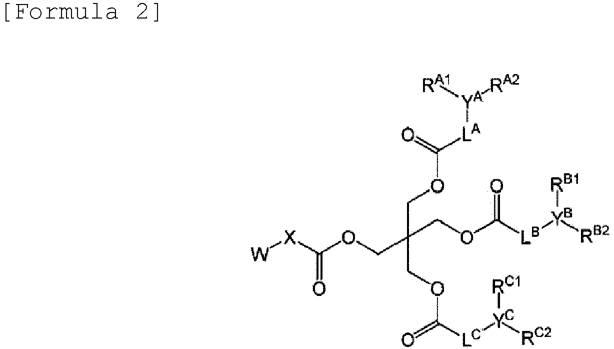

In the formula, W represents the formula -NR¹R² or the formula -N⁺R³R⁴R⁵(Z⁻), R¹ and R² each independently represent a C₁₋₄ alkyl group or a hydrogen atom, R³, R⁴ and R⁵ each independently represent a C₁₋₄ alkyl group, Z⁻ represents an anion, X represents an optionally substituted C₁₋₆ alkylene group, Y^{A}, Y^{B} and Y^{C} each independently represent an optionally substituted methine group, L^{A}, L^{B} and L^{C} each independently represent an optionally substituted methylene group or a bond, and R^{A1}, R^{A2}, R^{B1}, R^{B2}, R^{C1} and R^{C2} each independently represent an optionally substituted C₄₋₁₀ alkyl group.

Patent Literature 3 describes a compound represented by the following formula or a salt thereof, etc.

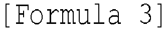

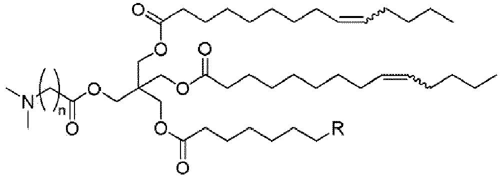

In the formula, n represents an integer of 2 to 5, R represents a linear C₁₋₅ alkyl group, a linear C₇₋₁₁ alkenyl group or a linear C₁₁ alkadienyl group, and the wavy lines each independently represent a cis or trans bond.

Patent Literature 4 describes a compound represented by the following formula or a salt thereof, etc.

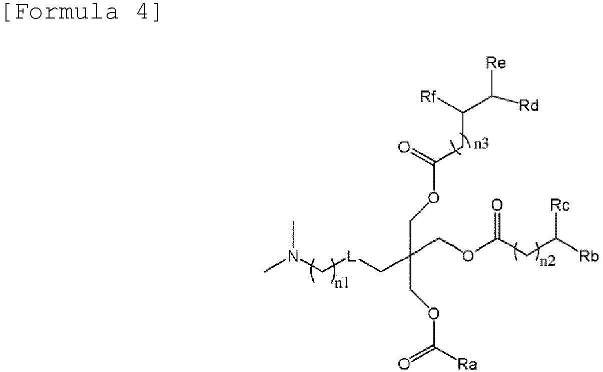

In the formula, n1 represents an integer of 2 to 6, n2 represents an integer of 0 to 2, n3 represents an integer of 0 to 2, L represents -C(O)O- or -NHC(O)O-, Ra represents a linear C₅₋₁₃ alkyl group, a linear C₁₃₋₁₇ alkenyl group or a linear C₁₇ alkadienyl group, Rb represents a linear C₂₋₉ alkyl group, Rc represents a hydrogen atom or a linear C₂₋₉ alkyl group, Rd represents a hydrogen atom or a linear C₂₋₉ alkyl group, Re represents a linear C₂₋₉ alkyl group, and Rf represents a linear C₂₋₉ alkyl group.

### Citation List

### Patent Literature

Patent Literature 1: WO2003/102150
Patent Literature 2: WO2016/021683
Patent Literature 3: WO2019/131839
Patent Literature 4: WO2020/032184

### Summary of Invention

### Technical Problem

Cationic lipids capable of transferring a nucleic acid to a cell with high efficiency are expected to contribute to the development of nucleic acid medicaments that are excellent in exertion of drug efficacy, safety (low toxicity), etc. and have a therapeutically excellent effect. Also, cationic lipids capable of transferring a nucleic acid to various cells are expected to permit development of nucleic acid medicaments for various types of diseases caused in various tissues. The inventions of cationic lipids described in Patent Literatures 3 and 4 can exert given effects on the transfer of a nucleic acid to a cell with excellent efficiency or the transfer of a nucleic acid to various cells and however, are still susceptible to further improvement, for example, improvement in *in vivo* degradability (i.e., improvement in safety as medicaments), toward applications as nucleic acid medicaments.

An object of the present invention is to provide a technique for improving *in vivo* degradability (safety as medicaments) while being capable of transferring a nucleic acid to a cell with excellent efficiency, and a cationic lipid for use in this technique, etc. In another aspect, an object of the present invention is to provide a technique for improving *in vivo* degradability (safety as medicaments) while being capable of transferring a nucleic acid to various cells, and a compound for use in this technique, etc.

### Solution to Problem

The present inventors have conducted diligent studies to attain the objects and consequently completed the present invention by finding that use of a compound represented by the formula (I) given below, i.e., a cationic lipid having two ester bonds in at least one side chain among three side chains, or a salt thereof can attain the objects.

Specifically, the present invention relates to at least the following aspects.
[1] A compound or a salt thereof, wherein the compound is represented by the following formula (I): 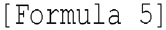 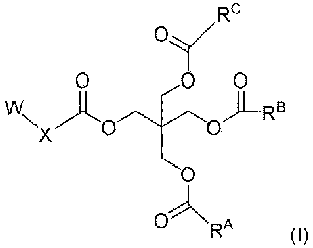 wherein
   W represents -NR¹R² or -N⁺R¹¹R¹²R¹³(Z⁻),
   R¹ and R² each independently represent H or an optionally substituted C₁₋₅ alkyl group,
   R¹¹, R¹² and R¹³ each independently represent an optionally substituted C₁₋₅ alkyl group,
   Z⁻ represents an anion,
   X represents an optionally substituted C₂₋₆ alkylene group,
   R^{A} and R^{B} each independently represent an optionally substituted C₁₋₁₇ alkyl group, an optionally substituted C₃₋₁₇ alkenyl group, an optionally substituted C₁₅₋₁₇ alkadienyl group, -R³-C(O)O-R⁴ or -R³-OC(O)-R⁴,
   R^{C} represents -R³-C(O)O-R⁴ or -R³-OC(O)-R⁴,
   R³ represents an optionally substituted C₁₋₁₆ alkylene group, an optionally substituted C₄₋₁₆ alkenylene group or an optionally substituted C₇₋₁₆ alkadienylene group, and
   R⁴ represents H, an optionally substituted C₁₋₁₈ alkyl group, an optionally substituted C₃₋₁₈ alkenyl group or an optionally substituted C₁₅₋₁₈ alkadienyl group,
[2] The compound according to [1] or a salt thereof, wherein the compound is represented by the following formula (II): 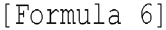 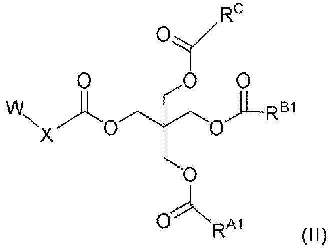 wherein
   W represents -NR¹R² or -N⁺R¹¹R¹²R¹³(Z⁻),
   R¹ and R² each independently represent H or an optionally substituted C₁₋₅ alkyl group,
   R¹¹, R¹² and R¹³ each independently represent an optionally substituted C₁₋₅ alkyl group,
   Z⁻ represents an anion,
   X represents an optionally substituted C₂₋₆ alkylene group,
   R^{A1} and R^{B1} each independently represent an optionally substituted C₁₋₁₇ alkyl group, an optionally substituted C₃₋₁₇ alkenyl group, or an optionally substituted C₁₅₋₁₇ alkadienyl group,
   R^{C} represents -R³-C(O)O-R⁴ or -R³-OC(O)-R⁴,
   R³ represents an optionally substituted C₁₋₁₆ alkylene group, an optionally substituted C₄₋₁₆ alkenylene group or an optionally substituted C₇₋₁₆ alkadienylene group, and
   R⁴ represents H, an optionally substituted C₁₋₁₈ alkyl group, an optionally substituted C₃₋₁₈ alkenyl group or an optionally substituted C₁₅₋₁₈ alkadienyl group.
[3] The compound according to [1] or a salt thereof, wherein the compound is represented by the following formula (III): 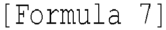 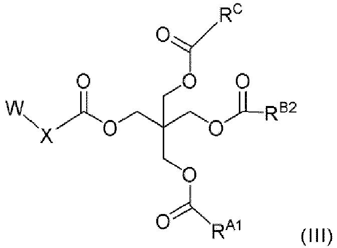 wherein
   W represents -NR¹R² or -N⁺R¹¹R¹²R¹³(Z⁻),
   R¹ and R² each independently represent H or an optionally substituted C₁₋₅ alkyl group,
   R¹¹, R¹² and R¹³ each independently represent an optionally substituted C₁₋₅ alkyl group,
   Z⁻ represents an anion,
   X represents an optionally substituted C₂₋₆ alkylene group,
   R^{A1} represents an optionally substituted C₁₋₁₇ alkyl group, an optionally substituted C₃₋₁₇ alkenyl group, or an optionally substituted C₁₅₋₁₇ alkadienyl group,
   R^{B2} and R^{C} each independently represent -R³-C(O)O-R⁴ or -R³-OC(O)-R⁴,
   R³ represents an optionally substituted C₁₋₁₆ alkylene group, an optionally substituted C₄₋₁₆ alkenylene group or an optionally substituted C₇₋₁₆ alkadienylene group, and
   R⁴ represents H, an optionally substituted C₁₋₁₈ alkyl group, an optionally substituted C₃₋₁₈ alkenyl group or an optionally substituted C₁₅₋₁₈ alkadienyl group.
[4] The compound according to [1] or a salt thereof, wherein the compound is represented by the following formula (IV): 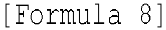 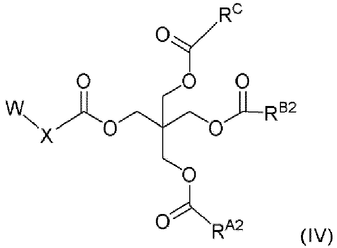 wherein
   W represents -NR¹R² or -N⁺R¹¹R¹²R¹³(Z⁻),
   R¹ and R² each independently represent H or an optionally substituted C₁₋₅ alkyl group,
   R¹¹, R¹² and R¹³ each independently represent an optionally substituted C₁₋₅ alkyl group,
   Z⁻ represents an anion,
   X represents an optionally substituted C₂₋₆ alkylene group,
   R^{A2}, R^{B2} and R^{C} each independently represent -R³-C(O)O-R⁴ or -R³-OC(O)-R⁴,
   R³ represents an optionally substituted C₁₋₁₆ alkylene group, an optionally substituted C₄₋₁₆ alkenylene group or an optionally substituted C₇₋₁₆ alkadienylene group, and
   R⁴ represents H, an optionally substituted C₁₋₁₈ alkyl group, an optionally substituted C₃₋₁₈ alkenyl group or an optionally substituted C₁₅₋₁₈ alkadienyl group.
[5] 1,1'-[2-({[4-(Dimethylamino)butanoyl]oxy]methyl)-2-{[(3-pentyloctanoyl)oxy]methyl}propane-1,3-diyl] 6,6'-diheptyl dihexanedioate.
[6] 1,1'-{2-({[4-(Dimethylamino)butanoyl]oxy}methyl)-2-[({4-oxo-4-[(undecan-6-yl)oxy]butanoyl}oxy)methyl]propane-1,3-diyl} 4,4'-diundecan-6-yl dibutanedioate.
[7] Decyl 2-({[4-(Dimethylamino)butanoyl]oxy}methyl)-3-[(3-pentyloctanoyl)oxy]-2-{[(3-pentyloctanoyl)oxy]methyl}propyl hexanedioate.
[8] 1,1'-[2-({[4-(Dimethylamino)butanoyl]oxy}methyl)-2-{[(3-pentyloctanoyl)oxy]methyl}propane-1,3-diyl] 6,6'-bis(2-propylpentyl) dihexanedioate.
[9] 1,1'-{2-({[4-(Dimethylamino)butanoyl]oxy}methyl)-2-[({3-oxo-3-[(2-pentylheptyl)oxy]propanoyl}oxy)methyl]propane-1,3-diyl} 3,3'-bis(2-pentylheptyl) dipropanedioate.
[10] 1,1'-[2-({[4-(Dimethylamino)butanoyl]oxy}methyl)-2-{[(3-pentyloctanoyl)oxy]methyl}propane-1,3-diyl] 3,3'-dihexyl bis(hexylpropanedioate).
[11] A lipid particle comprising a compound according to [1] or a salt thereof.
[12] A composition for nucleic acid transfer comprising a nucleic acid and a lipid particle according to [11].
[13] The composition according to [12], wherein the nucleic acid is DNA or RNA.
[14] The composition according to [13], wherein the DNA is Nanoplasmid.
[15] The composition according to [13], wherein the DNA is controlled by CAG promoter or CMV promoter.

In the present specification, the compound represented by the formulas (I)-(IV) is also referred to as the "compound (I)"-"compound (IV)". The "compound represented by formula (I) or a salt thereof" is also referred to as the "compound of the present invention". The "lipid particle comprising (or containing) the compound represented by the formula (I) or the salt thereof (the compound of the present invention)" is also referred to as the "lipid particle of the present invention". The "composition for nucleic acid transfer comprising (or containing) a nucleic acid and the lipid particle of the present invention" is also referred to as the "composition of the present invention".

### Advantageous Effects of Invention

The present invention enables *in vivo* degradability (safety as medicaments) to be improved while transferring a nucleic acid to a cell, a tissue or an organ with excellent efficiency. The present invention also enables *in vivo* degradability (safety as medicaments) to be improved while transferring a nucleic acid to many types of cells, tissues or organs (e.g., cancer cells). The present invention enables a medicament or a reagent for research to be obtained which transfers a nucleic acid to many types of cells, tissues or organs. In the case of transferring a nucleic acid to a cell, a tissue or an organ according to the present invention, the efficiency of exertion of the activity (e.g., drug efficacy) of the nucleic acid is high.

### Description of Embodiments

The compound of the present invention is a compound represented by the following formula (I) (compound (I)) or a salt thereof.

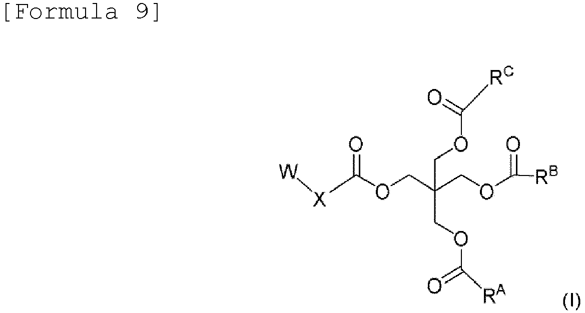

In the formula (I),
W represents -NR¹R² or -N⁺R¹¹R¹²R¹³(Z⁻),
R¹ and R² each independently represent H or an optionally substituted C₁₋₅ alkyl group,
R¹¹, R¹² and R¹³ each independently represent an optionally substituted C₁₋₅ alkyl group,
Z⁻ represents an anion,
X represents an optionally substituted C₂₋₆ alkylene group,
R^{A} and R^{B} each independently represent an optionally substituted C₁₋₁₇ alkyl group, an optionally substituted C₃₋₁₇ alkenyl group, an optionally substituted C₁₅₋₁₇ alkadienyl group, -R³-C(O)O-R⁴ or -R³-OC(O)-R⁴,
R^{C} represents -R³-C(O)O-R⁴ or -R³-OC(O)-R⁴,
R³ represents an optionally substituted C₁₋₁₆ alkylene group, an optionally substituted C₄₋₁₆ alkenylene group or an optionally substituted C₇₋₁₆ alkadienylene group, and
R⁴ represents H, an optionally substituted C₁₋₁₈ alkyl group, an optionally substituted C₃₋₁₈ alkenyl group or an optionally substituted C₁₅₋₁₈ alkadienyl group.

The "C₁₋₅ alkyl group" (R¹, R², R¹¹, R¹² and R¹³) refers to an alkyl group having 1 to 5 carbon atoms, which may be linear or branched. For the branched form, the number of bonds (how many carbon atoms are bonded) and a binding position (what number of carbon atom is bonded) in the branched chain are arbitrary. Specific examples of the C₁₋₅ alkyl group include the following:
[C₁] methyl;
[C₂] ethyl;
[C₃] propyl and 1-methylethyl (also called isopropyl);
[C₄] butyl, 1-methylpropyl (also called sec-butyl), 2-methylpropyl (also called isobutyl), and 1,1-dimethylethyl (also called tert-butyl); and
[C₅] pentyl, 1-methylbutyl, 2-methylbutyl, 3-methylbutyl (also called isopentyl), 1-ethylpropyl, 1,1-dimethylpropyl, 1,2-dimethylpropyl, and 2,2-dimethylpropyl (also called neopentyl).

The "optionally substituted C₁₋₅ alkyl group" of R¹ and R² is preferably an "optionally substituted C₁₋₄ alkyl group", more preferably an "optionally substituted C₁₋₃ alkyl group". The "optionally substituted C₁₋₅ alkyl group" of R¹¹, R¹² and R¹³ is preferably an "optionally substituted C₁₋₃ alkyl group".

The "C₂₋₆ alkylene group" (X) refers to a divalent group derived from an alkyl group having 2 to 6 carbon atoms, which may be linear or branched. For the branched form, the number of bonds and a binding position in the branched chain are arbitrary. Specific examples of the C₂₋₆ alkylene group include the following:
[C₂] ethylene and methylmethylene;
[C₃] propylene, 1-methylethylene, 2-methylethylene, and ethylmethylene;
[C₄] butylene, 1-methylpropylene, 2-methylpropylene, 3-methylpropylene, 1-ethylethylene, 2-ethylethylene, 1,2-dimethylethylene, and propylmethylene;
[C₅] pentylene, 1-methylbutylene, 2-methylbutylene, 3-methylbutylene, 4-methylbutylene, 1-ethylpropylene, 2-ethylpropylene, 3-ethylpropylene, 1,1-dimethylpropylene, 1,2-dimethylpropylene, 1,3-dimethylpropylene, 2,2-dimethylpropylene, 2,3-dimethylpropylene, 1-propylethylene, 2-propylethylene, and butylmethylene; and
[C₆] hexylene, 1-methylpentylene, 2-methylpentylene, 3-methylpentylene, 4-methylpentylene, 5-methylpentylene, 1-ethylbutylene, 2-ethylbutylene, 3-ethylbutylene, 4-ethylbutylene, 1,1-dimethylbutylene, 1,2-dimethylbutylene, 1,3-dimethylbutylene, 1,4-dimethylbutylene, 2,2-dimethylbutylene, 2,3-dimethylbutylene, 2,4-dimethylbutylene, 3,3-dimethylbutylene, 3,4-dimethylbutylene, 1-propylpropylene, 2-propylpropylene, 3-propylpropylene, 1-methyl-1-ethylpropylene, 1-methyl-2-ethylpropylene, 1-ethyl-2-methylpropylene, 1-methyl-3-ethylpropylene, 1-ethyl-3-methylpropylene, 2-methyl-2-ethylpropylene, 2-methyl-3-ethylpropylene, 2-ethyl-3-methylpropylene, 3-methyl-3-ethylpropylene, 1-butylethylene, 2-butylethylene, and pentylmethylene.

The "optionally substituted C₂₋₆ alkylene group" of X is preferably an "optionally substituted C₂₋₅ alkylene group", more preferably an "optionally substituted C₂₋₄ alkylene group".

The "C₁₋₁₇ alkyl group" (R^{A} and R^{B}) refers to an alkyl group having 1 to 17 carbon atoms, which may be linear or branched. For the branched form, the number of bonds and a binding position in the branched chain are arbitrary. Specific examples of the C₁₋₁₇ alkyl group include the specific examples of the C₁₋₅ alkyl group as well as the following, though the C₁₋₁₇ alkyl group that can be used in the present invention is not limited to these examples:
[C₆] hexyl, 1-methylpentyl, 2-methylpentyl, 3-methylpentyl, 4-methylpentyl (also called isohexyl), 1-ethylbutyl, 2-ethylbutyl, 1,1-dimethylbutyl, 1,2-dimethylbutyl, 1,3-dimethylbutyl, 2,2-dimethylbutyl, 2,3-dimethylbutyl, and 3,3-dimethylbutyl;
[C₇] heptyl, 1-methylhexyl, 2-methylhexyl, 3-methylhexyl, 4-methylhexyl, 5-methylhexyl, 1-ethylpentyl, 2-ethylpentyl, 3-ethylpentyl, and 1-propylbutyl;
[C₈] octyl, 1-methylpentyl, 2-methylpentyl, 3-methylpentyl, 4-methylpentyl, 5-methylpentyl, 6-methylpentyl, 1-ethylhexyl, 2-ethylhexyl, 3-ethylhexyl, 4-ethylhexyl, 1-propylpentyl, and 2-propylpentyl;
[C₉] nonyl, 1-methyloctyl, 2-methyloctyl, 3-methyloctyl, 4-methyloctyl, 5-methyloctyl, 6-methyloctyl, 7-methyloctyl, 1-ethylheptyl, 2-ethylheptyl, 3-ethylheptyl, 4-ethylheptyl, 5-ethylheptyl, 1-butylhexyl, 2-butylhexyl, 3-butylhexyl, and 1-butylpentyl;
[C₁₀] decyl, 1-methylnonyl, 2-methylnonyl, 3-methylnonyl, 4-methylnonyl, 5-methylnonyl, 6-methylnonyl, 7-methylnonyl, 8-methylnonyl, 1-ethyloctyl, 2-ethyloctyl, 3-ethyloctyl, 4-ethyloctyl, 5-ethyloctyl, 6-ethyloctyl, 1-propylheptyl, 2-propylheptyl, 3-propylheptyl, 1-butylhexyl, and 2-butylhexyl;
[C₁₁] undecyl, 1-methyldecyl, 2-methyldecyl, 3-methyldecyl, 4-methyldecyl, 5-methyldecyl, 6-methyldecyl, 7-methyldecyl, 8-methyldecyl, 9-methyldecyl, 1-ethylnonyl, 2-ethylnonyl, 3-ethylnonyl, 4-ethylnonyl, 5-ethylnonyl, 6-ethylnonyl, 7-ethylnonyl, 1-propyloctyl, 2-propyloctyl, 3-propyloctyl, 4-propyloctyl, 5-propyloctyl, 1-butylheptyl, 2-butylheptyl, 3-butylheptyl, and 1-pentylhexyl;
[C₁₂] dodecyl, 1-methylundecyl, 2-methylundecyl, 3-methylundecyl, 4-methylundecyl, 5-methylundecyl, 6-methylundecyl, 7-methylundecyl, 8-methylundecyl, 9-methylundecyl, 10-methylundecyl, 1-ethyldecyl, 2-ethyldecyl, 3-ethyldecyl, 4-ethyldecyl, 5-ethyldecyl, 6-ethyldecyl, 7-ethyldecyl, 8-ethyldecyl, 1-propylnonyl, 2-propylnonyl, 3-propylnonyl, 4-propylnonyl, 5-propylnonyl, 6-propylnonyl, 1-butyloctyl, 2-butyloctyl, 3-butyloctyl, 4-butyloctyl, 1-pentylheptyl, and 2-pentylheptyl;
[C₁₃] tridecyl, 1-methyldodecyl, 2-methyldodecyl, 3-methyldodecyl, 4-methyldodecyl, 5-methyldodecyl, 6-methyldodecyl, 7-methyldodecyl, 8-methyldodecyl, 9-methyldodecyl, 10-methyldodecyl, 11-methyldodecyl, 1-ethylundecyl, 2-ethylundecyl, 3-ethylundecyl, 4-ethylundecyl, 5-ethylundecyl, 6-ethylundecyl, 7-ethylundecyl, 8-ethylundecyl, 9-ethylundecyl, 1-propyldecyl, 2-propyldecyl, 3-propyldecyl, 4-propyldecyl, 5-propyldecyl, 6-propyldecyl, 7-propyldecyl, 1-butylnonyl, 2-butylnonyl, 3-butylnonyl, 4-butylnonyl, 5-butylnonyl, 1-pentyloctyl, 2-pentyloctyl, 3-pentyloctyl, 1-hexylpentyl, and 3,4-dipropylheptyl;
[C₁₄] tetradecyl, 1-methyltridecyl, 2-methyltridecyl, 3-methyltridecyl, 4-methyltridecyl, 5-methyltridecyl, 6-methyltridecyl, 7-methyltridecyl, 8-methyltridecyl, 9-methyltridecyl, 10-methyltridecyl, 11-methyltridecyl, 12-methyltridecyl, 1-ethyldodecyl, 2-ethyldodecyl, 3-ethyldodecyl, 4-ethyldodecyl, 5-ethyldodecyl, 6-ethyldodecyl, 7-ethyldodecyl, 8-ethyldodecyl, 9-ethyldodecyl, 10-ethyldodecyl, 1-propylundecyl, 2-propylundecyl, 3-propylundecyl, 4-propylundecyl, 5-propylundecyl, 6-propylundecyl, 7-propylundecyl, 8-propylundecyl, 1-butyldecyl, 2-butyldecyl, 3-butyldecyl, 4-butyldecyl, 5-butyldecyl, 6-butyldecyl, 1-pentylnonyl, 2-pentylnonyl, 3-pentylnonyl, 4-pentylnonyl, 1-hexyloctyl, and 2-hexyloctyl;
[C₁₅] pentadecyl, 1-methyltetradecyl, 2-methyltetradecyl, 3-methyltetradecyl, 4-methyltetradecyl, 5-methyltetradecyl, 6-methyltetradecyl, 7-methyltetradecyl, 8-methyltetradecyl, 9-methyltetradecyl, 10-methyltetradecyl, 11-methyltetradecyl, 12-methyltetradecyl, 13-methyltetradecyl, 1-ethyltridecyl, 2-ethyltridecyl, 3-ethyltridecyl, 4-ethyltridecyl, 5-ethyltridecyl, 6-ethyltridecyl, 7-ethyltridecyl, 8-ethyltridecyl, 9-ethyltridecyl, 10-ethyltridecyl, 11-ethyltridecyl, 1-propyldodecyl, 2-propyldodecyl, 3-propyldodecyl, 4-propyldodecyl, 5-propyldodecyl, 6-propyldodecyl, 7-propyldodecyl, 8-propyldodecyl, 9-propyldodecyl, 1-butylundecyl, 2-butylundecyl, 3-butylundecyl, 4-butylundecyl, 5-butylundecyl, 6-butylundecyl, 7-butylundecyl, 1-pentyldecyl, 2-pentyldecyl, 3-pentyldecyl, 4-pentyldecyl, 5-pentyldecyl, 1-hexylnonyl, 2-hexylnonyl, 3-hexylnonyl, and 1-heptyloctyl;
[C₁₆] hexadecyl, 1-methylpentadecyl, 2-methylpentadecyl, 3-methylpentadecyl, 4-methylpentadecyl, 5-methylpentadecyl, 6-methylpentadecyl, 7-methylpentadecyl, 8-methylpentadecyl, 9-methylpentadecyl, 10-methylpentadecyl, 11-methylpentadecyl, 12-methylpentadecyl, 13-methylpentadecyl, 14-methylpentadecyl, 1-ethyltetradecyl, 2-ethyltetradecyl, 3-ethyltetradecyl, 4-ethyltetradecyl, 5-ethyltetradecyl, 6-ethyltetradecyl, 7-ethyltetradecyl, 8-ethyltetradecyl, 9-ethyltetradecyl, 10-ethyltetradecyl, 11-ethyltetradecyl, 12-ethyltetradecyl, 1-propyltridecyl, 2-propyltridecyl, 3-propyltridecyl, 4-propyltridecyl, 5-propyltridecyl, 6-propyltridecyl, 7-propyltridecyl, 8-propyltridecyl, 9-propyltridecyl, 10-propyltridecyl, 1-butyldodecyl, 2-butyldodecyl, 3-butyldodecyl, 4-butyldodecyl, 5-butyldodecyl, 6-butyldodecyl, 7-butyldodecyl, 8-butyldodecyl, 1-pentylundecyl, 2-pentylundecyl, 3-pentylundecyl, 4-pentylundecyl, 5-pentylundecyl, 6-pentylundecyl, 1-hexyldecyl, 2-hexyldecyl, 3-hexyldecyl, 4-hexyldecyl, 1-heptylnonyl, 2-heptylnonyl, and 3,4-dibutyloctyl; and
[C₁₇] heptadecyl, 1-methylhexadecyl, 2-methylhexadecyl, 3-methylhexadecyl, 4-methylhexadecyl, 5-methylhexadecyl, 6-methylhexadecyl, 7-methylhexadecyl, 8-methylhexadecyl, 9-methylhexadecyl, 10-methylhexadecyl, 11-methylhexadecyl, 12-methylhexadecyl, 13-methylhexadecyl, 14-methylhexadecyl, 15-methylhexadecyl, 1-ethylpentadecyl, 2-ethylpentadecyl, 3-ethylpentadecyl, 4-ethylpentadecyl, 5-ethylpentadecyl, 6-ethylpentadecyl, 7-ethylpentadecyl, 8-ethylpentadecyl, 9-ethylpentadecyl, 10-ethylpentadecyl, 11-ethylpentadecyl, 12-ethylpentadecyl, 13-ethylpentadecyl, 1-propyltetradecyl, 2-propyltetradecyl, 3-propyltetradecyl, 4-propyltetradecyl, 5-propyltetradecyl, 6-propyltetradecyl, 7-propyltetradecyl, 8-propyltetradecyl, 9-propyltetradecyl, 10-propyltetradecyl, 11-propyltetradecyl, 1-butyltridecyl, 2-butyltridecyl, 3-butyltridecyl, 4-butyltridecyl, 5-butyltridecyl, 6-butyltridecyl, 7-butyltridecyl, 8-butyltridecyl, 9-butyltridecyl, 1-pentyldodecyl, 2-pentyldodecyl, 3-pentyldodecyl, 4-pentyldodecyl, 5-pentyldodecyl, 6-pentyldodecyl, 7-pentyldodecyl, 1-hexylundecyl, 2-hexylundecyl, 3-hexylundecyl, 4-hexylundecyl, 5-hexylundecyl, 1-heptyldecyl, 2-heptyldecyl, 3-heptyldecyl, and 1-octylnonyl.

The "C₃₋₁₇ alkenyl group" (R^{A} and R^{B}) refers to an alkenyl group having 3 to 17 carbon atoms, which may be linear or branched. For the branched form, the number of bonds and a binding position in the branched chain are arbitrary, and the position of a carbon-carbon double bond is also arbitrary. Specific examples of the C₃₋₁₇ alkenyl group include the following, though the C₃₋₁₇ alkenyl group that can be used in the present invention is not limited to these examples:
[C₃] 1-propenyl and 2-propenyl;
[C₄] 1-butenyl, 2-butenyl, 3-butenyl, 1-methyl-1-propenyl, 2-methyl-1-propenyl, 1-methyl-2-propenyl, and 2-methyl-2-propenyl;
[C₅] 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1-ethyl-1-propenyl, 2-ethyl-1-propenyl, 1-ethyl-2-propenyl, and 2-ethyl-3-propenyl;
[C₆] 1-hexenyl, 2-hexenyl, 3-hexenyl, 4-hexenyl, 5-hexenyl, 1-methyl-1-pentenyl, 1-methyl-2-pentenyl, 1-methyl-3-pentenyl, 1-methyl-4-pentenyl, 2-methyl-1-pentenyl, 2-methyl-2-pentenyl, 2-methyl-3-pentenyl, 2-methyl-4-pentenyl, 3-methyl-1-pentenyl, 3-methyl-2-pentenyl, 3-methyl-3-pentenyl, 3-methyl-4-pentenyl, 4-methyl-1-pentenyl, 4-methyl-2-pentenyl, 4-methyl-3-pentenyl, 4-methyl-4-pentenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 3-ethyl-1-butenyl, 3-ethyl-2-butenyl, 3-ethyl-3-butenyl, 1-propyl-1-propenyl, 2-propyl-1-propenyl, 1-propyl-2-propenyl, and 2-propyl-2-propenyl;
[C₇] 1-heptenyl, 2-heptenyl, 3-heptenyl, 4-heptenyl, 5-heptenyl, and 6-heptenyl;
[C₈] 1-octenyl, 2-octenyl, 3-octenyl, 4-octenyl, 5-octenyl, 6-octenyl, and 7-octenyl;
[C₉] 1-nonenyl, 2-nonenyl, 3-nonenyl, 4-nonenyl, 5-nonenyl, 6-nonenyl, 7-nonenyl, and 8-nonenyl;
[C₁₀] 1-decenyl, 2-decenyl, 3-decenyl, 4-decenyl, 5-decenyl, 6-decenyl, 7-decenyl, 8-decenyl, and 9-decenyl (C₁₀);
[C₁₁] 1-undecenyl, 2-undecenyl, 3-undecenyl, 4-undecenyl, 5-undecenyl, 6-undecenyl, 7-undecenyl, 8-undecenyl, 9-undecenyl, and 10-undecenyl;
[C₁₂] 1-dodecenyl, 2-dodecenyl, 3-dodecenyl, 4-dodecenyl, 5-dodecenyl, 6-dodecenyl, 7-dodecenyl, 8-dodecenyl, 9-dodecenyl, 10-dodecenyl, and 11-dodecenyl;
[C₁₃] 1-tridecenyl, 2-tridecenyl, 3-tridecenyl, 4-tridecenyl, 5-tridecenyl, 6-tridecenyl, 7-tridecenyl, 8-tridecenyl, 9-tridecenyl, 10-tridecenyl, 11-tridecenyl, and 12-tridecenyl;
[C₁₄] 1-tetradecenyl, 2-tetradecenyl, 3-tetradecenyl, 4-tetradecenyl, 5-tetradecenyl, 6-tetradecenyl, 7-tetradecenyl, 8-tetradecenyl, 9-tetradecenyl, 10-tetradecenyl, 11-tetradecenyl, 12-tetradecenyl, and 13-tetradecenyl;
[C₁₅] 1-pentadecenyl, 2-pentadecenyl, 3-pentadecenyl, 4-pentadecenyl, 5-pentadecenyl, 6-pentadecenyl, 7-pentadecenyl, 8-pentadecenyl, 9-pentadecenyl, 10-pentadecenyl, 11-pentadecenyl, 12-pentadecenyl, 13-pentadecenyl, and 14-pentadecenyl;
[C₁₆] ;1-hexadecenyl, 2-hexadecenyl, 3-hexadecenyl, 4-hexadecenyl, 5-hexadecenyl, 6-hexadecenyl, 7-hexadecenyl, 8-hexadecenyl, 9-hexadecenyl, 10-hexadecenyl, 11-hexadecenyl, 12-hexadecenyl, 13-hexadecenyl, 14-hexadecenyl, and 15-hexadecenyl; and
[C₁₇] 1-heptadecenyl, 2-heptadecenyl, 3-heptadecenyl, 4-heptadecenyl, 5-heptadecenyl, 6-heptadecenyl, 7-heptadecenyl, 8-heptadecenyl, 9-heptadecenyl, 10-heptadecenyl, 11-heptadecenyl, 12-heptadecenyl, 13-heptadecenyl, 14-heptadecenyl, 15-heptadecenyl, and 16-heptadecenyl. C₃₋₁₇ alkenyl groups contain one carbon-carbon double bond and may therefore assume cis and trans structures, any of which may be allowed.

The "C₁₅₋₁₇ alkadienyl group" (R^{A} and R^{B}) refers to an alkadienyl group having 15 to 17 carbon atoms, which may be linear or branched. For the branched form, the number of bonds and a binding position in the branched chain are arbitrary, and the positions of two carbon-carbon double bonds are also arbitrary. Specific examples of the C₁₅₋₁₇ alkadienyl group include the following, though the C₁₅₋₁₇ alkadienyl group that can be used in the present invention is not limited to these examples:
[C₁₅] 1,3-pentadecadienyl, 1,4-pentadecadienyl, 1,5-pentadecadienyl, 1,6-pentadecadienyl, 1,7-pentadecadienyl, 1,8-pentadecadienyl, 1,9-pentadecadienyl, 1,10-pentadecadienyl, 1,11-pentadecadienyl, 1,12-pentadecadienyl, 1,13-pentadecadienyl, 1,14-pentadecadienyl, 2,4-pentadecadienyl, 2,5-pentadecadienyl, 2,6-pentadecadienyl, 2,7-pentadecadienyl, 2,8-pentadecadienyl, 2,9-pentadecadienyl, 2,10-pentadecadienyl, 2,11-pentadecadienyl, 2,12-pentadecadienyl, 2,13-pentadecadienyl, 2,14-pentadecadienyl, 3,5-pentadecadienyl, 3,6-pentadecadienyl, 3,7-pentadecadienyl, 3,8-pentadecadienyl, 3,9-pentadecadienyl, 3,10-pentadecadienyl, 3,11-pentadecadienyl, 3,12-pentadecadienyl, 3,13-pentadecadienyl, 3,14-pentadecadienyl, 4,6-pentadecadienyl, 4,7-pentadecadienyl, 4,8-pentadecadienyl, 4,9-pentadecadienyl, 4,10-pentadecadienyl, 4,11-pentadecadienyl, 4,12-pentadecadienyl, 4,13-pentadecadienyl, 4,14-pentadecadienyl, 5,7-pentadecadienyl, 5,8-pentadecadienyl, 5,9-pentadecadienyl, 5,10-pentadecadienyl, 5,11-pentadecadienyl, 5,12-pentadecadienyl, 5,13-pentadecadienyl, 5,14-pentadecadienyl, 6,8-pentadecadienyl, 6,9-pentadecadienyl, 6,10-pentadecadienyl, 6,11-pentadecadienyl, 6,12-pentadecadienyl, 6,13-pentadecadienyl, 6,14-pentadecadienyl, 7,9-pentadecadienyl, 7,10-pentadecadienyl, 7,11-pentadecadienyl, 7,12-pentadecadienyl, 7,13-pentadecadienyl, 7,14-pentadecadienyl, 8,10-pentadecadienyl, 8,11-pentadecadienyl, 8,12-pentadecadienyl, 8,13-pentadecadienyl, 8,14-pentadecadienyl, 9,11-pentadecadienyl, 9,12-pentadecadienyl, 9,13-pentadecadienyl, 9,14-pentadecadienyl, 10,12-pentadecadienyl, 10,13-pentadecadienyl, 10,14-pentadecadienyl, 11,13-pentadecadienyl, 11,14-pentadecadienyl, and 12,14-pentadecadienyl;
[C₁₆] 1,3-hexadecadienyl, 1,4-hexadecadienyl, 1,5-hexadecadienyl, 1,6-hexadecadienyl, 1,7-hexadecadienyl, 1,8-hexadecadienyl, 1,9-hexadecadienyl, 1,10-hexadecadienyl, 1,11-hexadecadienyl, 1,12-hexadecadienyl, 1,13-hexadecadienyl, 1,14-hexadecadienyl, 1,15-hexadecadienyl, 2,4-hexadecadienyl, 2,5-hexadecadienyl, 2,6-hexadecadienyl, 2,7-hexadecadienyl, 2,8-hexadecadienyl, 2,9-hexadecadienyl, 2,10-hexadecadienyl, 2,11-hexadecadienyl, 2,12-hexadecadienyl, 2,13-hexadecadienyl, 2,14-hexadecadienyl, 2,15-hexadecadienyl, 3,5-hexadecadienyl, 3,6-hexadecadienyl, 3,7-hexadecadienyl, 3,8-hexadecadienyl, 3,9-hexadecadienyl, 3,10-hexadecadienyl, 3,11-hexadecadienyl, 3,12-hexadecadienyl, 3,13-hexadecadienyl, 3,14-hexadecadienyl, 3,15-hexadecadienyl, 4,6-hexadecadienyl, 4,7-hexadecadienyl, 4,8-hexadecadienyl, 4,9-hexadecadienyl, 4,10-hexadecadienyl, 4,11-hexadecadienyl, 4,12-hexadecadienyl, 4,13-hexadecadienyl, 4,14-hexadecadienyl, 4,15-hexadecadienyl, 5,7-hexadecadienyl, 5,8-hexadecadienyl, 5,9-hexadecadienyl, 5,10-hexadecadienyl, 5,11-hexadecadienyl, 5,12-hexadecadienyl, 5,13-hexadecadienyl, 5,14-hexadecadienyl, 5,15-hexadecadienyl, 6,8-hexadecadienyl, 6,9-hexadecadienyl, 6,10-hexadecadienyl, 6,11-hexadecadienyl, 6,12-hexadecadienyl, 6,13-hexadecadienyl, 6,14-hexadecadienyl, 6,15-hexadecadienyl, 7,9-hexadecadienyl, 7,10-hexadecadienyl, 7,11-hexadecadienyl, 7,12-hexadecadienyl, 7,13-hexadecadienyl, 7,14-hexadecadienyl, 7,15-hexadecadienyl, 8,10-hexadecadienyl, 8,11-hexadecadienyl, 8,12-hexadecadienyl, 8,13-hexadecadienyl, 8,14-hexadecadienyl, 8,15-hexadecadienyl, 9,11-hexadecadienyl, 9,12-hexadecadienyl, 9,13-hexadecadienyl, 9,14-hexadecadienyl, 9,15-hexadecadienyl, 10,12-hexadecadienyl, 10,13-hexadecadienyl, 10,14-hexadecadienyl, 10,15-hexadecadienyl, 11,13-hexadecadienyl, 11,14-hexadecadienyl, 11,15-hexadecadienyl, 12,14-hexadecadienyl, 12,15-hexadecadienyl, 13,15-hexadecadienyl, and 13,16-hexadecadienyl; and
[C₁₇] 1,3-heptadecadienyl, 1,4-heptadecadienyl, 1,5-heptadecadienyl, 1,6-heptadecadienyl, 1,7-heptadecadienyl, 1,8-heptadecadienyl, 1,9-heptadecadienyl, 1,10-heptadecadienyl, 1,11-heptadecadienyl, 1,12-heptadecadienyl, 1,13-heptadecadienyl, 1,14-heptadecadienyl, 1,15-heptadecadienyl, 1,16-heptadecadienyl, 2,4-heptadecadienyl, 2,5-heptadecadienyl, 2,6-heptadecadienyl, 2,7-heptadecadienyl, 2,8-heptadecadienyl, 2,9-heptadecadienyl, 2,10-heptadecadienyl, 2,11-heptadecadienyl, 2,12-heptadecadienyl, 2,13-heptadecadienyl, 2,14-heptadecadienyl, 2,15-heptadecadienyl, 2,16-heptadecadienyl, 3,5-heptadecadienyl, 3,6-heptadecadienyl, 3,7-heptadecadienyl, 3,8-heptadecadienyl, 3,9-heptadecadienyl, 3,10-heptadecadienyl, 3,11-heptadecadienyl, 3,12-heptadecadienyl, 3,13-heptadecadienyl, 3,14-heptadecadienyl, 3,15-heptadecadienyl, 3,16-heptadecadienyl, 4,6-heptadecadienyl, 4,7-heptadecadienyl, 4,8-heptadecadienyl, 4,9-heptadecadienyl, 4,10-heptadecadienyl, 4,11-heptadecadienyl, 4,12-heptadecadienyl, 4,13-heptadecadienyl, 4,14-heptadecadienyl, 4,15-heptadecadienyl, 4,16-heptadecadienyl, 5,7-heptadecadienyl, 5,8-heptadecadienyl, 5,9-heptadecadienyl, 5,10-heptadecadienyl, 5,11-heptadecadienyl, 5,12-heptadecadienyl, 5,13-heptadecadienyl, 5,14-heptadecadienyl, 5,15-heptadecadienyl, 5,16-heptadecadienyl, 6,8-heptadecadienyl, 6,9-heptadecadienyl, 6,10-heptadecadienyl, 6,11-heptadecadienyl, 6,12-heptadecadienyl, 6,13-heptadecadienyl, 6,14-heptadecadienyl, 6,15-heptadecadienyl, 6,16-heptadecadienyl, 7,9-heptadecadienyl, 7,10-heptadecadienyl, 7,11-heptadecadienyl, 7,12-heptadecadienyl, 7,13-heptadecadienyl, 7,14-heptadecadienyl, 7,15-heptadecadienyl, 7,16-heptadecadienyl, 8,10-heptadecadienyl, 8,11-heptadecadienyl, 8,12-heptadecadienyl, 8,13-heptadecadienyl, 8,14-heptadecadienyl, 8,15-heptadecadienyl, 8,16-heptadecadienyl, 9,11-heptadecadienyl, 9,12-heptadecadienyl, 9,13-heptadecadienyl, 9,14-heptadecadienyl, 9,15-heptadecadienyl, 9,16-heptadecadienyl, 10,12-heptadecadienyl, 10,13-heptadecadienyl, 10,14-heptadecadienyl, 10,15-heptadecadienyl, 10,16-heptadecadienyl, 11,13-heptadecadienyl, 11,14-heptadecadienyl, 11,15-heptadecadienyl, 11,16-heptadecadienyl, 12,14-heptadecadienyl, 12,15-heptadecadienyl, 12,16-heptadecadienyl, 13,15-heptadecadienyl, 13,16-heptadecadienyl, and 14,16-heptadecadienyl (C₁₇). C₁₅₋₁₇ alkadienyl groups contain two carbon-carbon double bonds and may therefore assume cis and trans structures at each of the bonds independently, any of which may be allowed.

The "C₁₋₁₆ alkylene group" (R³) refers to a divalent group derived from an alkyl group having 1 to 16 carbon atoms, which may be linear or branched. For the branched form, a binding position in the branched chain is arbitrary. Specific examples of the C₁₋₁₆ alkylene group include the specific examples of the C₂₋₆ alkylene group as well as the following, though the C₁₋₁₆ alkylene group that can be used in the present invention is not limited to these examples:
[C₁] methylene;
[C₇] heptylene, 1-methylhexylene, 2-methylhexylene, 3-methylhexylene, 4-methylhexylene, 5-methylhexylene, 6-methylhexylene, 1-ethylpentylene, 2-ethylpentylene, 3-ethylpentylene, 4-ethylpentylene, 5-ethylpentylene, 1-propylbutylene, 2-propylbutylene, 3-propylbutylene, 4-propylbutylene), 1-butylpropylene, 2-butylpropylene, 3-butylpropylene, 1-pentylethylene, 2-pentylethylene, and hexylmethylene;
[C₈] octylene, 1-methylheptylene, 2-methylheptylene, 3-methylheptylene, 4-methylheptylene, 5-methylheptylene, 6-methylheptylene, 1-ethylhexylene, 2-ethylhexylene, 3-ethylhexylene, 4-ethylhexylene, 5-ethylhexylene, 6-ethylhexylene, 1-propylpentylene, 2-propylpentylene, 3-propylpentylene, 4-propylpentylene, 5-propylpentylene, 1-butylbutylene, 2-butylbutylene, 3-butylbutylene, 4-butylbutylene, 1-pentylpropylene, 2-pentylpropylene, 3-pentylpropylene, 1-hexylethylene, 2-hexylethylene, and heptylmethylene;
[C₉] nonylene, 1-methyloctylene, 2-methyloctylene, 3-methyloctylene, 4-methyloctylene, 5-methyloctylene, 6-methyloctylene, 7-methyloctylene, 8-methyloctylene, 1-ethylheptylene, 2-ethylheptylene, 3-ethylheptylene, 4-ethylheptylene, 5-ethylheptylene, 6-ethylheptylene, 7-ethylheptylene, 1-propylhexylene, 2-propylhexylene, 3-propylhexylene, 4-propylhexylene, 5-propylhexylene, 6-propylhexylene, 1-butylpentylene, 2-butylpentylene, 3-butylpentylene, 4-butylpentylene, 5-butylpentylene, 1-pentylbutylene, 2-pentylbutylene, 3-pentylbutylene, 4-pentylbutylene, 1-hexylpropylene, 2-hexylpropylene, 3-hexylpropylene, 1-heptylethylene, 2-heptylethylene, and octylmethylene;
[C₁₀] decylene, 1-methylnonylene, 2-methylnonylene, 3-methylnonylene, 4-methylnonylene, 5-methylnonylene, 6-methylnonylene, 7-methylnonylene, 8-methylnonylene, 9-methylnonylene, 1-ethyloctylene, 2-ethyloctylene, 3-ethyloctylene, 4-ethyloctylene, 5-ethyloctylene, 6-ethyloctylene, 7-ethyloctylene, 8-ethyloctylene, 1-propylheptylene, 2-propylheptylene, 3-propylheptylene, 4-propylheptylene, 5-propylheptylene, 6-propylheptylene, 7-propylheptylene, 1-butylhexylene, 2-butylhexylene, 3-butylhexylene, 4-butylhexylene, 5-butylhexylene, 6-butylhexylene, 1-pentylpentylene, 2-pentylpentylene, 3-pentylpentylene, 4-pentylpentylene, 5-pentylpentylene, 1-hexylbutylene, 2-hexylbutylene, 3-hexylbutylene, 4-hexylbutylene, 1-heptylpropylene, 2-heptylpropylene, 3-heptylpropylene, 1-octylethylene, 2-octylethylene, and nonylmethylene;
[C₁₁] undecylene, 1-methyldecylene, 2-methyldecylene, 3-methyldecylene, 4-methyldecylene, 5-methyldecylene, 6-methyldecylene, 7-methyldecylene, 8-methyldecylene, 9-methyldecylene, 10-methyldecylene, 1-ethylnonylene, 2-ethylnonylene, 3-ethylnonylene, 4-ethylnonylene, 5-ethylnonylene, 6-ethylnonylene, 7-ethylnonylene, 8-ethylnonylene, 9-ethylnonylene, 1-propyloctylene, 2-propyloctylene, 3-propyloctylene, 4-propyloctylene, 5-propyloctylene, 6-propyloctylene, 7-propyloctylene, 8-propyloctylene, 1-butylheptylene, 2-butylheptylene, 3-butylheptylene, 4-butylheptylene, 5-butylheptylene, 6-butylheptylene, 7-butylheptylene, 1-pentylhexylene, 2-pentylhexylene, 3-pentylhexylene, 4-pentylhexylene, 5-pentylhexylene, 6-pentylhexylene, 1-hexylpentylene, 2-hexylpentylene, 3-hexylpentylene, 4-hexylpentylene, 5-hexylpentylene, 1-heptylbutylene, 2-heptylbutylene, 3-heptylbutylene, 4-heptylbutylene, 1-octylpropylene, 2-octylpropylene, 3-octylpropylene, 1-nonylethylene, 2-nonylethylene, and decylmethylene;
[C₁₂] dodecylene, 1-methylundecylene, 2-methylundecylene, 3-methylundecylene, 4-methylundecylene, 5-methylundecylene, 6-methylundecylene, 7-methylundecylene, 8-methylundecylene, 9-methylundecylene, 10-methylundecylene, 11-methylundecylene, 1-ethyldecylene, 2-ethyldecylene, 3-ethyldecylene, 4-ethyldecylene, 5-ethyldecylene, 6-ethyldecylene, 7-ethyldecylene, 8-ethyldecylene, 9-ethyldecylene, 10-ethyldecylene, 1-propylnonylene, 2-propylnonylene, 3-propylnonylene, 4-propylnonylene, 5-propylnonylene, 6-propylnonylene, 7-propylnonylene, 8-propylnonylene, 9-propylnonylene, 1-butyloctylene, 2-butyloctylene, 3-butyloctylene, 4-butyloctylene, 5-butyloctylene, 6-butyloctylene, 7-butyloctylene, 8-butyloctylene, 1-pentylheptylene, 2-pentylheptylene, 3-pentylheptylene, 4-pentylheptylene, 5-pentylheptylene, 6-pentylheptylene, 7-pentylheptylene, 1-hexylhexylene, 2-hexylhexylene, 3-hexylhexylene, 4-hexylhexylene, 5-hexylhexylene, 6-hexylhexylene, 1-heptylpentylene, 2-heptylpentylene, 3-heptylpentylene, 4-heptylpentylene, 5-heptylpentylene, 1-octylbutylene, 2-octylbutylene, 3-octylbutylene, 4-octylbutylene, 1-nonylpropylene, 2-nonylpropylene, 3-nonylpropylene, 1-decylethylene, 2-decylethylene, and undecylmethylene;
[C₁₃] tridecylene, 1-methyldodecylene, 2-methyldodecylene, 3-methyldodecylene, 4-methyldodecylene, 5-methyldodecylene, 6-methyldodecylene, 7-methyldodecylene, 8-methyldodecylene, 9-methyldodecylene, 10-methyldodecylene, 11-methyldodecylene, 12-methyldodecylene, 1-ethylundecylene, 2-ethylundecylene, 3-ethylundecylene, 4-ethylundecylene, 5-ethylundecylene, 6-ethylundecylene, 7-ethylundecylene, 8-ethylundecylene, 9-ethylundecylene, 10-ethylundecylene, 11-ethylundecylene, 1-propyldecylene, 2-propyldecylene, 3-propyldecylene, 4-propyldecylene, 5-propyldecylene, 6-propyldecylene, 7-propyldecylene, 8-propyldecylene, 9-propyldecylene, 10-propyldecylene, 1-butylnonylene, 2-butylnonylene, 3-butylnonylene, 4-butylnonylene, 5-butylnonylene, 6-butylnonylene, 7-butylnonylene, 8-butylnonylene, 9-butylnonylene, 1-pentyloctylene, 2-pentyloctylene, 3-pentyloctylene, 4-pentyloctylene, 5-pentyloctylene, 6-pentyloctylene, 7-pentyloctylene, 8-pentyloctylene, 1-hexylheptylene, 2-hexylheptylene, 3-hexylheptylene, 4-hexylheptylene, 5-hexylheptylene, 6-hexylheptylene, 7-hexylheptylene, 1-heptylhexylene, 2-heptylhexylene, 3-heptylhexylene, 4-heptylhexylene, 5-heptylhexylene, 6-heptylhexylene, 1-octylpentylene, 2-octylpentylene, 3-octylpentylene, 4-octylpentylene, 5-octylpentylene, 1-nonylbutylene, 2-nonylbutylene, 3-nonylbutylene, 4-nonylbutylene, 1-decylpropylene, 2-decylpropylene, 3-decylpropylene, 1-undecylethylene, 2-undecylethylene, and dodecylmethylene;
[C₁₄] tetradecylene, 1-methyltridecylene, 2-methyltridecylene, 3-methyltridecylene, 4-methyltridecylene, 5-methyltridecylene, 6-methyltridecylene, 7-methyltridecylene, 8-methyltridecylene, 9-methyltridecylene, 10-methyltridecylene, 11-methyltridecylene, 12-methyltridecylene, 13-methyltridecylene, 1-ethyldodecylene, 2-ethyldodecylene, 3-ethyldodecylene, 4-ethyldodecylene, 5-ethyldodecylene, 6-ethyldodecylene, 7-ethyldodecylene, 8-ethyldodecylene, 9-ethyldodecylene, 10-ethyldodecylene, 11-ethyldodecylene, 12-ethyldodecylene, 1-propylundecylene, 2-propylundecylene, 3-propylundecylene, 4-propylundecylene, 5-propylundecylene, 6-propylundecylene, 7-propylundecylene, 8-propylundecylene, 9-propylundecylene, 10-propylundecylene, 11-propylundecylene, 1-butyldecylene, 2-butyldecylene, 3-butyldecylene, 4-butyldecylene, 5-butyldecylene, 6-butyldecylene, 7-butyldecylene, 8-butyldecylene, 9-butyldecylene, 10-butyldecylene, 1-pentylnonylene, 2-pentylnonylene, 3-pentylnonylene, 4-pentylnonylene, 5-pentylnonylene, 6-pentylnonylene, 7-pentylnonylene, 8-pentylnonylene, 9-pentylnonylene, 1-hexyloctylene, 2-hexyloctylene, 3-hexyloctylene, 4-hexyloctylene, 5-hexyloctylene, 6-hexyloctylene, 7-hexyloctylene, 8-hexyloctylene, 1-heptylheptylene, 2-heptylheptylene, 3-heptylheptylene, 4-heptylheptylene, 5-heptylheptylene, 6-heptylheptylene, 7-heptylheptylene, 1-octylhexylene, 2-octylhexylene, 3-octylhexylene, 4-octylhexylene, 5-octylhexylene, 6-octylhexylene, 1-nonylpentylene, 2-nonylpentylene, 3-nonylpentylene, 4-nonylpentylene, 5-nonylpentylene, 1-decylbutylene, 2-decylbutylene, 3-decylbutylene, 4-decylbutylene, 1-undecylpropylene, 2-undecylpropylene, 3-undecylpropylene, 1-dodecylethylene, 2-dodecylethylene, and tridecylmethylene;
[C₁₅] pentadecylene, 1-methyltetradecylene, 2-methyltetradecylene, 3-methyltetradecylene, 4-methyltetradecylene, 5-methyltetradecylene, 6-methyltetradecylene, 7-methyltetradecylene, 8-methyltetradecylene, 9-methyltetradecylene, 10-methyltetradecylene, 11-methyltetradecylene, 12-methyltetradecylene, 13-methyltetradecylene, 14-methyltetradecylene, 1-ethyltridecylene, 2-ethyltridecylene, 3-ethyltridecylene, 4-ethyltridecylene, 5-ethyltridecylene, 6-ethyltridecylene, 7-ethyltridecylene, 8-ethyltridecylene, 9-ethyltridecylene, 10-ethyltridecylene, 11-ethyltridecylene, 12-ethyltridecylene, 13-ethyltridecylene, 1-propyldodecylene, 2-propyldodecylene, 3-propyldodecylene, 4-propyldodecylene, 5-propyldodecylene, 6-propyldodecylene, 7-propyldodecylene, 8-propyldodecylene, 9-propyldodecylene, 10-propyldodecylene, 11-propyldodecylene, 12-propyldodecylene, 1-butylundecylene, 2-butylundecylene, 3-butylundecylene, 4-butylundecylene, 5-butylundecylene, 6-butylundecylene, 7-butylundecylene, 8-butylundecylene, 9-butylundecylene, 10-butylundecylene, 11-butylundecylene, 1-pentyldecylene, 2-pentyldecylene, 3-pentyldecylene, 4-pentyldecylene, 5-pentyldecylene, 6-pentyldecylene, 7-pentyldecylene, 8-pentyldecylene, 9-pentyldecylene, 10-pentyldecylene, 1-hexylnonylene, 2-hexylnonylene, 3-hexylnonylene, 4-hexylnonylene, 5-hexylnonylene, 6-hexylnonylene, 7-hexylnonylene, 8-hexylnonylene, 9-hexylnonylene, 1-heptyloctylene, 2-heptyloctylene, 3-heptyloctylene, 4-heptyloctylene, 5-heptyloctylene, 6-heptyloctylene, 7-heptyloctylene, 8-heptyloctylene, 1-octylheptylene, 2-octylheptylene, 3-octylheptylene, 4-octylheptylene, 5-octylheptylene, 6-octylheptylene, 7-octylheptylene, 1-nonylhexylene, 2-nonylhexylene, 3-nonylhexylene, 4-nonylhexylene, 5-nonylhexylene, 6-nonylhexylene, 1-decylpentylene, 2-decylpentylene, 3-decylpentylene, 4-decylpentylene, 5-decylpentylene, 1-undecylbutylene, 2-undecylbutylene, 3-undecylbutylene, 4-undecylbutylene, 1-dodecylpropylene, 2-dodecylpropylene, 3-dodecylpropylene, 1-tridecylethylene, 2-tridecylethylene, and tetradecylmethylene; and
[C₁₆] hexadecylene, 1-methylpentadecylene, 2-methylpentadecylene, 3-methylpentadecylene, 4-methylpentadecylene, 5-methylpentadecylene, 6-methylpentadecylene, 7-methylpentadecylene, 8-methylpentadecylene, 9-methylpentadecylene, 10-methylpentadecylene, 11-methylpentadecylene, 12-methylpentadecylene, 13-methylpentadecylene, 14-methylpentadecylene, 15-methylpentadecylene, 1-ethyltetradecylene, 2-ethyltetradecylene, 3-ethyltetradecylene, 4-ethyltetradecylene, 5-ethyltetradecylene, 6-ethyltetradecylene, 7-ethyltetradecylene, 8-ethyltetradecylene, 9-ethyltetradecylene, 10-ethyltetradecylene, 11-ethyltetradecylene, 12-ethyltetradecylene, 13-ethyltetradecylene, 1-propyltridecylene, 2-propyltridecylene, 3-propyltridecylene, 4-propyltridecylene, 5-propyltridecylene, 6-propyltridecylene, 7-propyltridecylene, 8-propyltridecylene, 9-propyltridecylene, 10-propyltridecylene, 11-propyltridecylene, 12-propyltridecylene, 13-propyltridecylene, 1-butyldodecylene, 2-butyldodecylene, 3-butyldodecylene, 4-butyldodecylene, 5-butyldodecylene, 6-butyldodecylene, 7-butyldodecylene, 8-butyldodecylene, 9-butyldodecylene, 10-butyldodecylene, 11-butyldodecylene, 12-butyldodecylene, 1-pentylundecylene, 2-pentylundecylene, 3-pentylundecylene, 4-pentylundecylene, 5-pentylundecylene, 6-pentylundecylene, 7-pentylundecylene, 8-pentylundecylene, 9-pentylundecylene, 10-pentylundecylene, 11-pentylundecylene, 1-hexyldecylene, 2-hexyldecylene, 3-hexyldecylene, 4-hexyldecylene, 5-hexyldecylene, 6-hexyldecylene, 7-hexyldecylene, 8-hexyldecylene, 9-hexyldecylene, 10-hexyldecylene, 1-heptylnonylene, 2-heptylnonylene, 3-heptylnonylene, 4-heptylnonylene, 5-heptylnonylene, 6-heptylnonylene, 7-heptylnonylene, 8-heptylnonylene, 9-heptylnonylene, 1-octyloctylene, 2-octyloctylene, 3-octyloctylene, 4-octyloctylene, 5-octyloctylene, 6-octyloctylene, 7-octyloctylene, 8-octyloctylene, 1-nonylheptylene, 2-nonylheptylene, 3-nonylheptylene, 4-nonylheptylene, 5-nonylheptylene, 6-nonylheptylene, 7-nonylheptylene, 1-decylhexylene, 2-decylhexylene, 3-decylhexylene, 4-decylhexylene, 5-decylhexylene, 6-decylhexylene, 1-undecylpentylene, 2-undecylpentylene, 3-undecylpentylene, 4-undecylpentylene, 5-undecylpentylene, 1-dodecylbutylene, 2-dodecylbutylene, 3-dodecylbutylene, 4-dodecylbutylene, 1-tridecylpropylene, 2-tridecylpropylene, 3-tridecylpropylene, 1-tetradecylethylene, 2-tetradecylethylene, and pentadecylmethylene.

The "optionally substituted C₁₋₁₆ alkylene group" of R³ is preferably an "optionally substituted C₁₋₁₀ alkylene group".

The "C₄₋₁₆ alkenylene group" (R³) refers to a divalent group derived from an alkenyl group having 4 to 16 carbon atoms, which may be linear or branched. For the branched form, the number of bonds and a binding position in the branched chain are arbitrary, and the position of a carbon-carbon double bond is also arbitrary. Specific examples of the C₄₋₁₆ alkenylene group include the following, though the C₄₋₁₆ alkenylene group that can be used in the present invention is not limited to these examples:
[C₄] 1-butenylene, 2-butenylene, 3-butenylene, 1-methyl-1-propenylene, 2-methyl-1-propenylene, 1-methyl-2-propenylene, and 2-methyl-2-propenylene;
[C₅] 1-pentenylene, 2-pentenylene, 3-pentenylene, 4-pentenylene, 1-methyl-1-butenylene, 2-methyl-1-butenylene, 3-methyl-1-butenylene, 1-methyl-2-butenylene, 2-methyl-2-butenylene, 3-methyl-2-butenylene, 1-methyl-3-butenylene, 2-methyl-3-butenylene, 3-methyl-3-butenylene, 1-ethyl-1-propenylene, 2-ethyl-1-propenylene, 1-ethyl-2-propenylene, and 2-ethyl-3-propenylene;
[C₆] 1-hexenylene, 2-hexenylene, 3-hexenylene, 4-hexenylene, 5-hexenylene, 1-methyl-1-pentenylene, 1-methyl-2-pentenylene, 1-methyl-3-pentenylene, 1-methyl-4-pentenylene, 2-methyl-1-pentenylene, 2-methyl-2-pentenylene, 2-methyl-3-pentenylene, 2-methyl-4-pentenylene, 3-methyl-1-pentenylene, 3-methyl-2-pentenylene, 3-methyl-3-pentenylene, 3-methyl-4-pentenylene, 4-methyl-1-pentenylene, 4-methyl-2-pentenylene, 4-methyl-3-pentenylene, 4-methyl-4-pentenylene, 1-ethyl-1-butenylene, 1-ethyl-2-butenylene, 1-ethyl-3-butenylene, 2-ethyl-1-butenylene, 2-ethyl-2-butenylene, 2-ethyl-3-butenylene, 3-ethyl-1-butenylene, 3-ethyl-2-butenylene, 3-ethyl-3-butenylene, 1-propyl-1-propenylene, 2-propyl-1-propenylene, 1-propyl-2-propenylene, and 2-propyl-2-propenylene;
[C₇] 1-heptenylene, 2-heptenylene, 3-heptenylene, 4-heptenylene, 5-heptenylene, and 6-heptenylene;
[C₈] 1-octenylene, 2-octenylene, 3-octenylene, 4-octenylene, 5-octenylene, 6-octenylene, and 7-octenylene;
[C₉] 1-nonenylene, 2-nonenylene, 3-nonenylene, 4-nonenylene, 5-nonenylene, 6-nonenylene, 7-nonenylene, and 8-nonenylene;
[C₁₀] 1-decenylene, 2-decenylene, 3-decenylene, 4-decenylene, 5-decenylene, 6-decenylene, 7-decenylene, 8-decenylene, and 9-decenylene (C₁₀);
[C₁₁] 1-undecenylene, 2-undecenylene, 3-undecenylene, 4-undecenylene, 5-undecenylene, 6-undecenylene, 7-undecenylene, 8-undecenylene, 9-undecenylene, and 10-undecenylene;
[C₁₂] 1-dodecenylene, 2-dodecenylene, 3-dodecenylene, 4-dodecenylene, 5-dodecenylene, 6-dodecenylene, 7-dodecenylene, 8-dodecenylene, 9-dodecenylene, 10-dodecenylene, and 11-dodecenylene;
[C₁₃] 1-tridecenylene, 2-tridecenylene, 3-tridecenylene, 4-tridecenylene, 5-tridecenylene, 6-tridecenylene, 7-tridecenylene, 8tridecenylene, 9-tridecenylene, 10-tridecenylene, 11-tridecenylene, and 12-tridecenylene;
[C₁₄] 1-tetradecenylene, 2-tetradecenylene, 3-tetradecenylene, 4-tetradecenylene, 5-tetradecenylene, 6-tetradecenylene, 7-tetradecenylene, 8-tetradecenylene, 9-tetradecenylene, 10-tetradecenylene, 11-tetradecenylene, 12-tetradecenylene, and 13-tetradecenylene;
[C₁₅] 1-pentadecenylene, 2-pentadecenylene, 3-pentadecenylene, 4-pentadecenylene, 5-pentadecenylene, 6-pentadecenylene, 7-pentadecenylene, 8-pentadecenylene, 9-pentadecenylene, 10-pentadecenylene, 11-pentadecenylene, 12-pentadecenylene, 13-pentadecenylene, and 14-pentadecenylene; and
[C₁₆] 1-hexadecenylene, 2-hexadecenylene, 3-hexadecenylene, 4-hexadecenylene, 5-hexadecenylene, 6-hexadecenylene, 7-hexadecenylene, 8-hexadecenylene, 9-hexadecenylene, 10-hexadecenylene, 11-hexadecenylene, 12-hexadecenylene, 13-hexadecenylene, 14-hexadecenylene, and 15-hexadecenylene. The C₄₋₁₆ alkenylene group contains one carbon-carbon double bond and may therefore assume cis and trans structures, any of which may be allowed.

The "C₇₋₁₆ alkadienylene group" (R³) refers to a divalent group derived from an alkadienyl group having 7 to 16 carbon atoms, which may be linear or branched. For the branched form, the number of bonds and a binding position in the branched chain are arbitrary, and the position of a carbon-carbon double bond is also arbitrary. Specific examples of the C₇₋₁₆ alkadienylene group include the following, though the C₇₋₁₆ alkadienylene group that can be used in the present invention is not limited to these examples:
[C₇] 1,3-heptadienylene, 1,4-heptadienylene, 1,5-heptadienylene, 1,6-heptadienylene, 2,4-heptadienylene, 2,5-heptadienylene, 2,6-heptadienylene, 3,5-heptadienylene, and 3,6-heptadienylene;
[C₈] 1,3-octadienylene, 1,4-octadienylene, 1,5-octadienylene, 1,6-octadienylene, 1,7-octadienylene, 2,4-octadienylene, 2,5-octadienylene, 2,6-octadienylene, 2,7-octadienylene, 3,5-octadienylene, 3,6-octadienylene, 3,7-octadienylene, 4,6-octadienylene, 4,7-octadienylene, and 5,7-octadienylene;
[C₉] 1,3-nonadienylene, 1,4-nonadienylene, 1,5-nonadienylene, 1,6-nonadienylene, 1,7-nonadienylene, 1,8-nonadienylene, 2,4-nonadienylene, 2,5-nonadienylene, 2,6-nonadienylene, 2,7-nonadienylene, 2,8-nonadienylene, 3,5-nonadienylene, 3,6-nonadienylene, 3,7-nonadienylene, 3,8-nonadienylene, 4,6-nonadienylene, 4,7-nonadienylene, 4,8-nonadienylene, 5,7-nonadienylene, 5,8-nonadienylene, and 6,8-nonadienylene;
[C₁₀] 1,3-decadienylene, 1,4-decadienylene, 1,5-decadienylene, 1,6-decadienylene, 1,7-decadienylene, 1,8-decadienylene, 1,9-decadienylene, 2,4-decadienylene, 2,5-decadienylene, 2,6-decadienylene, 2,7-decadienylene, 2,8-decadienylene, 2,9-decadienylene, 3,5-decadienylene, 3,6-decadienylene, 3,7-decadienylene, 3,8-decadienylene, 3,9-decadienylene, 4,6-decadienylene, 4,7-decadienylene, 4,8-decadienylene, 4,9-decadienylene, 5,7-decadienylene, 5,8-decadienylene, 5,9-decadienylene, 6,8-decadienylene, 6,9-decadienylene, and 7,9-decadienylene;
[C₁₁] 1,3-undecadienylene, 1,4-undecadienylene, 1,5-undecadienylene, 1,6-undecadienylene, 1,7-undecadienylene, 1,8-undecadienylene, 1,9-undecadienylene, 1,10-undecadienylene, 2,4-undecadienylene, 2,5-undecadienylene, 2,6-undecadienylene, 2,7-undecadienylene, 2,8-undecadienylene, 2,9-undecadienylene, 2,10-undecadienylene, 3,5-undecadienylene, 3,6-undecadienylene, 3,7-undecadienylene, 3,8-undecadienylene, 3,9-undecadienylene, 3,10-undecadienylene, 4,6-undecadienylene, 4,7-undecadienylene, 4,8-undecadienylene, 4,9-undecadienylene, 4,10-undecadienylene, 5,7-undecadienylene, 5,8-undecadienylene, 5,9-undecadienylene, 5,10-undecadienylene, 6,8-undecadienylene, 6,9-undecadienylene, 6,10-undecadienylene, 7,9-undecadienylene, 7,10-undecadienylene, and 8,10-undecadienylene;
[C₁₂] 1,3-dodecadienylene, 1,4-dodecadienylene, 1,5-dodecadienylene, 1,6-dodecadienylene, 1,7-dodecadienylene, 1,8-dodecadienylene, 1,9-dodecadienylene, 1,10-dodecadienylene, 1,11-dodecadienylene, 2,4-dodecadienylene, 2,5-dodecadienylene, 2,6-dodecadienylene, 2,7-dodecadienylene, 2,8-dodecadienylene, 2,9-dodecadienylene, 2,10-dodecadienylene, 2,11-dodecadienylene, 3,5-dodecadienylene, 3,6-dodecadienylene, 3,7-dodecadienylene, 3,8-dodecadienylene, 3,9-dodecadienylene, 3,10-dodecadienylene, 3,11-dodecadienylene, 4,6-dodecadienylene, 4,7-dodecadienylene, 4,8-dodecadienylene, 4,9-dodecadienylene, 4,10-dodecadienylene, 4,11-dodecadienylene, 5,7-dodecadienylene, 5,8-dodecadienylene, 5,9-dodecadienylene, 5,10-dodecadienylene, 5,11-dodecadienylene, 6,8-dodecadienylene, 6,9-dodecadienylene, 6,10-dodecadienylene, 6,11-dodecadienylene, 7,9-dodecadienylene, 7,10-dodecadienylene, 7,11-dodecadienylene, 8,10-dodecadienylene, 8,11-dodecadienylene, and 9,11-dodecadienylene;
[C₁₃] 1,3-tridecadienylene, 1,4-tridecadienylene, 1,5-tridecadienylene, 1,6-tridecadienylene, 1,7-tridecadienylene, 1,8-tridecadienylene, 1,9-tridecadienylene, 1,10-tridecadienylene, 1,11-tridecadienylene, 1,12-tridecadienylene, 2,4-tridecadienylene, 2,5-tridecadienylene, 2,6-tridecadienylene, 2,7-tridecadienylene, 2,8-tridecadienylene, 2,9-tridecadienylene, 2,10-tridecadienylene, 2,11-tridecadienylene, 2,12-tridecadienylene, 3,5-tridecadienylene, 3,6-tridecadienylene, 3,7-tridecadienylene, 3,8-tridecadienylene, 3,9-tridecadienylene, 3,10-tridecadienylene, 3,11-tridecadienylene, 3,12-tridecadienylene, 4,6-tridecadienylene, 4,7-tridecadienylene, 4,8-tridecadienylene, 4,9-tridecadienylene, 4,10-tridecadienylene, 4,11-tridecadienylene, 4,12-tridecadienylene, 5,7-tridecadienylene, 5,8-tridecadienylene, 5,9-tridecadienylene, 5,10-tridecadienylene, 5,11-tridecadienylene, 5,12-tridecadienylene, 6,8-tridecadienylene, 6,9-tridecadienylene, 6,10-tridecadienylene, 6,11-tridecadienylene, 6,12-tridecadienylene, 7,9-tridecadienylene, 7,10-tridecadienylene, 7,11-tridecadienylene, 7,12-tridecadienylene, 8,10-tridecadienylene, 8,11-tridecadienylene, 8,12-tridecadienylene, 9,11-tridecadienylene, 9,12-tridecadienylene, and 10,12-tridecadienylene;
[C₁₄] 1,3-tetradecadienylene, 1,4-tetradecadienylene, 1,5-tetradecadienylene, 1,6-tetradecadienylene, 1,7-tetradecadienylene, 1,8-tetradecadienylene, 1,9-tetradecadienylene, 1,10-tetradecadienylene, 1,11-tetradecadienylene, 1,12-tetradecadienylene, 1,13-tetradecadienylene, 2,4-tetradecadienylene, 2,5-tetradecadienylene, 2,6-tetradecadienylene, 2,7-tetradecadienylene, 2,8-tetradecadienylene, 2,9-tetradecadienylene, 2,10-tetradecadienylene, 2,11-tetradecadienylene, 2,12-tetradecadienylene, 2,13-tetradecadienylene, 3,5-tetradecadienylene, 3,6-tetradecadienylene, 3,7-tetradecadienylene, 3,8-tetradecadienylene, 3,9-tetradecadienylene, 3,10-tetradecadienylene, 3,11-tetradecadienylene, 3,12-tetradecadienylene, 3,13-tetradecadienylene, 4,6-tetradecadienylene, 4,7-tetradecadienylene, 4,8-tetradecadienylene, 4,9-tetradecadienylene, 4,10-tetradecadienylene, 4,11-tetradecadienylene, 4,12-tetradecadienylene, 4,13-tetradecadienylene, 5,7-tetradecadienylene, 5,8-tetradecadienylene, 5,9-tetradecadienylene, 5,10-tetradecadienylene, 5,11-tetradecadienylene, 5,12-tetradecadienylene, 5,13-tetradecadienylene, 6,8-tetradecadienylene, 6,9-tetradecadienylene, 6,10-tetradecadienylene, 6,11-tetradecadienylene, 6,12-tetradecadienylene, 6,13-tetradecadienylene, 7,9-tetradecadienylene, 7,10-tetradecadienylene, 7,11-tetradecadienylene, 7,12-tetradecadienylene, 7,13-tetradecadienylene, 8,10-tetradecadienylene, 8,11-tetradecadienylene, 8,12-tetradecadienylene, 8,13-tetradecadienylene, 9,11-tetradecadienylene, 9,12-tetradecadienylene, 9,13-tetradecadienylene, 9,14-tetradecadienylene, 10,12-tetradecadienylene, 10,13-tetradecadienylene, and 11,13-tetradecadienylene;
[C₁₅] 1,3-pentadecadienylene, 1,4-pentadecadienylene, 1,5-pentadecadienylene, 1,6-pentadecadienylene, 1,7-pentadecadienylene, 1,8-pentadecadienylene, 1,9-pentadecadienylene, 1,10-pentadecadienylene, 1,11-pentadecadienylene, 1,12-pentadecadienylene, 1,13-pentadecadienylene, 1,14-pentadecadienylene, 2,4-pentadecadienylene, 2,5-pentadecadienylene, 2,6-pentadecadienylene, 2,7-pentadecadienylene, 2,8-pentadecadienylene, 2,9-pentadecadienylene, 2,10-pentadecadienylene, 2,11-pentadecadienylene, 2,12-pentadecadienylene, 2,13-pentadecadienylene, 2,14-pentadecadienylene, 3,5-pentadecadienylene, 3,6-pentadecadienylene, 3,7-pentadecadienylene, 3,8-pentadecadienylene, 3,9-pentadecadienylene, 3,10-pentadecadienylene, 3,11-pentadecadienylene, 3,12-pentadecadienylene, 3,13-pentadecadienylene, 3,14-pentadecadienylene, 4,6-pentadecadienylene, 4,7-pentadecadienylene, 4,8-pentadecadienylene, 4,9-pentadecadienylene, 4,10-pentadecadienylene, 4,11-pentadecadienylene, 4,12-pentadecadienylene, 4,13-pentadecadienylene, 4,14-pentadecadienylene, 5,7-pentadecadienylene, 5,8-pentadecadienylene, 5,9-pentadecadienylene, 5,10-pentadecadienylene, 5,11-pentadecadienylene, 5,12-pentadecadienylene, 5,13-pentadecadienylene, 5,14-pentadecadienylene, 6,8-pentadecadienylene, 6,9-pentadecadienylene, 6,10-pentadecadienylene, 6,11-pentadecadienylene, 6,12-pentadecadienylene, 6,13-pentadecadienylene, 6,14-pentadecadienylene, 7,9-pentadecadienylene, 7,10-pentadecadienylene, 7,11-pentadecadienylene, 7,12-pentadecadienylene, 7,13-pentadecadienylene, 7,14-pentadecadienylene, 8,10-pentadecadienylene, 8,11-pentadecadienylene, 8,12-pentadecadienylene, 8,13-pentadecadienylene, 8,14-pentadecadienylene, 9,11-pentadecadienylene, 9,12-pentadecadienylene, 9,13-pentadecadienylene, 9,14-pentadecadienylene, 10,12-pentadecadienylene, 10,13-pentadecadienylene, 10,14-pentadecadienylene, 11,13-pentadecadienylene, 11,14-pentadecadienylene, and 12,14-pentadecadienylene; and
[C₁₆] 1,3-hexadecadienylene, 1,4-hexadecadienylene, 1,5-hexadecadienylene, 1,6-hexadecadienylene, 1,7-hexadecadienylene, 1,8-hexadecadienylene, 1,9-hexadecadienylene, 1,10-hexadecadienylene, 1,11-hexadecadienylene, 1,12-hexadecadienylene, 1,13-hexadecadienylene, 1,14-hexadecadienylene, 1,15-hexadecadienylene, 2,4-hexadecadienylene, 2,5-hexadecadienylene, 2,6-hexadecadienylene, 2,7-hexadecadienylene, 2,8-hexadecadienylene, 2,9-hexadecadienylene, 2,10-hexadecadienylene, 2,11-hexadecadienylene, 2,12-hexadecadienylene, 2,13-hexadecadienylene, 2,14-hexadecadienylene, 2,15-hexadecadienylene, 3,5-hexadecadienylene, 3,6-hexadecadienylene, 3,7-hexadecadienylene, 3,8-hexadecadienylene, 3,9-hexadecadienylene, 3,10-hexadecadienylene, 3,11-hexadecadienylene, 3,12-hexadecadienylene, 3,13-hexadecadienylene, 3,14-hexadecadienylene, 3,15-hexadecadienylene, 4,6-hexadecadienylene, 4,7-hexadecadienylene, 4,8-hexadecadienylene, 4,9-hexadecadienylene, 4,10-hexadecadienylene, 4,11-hexadecadienylene, 4,12-hexadecadienylene, 4,13-hexadecadienylene, 4,14-hexadecadienylene, 4,15-hexadecadienylene, 5,7-hexadecadienylene, 5,8-hexadecadienylene, 5,9-hexadecadienylene, 5,10-hexadecadienylene, 5,11-hexadecadienylene, 5,12-hexadecadienylene, 5,13-hexadecadienylene, 5,14-hexadecadienylene, 5,15-hexadecadienylene, 6,8-hexadecadienylene, 6,9-hexadecadienylene, 6,10-hexadecadienylene, 6,11-hexadecadienylene, 6,12-hexadecadienylene, 6,13-hexadecadienylene, 6,14-hexadecadienylene, 6,15-hexadecadienylene, 7,9-hexadecadienylene, 7,10-hexadecadienylene, 7,11-hexadecadienylene, 7,12-hexadecadienylene, 7,13-hexadecadienylene, 7,14-hexadecadienylene, 7,15-hexadecadienylene, 8,10-hexadecadienylene, 8,11-hexadecadienylene, 8,12-hexadecadienylene, 8,13-hexadecadienylene, 8,14-hexadecadienylene, 8,15-hexadecadienylene, 9,11-hexadecadienylene, 9,12-hexadecadienylene, 9,13-hexadecadienylene, 9,14-hexadecadienylene, 9,15-hexadecadienylene, 10,12-hexadecadienylene, 10,13-hexadecadienylene, 10,14-hexadecadienylene, 10,15-hexadecadienylene, 11,13-hexadecadienylene, 11,14-hexadecadienylene, 11,15-hexadecadienylene, 12,14-hexadecadienylene, 12,15-hexadecadienylene, 13,15-hexadecadienylene, and 13,16-hexadecadienylene. The C₇₋₁₆ alkadienylene group contains two carbon-carbon double bonds and may therefore assume cis and trans structures at each of the bonds independently, any of which may be allowed.

The "C₁₋₁₈ alkyl group" (R⁴) refers to an alkyl group having 1 to 18 carbon atoms, which may be linear or branched. For the branched form, the number of bonds and a binding position in the branched chain are arbitrary. Specific examples of the C₁₋₁₈ alkyl group include the specific examples of the "C₁₋₁₇ alkyl group" (R^{A} and R^{B}) as well as the following, though the C₁₋₁₈ alkyl group that can be used in the present invention is not limited to these examples:
[C₁₈] octadecyl, 1-methylheptadecyl, 2-methylheptadecyl, 3-methylheptadecyl, 4-methylheptadecyl, 5-methylheptadecyl, 6-methylheptadecyl, 7-methylheptadecyl, 8-methylheptadecyl, 9-methylheptadecyl, 10-methylheptadecyl, 11-methylheptadecyl, 12-methylheptadecyl, 13-methylheptadecyl, 14-methylheptadecyl, 15-methylheptadecyl, 16-methylheptadecyl, 1-ethylhexadecyl, 2-ethylhexadecyl, 3-ethylhexadecyl, 4-ethylhexadecyl, 5-ethylhexadecyl, 6-ethylhexadecyl, 7-ethylhexadecyl, 8-ethylhexadecyl, 9-ethylhexadecyl, 10-ethylhexadecyl, 11-ethylhexadecyl, 12-ethylhexadecyl, 13-ethylhexadecyl, 14-ethylhexadecyl, 1-propylpentadecyl, 2-propylpentadecyl, 3-propylpentadecyl, 4-propylpentadecyl, 5-propylpentadecyl, 6-propylpentadecyl, 7-propylpentadecyl, 8-propylpentadecyl, 9-propylpentadecyl, 10-propylpentadecyl, 11-propylpentadecyl, 12-propylpentadecyl, 1-butyltetradecyl, 2-butyltetradecyl, 3-butyltetradecyl, 4-butyltetradecyl, 5-butyltetradecyl, 6-butyltetradecyl, 7-butyltetradecyl, 8-butyltetradecyl, 9-butyltetradecyl, 10-butyltetradecyl, 1-pentyltridecyl, 2-pentyltridecyl, 3-pentyltridecyl, 4-pentyltridecyl, 5-pentyltridecyl, 6-pentyltridecyl, 7-pentyltridecyl, 8-pentyltridecyl, 1-hexyldodecyl, 2-hexyldodecyl, 3-hexyldodecyl, 4-hexyldodecyl, 5-hexyldodecyl, 6-hexyldodecyl, 1-heptylundecyl, 2-heptylundecyl, 3-heptylundecyl, 4-heptylundecyl, 1-octyldecyl, and 2-octyldecyl.

The "optionally substituted C₁₋₁₈ alkyl group" of R⁴ is preferably an "optionally substituted C₁₋₁₆ alkyl group", more preferably an "optionally substituted C₁₋₁₄ alkyl group".

The "C₃₋₁₈ alkenyl group" (R⁴) refers to an alkenyl group having 3 to 18 carbon atoms, which may be linear or branched. For the branched form, the number of bonds and a binding position in the branched chain are arbitrary, and the position of a carbon-carbon double bond is also arbitrary. Specific examples of the C₃₋₁₈ alkenyl group include the specific examples of the "C₃₋₁₇ alkenyl group" (R^{A} and R^{B}) as well as the following, though the C₃₋₁₈ alkenyl group that can be used in the present invention is not limited to these examples:
[C₁₈] 1-octadecenyl, 2-octadecenyl, 3-octadecenyl, 4-octadecenyl, 5-octadecenyl, 6-octadecenyl, 7-octadecenyl, 8-octadecenyl, 9-octadecenyl, 10-octadecenyl, 11-octadecenyl, 12-octadecenyl, 13-octadecenyl, 14-octadecenyl, 15-octadecenyl, 16-octadecenyl, and 17-octadecenyl. The C₃₋₁₈ alkenyl group contains one carbon-carbon double bond and may therefore assume cis and trans structures, any of which may be allowed.

The "optionally substituted C₃₋₁₈ alkenyl group" of R⁴ is preferably an "optionally substituted C₅₋₁₈ alkenyl group", more preferably an "optionally substituted C₇₋₁₈ alkenyl group".

The "C₁₅₋₁₈ alkadienyl group" (R⁴) refers to an alkadienyl group having 15 to 18 carbon atoms, which may be linear or branched. For the branched form, the number of bonds and a binding position in the branched chain are arbitrary, and the position of a carbon-carbon double bond is also arbitrary. Specific examples of the C₁₅₋₁₈ alkadienyl group include the specific examples of the "C₁₅₋₁₇ alkadienyl group" (R^{A} and R^{B}) as well as the following, though the C₁₅₋₁₈ alkadienyl group that can be used in the present invention is not limited to these examples:
[C₁₈] 1,3-octadecadienyl, 1,4-octadecadienyl, 1,5-octadecadienyl, 1,6-octadecadienyl, 1,7-octadecadienyl, 1,8-octadecadienyl, 1,9-octadecadienyl, 1,10-octadecadienyl, 1,11-octadecadienyl, 1,12-octadecadienyl, 1,13-octadecadienyl, 1,14-octadecadienyl, 1,15-octadecadienyl, 1,16-octadecadienyl, 1,17-octadecadienyl, 2,4-octadecadienyl, 2,5-octadecadienyl, 2,6-octadecadienyl, 2,7-octadecadienyl, 2,8-octadecadienyl, 2,9-octadecadienyl, 2,10-octadecadienyl, 2,11-octadecadienyl, 2,12-octadecadienyl, 2,13-octadecadienyl, 2,14-octadecadienyl, 2,15-octadecadienyl, 2,16-octadecadienyl, 2,17-octadecadienyl, 3,5-octadecadienyl, 3,6-octadecadienyl, 3,7-octadecadienyl, 3,8-octadecadienyl, 3,9-octadecadienyl, 3,10-octadecadienyl, 3,11-octadecadienyl, 3,12-octadecadienyl, 3,13-octadecadienyl, 3,14-octadecadienyl, 3,15-octadecadienyl, 3,16-octadecadienyl, 3,17-octadecadienyl, 4,6-octadecadienyl, 4,7-octadecadienyl, 4,8-octadecadienyl, 4,9-octadecadienyl, 4,10-octadecadienyl, 4,11-octadecadienyl, 4,12-octadecadienyl, 4,13-octadecadienyl, 4,14-octadecadienyl, 4,15-octadecadienyl, 4,16-octadecadienyl, 4,17-octadecadienyl, 5,7-octadecadienyl, 5,8-octadecadienyl, 5,9-octadecadienyl, 5,10-octadecadienyl, 5,11-octadecadienyl, 5,12-octadecadienyl, 5,13-octadecadienyl, 5,14-octadecadienyl, 5,15-octadecadienyl, 5,16-octadecadienyl, 5,17-octadecadienyl, 6,8-octadecadienyl, 6,9-octadecadienyl, 6,10-octadecadienyl, 6,11-octadecadienyl, 6,12-octadecadienyl, 6,13-octadecadienyl, 6,14-octadecadienyl, 6,15-octadecadienyl, 6,16-octadecadienyl, 6,17-octadecadienyl, 7,9-octadecadienyl, 7,10-octadecadienyl, 7,11-octadecadienyl, 7,12-octadecadienyl, 7,13-octadecadienyl, 7,14-octadecadienyl, 7,15-octadecadienyl, 7,16-octadecadienyl, 7,17-octadecadienyl, 8,10-octadecadienyl, 8,11-octadecadienyl, 8,12-octadecadienyl, 8,13-octadecadienyl, 8,14-octadecadienyl, 8,15-octadecadienyl, 8,16-octadecadienyl, 8,17-octadecadienyl, 9,11-octadecadienyl, 9,12-octadecadienyl, 9,13-octadecadienyl, 9,14-octadecadienyl, 9,15-octadecadienyl, 9,16-octadecadienyl, 9,17-octadecadienyl, 10,12-octadecadienyl, 10,13-octadecadienyl, 10,14-octadecadienyl, 10,15-octadecadienyl, 10,16-octadecadienyl, 10,17-octadecadienyl, 11,13-octadecadienyl, 11,14-octadecadienyl, 11,15-octadecadienyl, 11,16-octadecadienyl, 11,17-octadecadienyl, 12,14-octadecadienyl, 12,15-octadecadienyl, 12,16-octadecadienyl, 12,17-octadecadienyl, 13,15-octadecadienyl, 13,16-octadecadienyl, 13,17-octadecadienyl, 14,16-octadecadienyl, 14,17-octadecadienyl, and 15,17-octadecadienyl. The C₁₅₋₁₈ alkadienyl group contains two carbon-carbon double bonds and may therefore assume cis and trans structures at each of the bonds independently, any of which may be allowed.

Examples of the "substituent" that may be carried by each of the C₁₋₅ alkyl group (R¹, R², R¹¹, R¹² or R¹³); the C₁₋₁₇ alkyl group, the C₃₋₁₇ alkenyl group or the C₁₅₋₁₇ alkadienyl group (R^{A} or R^{B}/R^{A1} or R^{B1}); the C₁₋₁₆ alkylene group, the C₄₋₁₆ alkenylene group or the C₇₋₁₆ alkadienylene group (R³); and the C₁₋₁₈ alkyl group, the C₃₋₁₈ alkenyl group or the C₁₅₋₁₈ alkadienyl group (R⁴) include halogen atoms, a cyano group, a nitro group, acyl groups (e.g., a formyl group, an acetyl group, and a propionyl group), an optionally further substituted amino group, an optionally further substituted carbamoyl group, an optionally further substituted thiocarbamoyl group, an optionally further substituted sulfamoyl group, an optionally further substituted hydroxy group, an optionally further substituted sulfanyl (SH) group, and an optionally further substituted silyl group. Examples of the "further substituent" that may be carried by these "substituents" include halogen atoms, a cyano group, a nitro group, an amino group, a hydroxy group, and a sulfanyl group.

Z⁻ is not particularly limited as long as the anion can be electrically bonded to W which is -N⁺R¹¹R¹²R¹³. A pharmacologically acceptable anion is preferred. Preferred examples of Z⁻ include: halide ions (fluoride ions, chloride ions, bromide ions and iodide ions); anions of inorganic acids such as nitric acid ions, sulfuric acid ions, and phosphoric acid ions; anions of organic acids such as formic acid ions, acetic acid ions, trifluoroacetic acid ions, phthalic acid ions, fumaric acid ions, oxalic acid ions, tartaric acid ions, maleic acid ions, citric acid ions, succinic acid ions, malic acid ions, methanesulfonic acid ions, benzenesulfonic acid ions, and p-toluenesulfonic acid ions; and aspartic acid ions.

The compound (I) can be classified into the following three types depending on the embodiments of R^{A} and R^{B}, more specifically, whether each of R^{A} and R^{B} is an optionally substituted C₁₋₁₇ alkyl group, an optionally substituted C₃₋₁₇ alkenyl group, or an optionally substituted C₁₅₋₁₇ alkadienyl group containing no ester bond (hereinafter, referred to as "R^{A/B} group 1") or is -R³-C(O)O-R⁴ or -R³-OC(O)-R⁴ containing an ester bond (hereinafter, referred to as "R^{A/B} group 2").

The first type is a compound represented by the following formula (II) (compound (II)). The formula (II) corresponds to the formula (I) wherein each of R^{A} and R^{B} is R^{A/B} group 1 (which contains no ester bond). In this case, R^{A} and R^{B} are represented by R^{A1} and R^{B1}, respectively. In the formula (II), the symbols other than R^{A1} and R^{B1} are as defined in the formula (I). The compound (II) is a cationic lipid in which only one side chain, i.e., the side chain containing R^{C}, among three side chains has two ester bonds.

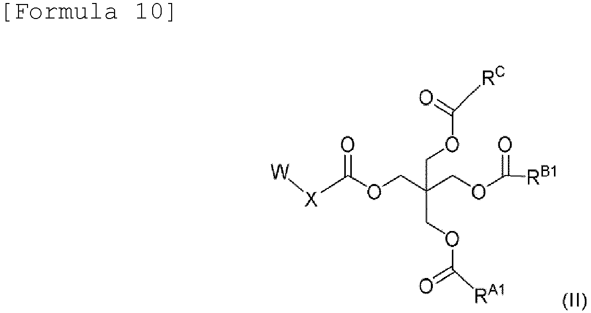

The second type is a compound represented by the following formula (III) (compound (III)). The formula (III) corresponds to the formula (I) wherein R^{A} is R^{A/B} group 1 (which contains no ester bond) and R^{B} is R^{A/B} group 2 (which contains an ester bond) . In this case, R^{A} and R^{B} are represented by R^{A1} and R^{B2}, respectively. In the formula (III), the symbols other than R^{A1} and R^{B2} are as defined in the formula (I). The compound (III) is a cationic lipid in which two side chains, i.e., the side chain containing R^{C} and the side chain containing R^{B2}, among three side chains each have two ester bonds.

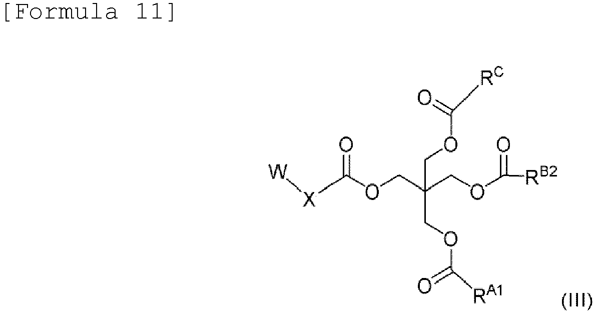

The third type is a compound represented by the following formula (IV) (compound (IV)). The formula (IV) corresponds to the formula (I) wherein each of R^{A} and R^{B} is -R³-C(O)O-R⁴ or -R³-OC(O)-R⁴ (R^{A/B} group 2) . In this case, R^{A} and R^{B} are represented by R^{A2} and R^{B2}, respectively. Specifically, in the formula (IV), all of R^{A2}, R^{B2} and R^{C} are defined as -R³-C(O)O-R⁴ or -R³-OC(O)-R⁴. In the formula (IV), the symbols other than R^{A2} and R^{B2} are as defined in the formula (I). The compound (IV) is a cationic lipid in which all of three side chains, i.e., all of the side chain containing R^{C}, the side chain containing R^{B2}, and the side chain containing R^{A2} each have two ester bonds.

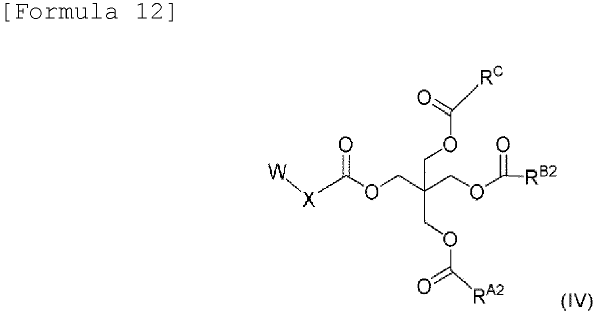

The salt of the compound (I) is preferably a pharmacologically acceptable salt. Examples thereof include salts with inorganic bases, salts with organic bases, salts with inorganic acids, salts with organic acids, and salts with basic or acidic amino acids.

Preferred examples of the salts with inorganic bases include: alkali metal salts such as sodium salt and potassium salt; alkaline earth metal salts such as calcium salt and magnesium salt; aluminum salt; and ammonium salt. Sodium salt, potassium salt, calcium salt, and magnesium salt are preferred, and sodium salt and potassium salt are more preferred.

Preferred examples of the salts with organic bases include salts with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine, and N,N-dibenzylethylenediamine.

Preferred examples of the salts with inorganic acids include salts with hydrofluoric acid, hydrochloric acid, hydrobromic acid, hydroiodic acid, nitric acid, sulfuric acid, and phosphoric acid. Salts with hydrochloric acid and salts with phosphoric acid are preferred.

Preferred examples of the salts with organic acids include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, and p-toluenesulfonic acid.

Preferred examples of the salts with basic amino acids include salts with arginine, lysine, and ornithine.

Preferred examples of the salts with acidic amino acids include salts with aspartic acid and glutamic acid.

In the present invention, the compound of the present invention can be used as a cationic lipid. The cationic lipid may form a complex with a plurality of molecules in a solvent or a dispersion medium. The complex may contain an additional component, in addition to the compound of the present invention. Examples of the additional component include other lipid components and nucleic acids.

Examples of the other lipid components include structured lipids that can constitute lipid particles. For example, at least one member selected from the group consisting of:
sterols (e.g., cholesterol, cholesterol ester, and cholesteryl hemisuccinate);
phospholipids (e.g., phosphatidylcholines (e.g., dipalmitoylphosphatidylcholine, distearoylphosphatidylcholine, lysophosphatidylcholine, dioleoylphosphatidylcholine, palmitoyloleoylphosphatidylcholine, dilinolenoylphosphatidylcholine, MC-1010 (NOF Corporation), MC-2020 (NOF Corporation), MC-4040 (NOF Corporation), MC-6060 (NOF Corporation), and MC-8080(NOF Corporation)), phosphatidylserines (e.g., dipalmitoylphosphatidylserine, distearoylphosphatidylserine, dioleoylphosphatidylserine, and palmitoyloleoylphosphatidylserine), phosphatidylethanolamines (e.g., dipalmitoylphosphatidylethanolamine, distearoylphosphatidylethanolamine, dioleoylphosphatidylethanolamine, palmitoyloleoylphosphatidylethanolamine, and lysophosphatidylethanolamine), phosphatidylinositol, and phosphatidic acid); and
polyethylene glycol lipids (PEG lipids) (e.g., PEG-DAA, PEG-DAG, PEG-phospholipid conjugate, PEG-Cer, PEG-cholesterol, PEG-C-DOMG, 2KPEG-CMG, GM-020 (NOF Corporation), GS-020 (NOF Corporation), and GS-050 (NOF Corporation))
can be used as such a structured lipid. In the present invention, all three members of a sterol (particularly, cholesterol), a phospholipid (particularly, phosphatidylcholine) and a polyethylene glycol lipid are preferably used as structured lipids.

The ratio between the compound of the present invention and the structured lipid in a mixed lipid component forming the lipid particle of the present invention can be appropriately adjusted according to a purpose or an application. For example, the ratio of the structured lipid is usually 0.008 to 4 mol, preferably 0.4 to 1.5 mol, based on 1 mol of the compound of the present invention. According to another definition, the contents in the mixed lipid component are usually 1 to 4 mol of the compound of the present invention, usually 0 to 3 mol of the sterol, usually 0 to 2 mol of the phospholipid and usually 0 to 1 mol of the polyethylene glycol lipid. In a more preferred aspect, in the case of using the compound of the present invention and an additional lipid component in a mixture, the contents are 1 to 1.5 mol of the compound of the present invention, 0 to 1.25 mol of the sterol, 0 to 0.5 mol of the phospholipid and 0 to 0.125 mol of the polyethylene glycol lipid.

The compound of the present invention can be used for producing the lipid particle of the present invention. The lipid particle of the present invention means the complex described above except that the complex does not contain a nucleic acid. The shape of the lipid particle of the present invention is not particularly limited and includes, for example, a complex assembled by the compound of the present invention, etc. so as to constitute a spherical shape, a complex assembled thereby without constituting a specific shape, a complex dissolved in a solvent, and a complex dispersed uniformly or nonuniformly in a dispersion medium.

The lipid particle of the present invention (e.g., a lipid particle constituted by the compound of the present invention and an additional structured lipid) can be used for producing, for example, the composition of the present invention containing the lipid particle and a nucleic acid (particularly, a nucleic acid which is a substance useful for a pharmaceutical application or an application for a research purpose). The composition of the present invention can be used as a medicament or a reagent. In the composition of the present invention, it is preferred that the maximum possible proportion of the nucleic acid should be encapsulated in the lipid particle (i.e., the rate of encapsulation should be high).

The "nucleic acid" can be any molecule of polymerized nucleotides and molecules having functions equivalent to those of the nucleotides. Examples thereof can include RNA which is a polymer of ribonucleotides, DNA which is a polymer of deoxyribonucleotides, a polymer of a mixture of ribonucleotides and deoxyribonucleotides, and a nucleotide polymer containing a nucleotide analog. Alternatively, a nucleotide polymer containing a nucleic acid derivative may be used. The nucleic acid may be a single-stranded nucleic acid or a double-stranded nucleic acid. The double-stranded nucleic acid also includes a double-stranded nucleic acid in which one of the strands hybridizes under stringent conditions to the other strand.

The nucleotide analog can be any molecule as long as the molecule is a ribonucleotide, a deoxyribonucleotide, RNA or DNA modified in order to improve nuclease resistance, in order to stabilize, in order to enhance affinity for a complementary strand nucleic acid, in order to enhance cell permeability, or in order to visualize the molecule, as compared with RNA or DNA. The nucleotide analog may be a naturally occurring molecule or a non-natural molecule. Examples thereof include a nucleotide analog with a modified sugar moiety and a nucleotide analog with a modified phosphodiester bond.

The nucleotide analog with a modified sugar moiety can be any molecule as long as an arbitrary chemical structural substance is added to or replaced for a portion or the whole of the chemical structure of a sugar in a nucleotide. Specific examples thereof include a nucleotide analog substituted by 2'-O-methyl ribose, a nucleotide analog substituted by 2'-O-propyl ribose, a nucleotide analog substituted by 2'-methoxyethoxy ribose, a nucleotide analog substituted by 2'-O-methoxyethyl ribose, a nucleotide analog substituted by 2'-O-[2-(guanidium)ethyl]ribose, a nucleotide analog substituted by 2'-fluoro ribose, a nucleic acid analog with a sugar moiety substituted by a morpholino ring (morpholino nucleic acid), bridged nucleic acid (BNA) having two cyclic structures by the introduction of a bridged structure to the sugar moiety, more specifically, locked nucleic acid (LNA) with an oxygen atom at position 2' and a carbon atom at position 4' bridged via methylene, and ethylene bridged nucleic acid (ENA) [Nucleic Acid Research, 32, e175 (2004)], and amide-bridged nucleic acid (AmNA) with a carbon atom at position 2' and a carbon atom at position 4' bridged via an amide bond, and can further include peptide nucleic acid (PNA)[Acc. Chem. Res., 32, 624 (1999)], oxypeptide nucleic acid (OPNA) [J. Am. Chem. Soc., 123, 4653 (2001)], and peptide ribonucleic acid (PRNA) [J. Am. Chem. Soc., 122, 6900 (2000)].

The nucleotide analog with a modified phosphodiester bond can be any molecule as long as an arbitrary chemical structural substance is added to or replaced for a portion or the whole of the chemical structure of a phosphodiester bond in a nucleotide. Specific examples thereof can include a nucleotide analog substituted by a phosphorothioate bond, and a nucleotide analog substituted by a N3'-P5' phosphoramidate bond [Cell Engineering, 16, 1463-1473 (1997)] [RNAi Method and Antisense Method, Kodansha Ltd. (2005)].

The nucleic acid derivative can be any molecule as long as the molecule is a nucleic acid with another chemical substance added thereto in order to improve nuclease resistance, in order to stabilize, in order to enhance affinity for a complementary strand nucleic acid, in order to enhance cell permeability, or in order to visualize the molecule, as compared with a nucleic acid. Specific examples thereof can include a 5'-polyamine-added derivative, a cholesterol-added derivative, a steroid-added derivative, a bile acid-added derivative, a vitamin-added derivative, a Cy5-added derivative, a Cy3-added derivative, a 6-FAM-added derivative, and a biotin-added derivative.

The nucleic acid according to the present invention is not particularly limited and may be a nucleic acid aimed at, for example, amelioration of a disease, a symptom, a disorder, or morbidity, and reduction of a disease, a symptom, a disorder or a pathological condition or prevention of onset thereof (in the present specification, also referred to as the "treatment, etc. of a disease"), or may be a nucleic acid for regulating the expression of the desired protein useful for research, albeit not contributing to the treatment, etc. of a disease.

Information on a gene or a polynucleotide related to a disease (in the present specification, also referred to as the "disease-related gene") is available from, for example, McKusick-Nathans Institute of Genetic Medicine, Johns Hopkins University (Baltimore, Md.) and National Center for Biotechnology Information, National Library of Medicine (Bethesda, Md.).

Specific examples of the nucleic acid according to the present invention include single-stranded DNA, double-stranded DNA, siRNA, miRNA, miRNA mimic, antisense nucleic acid, ribozyme, mRNA, gRNA, decoy nucleic acid, and aptamer, each of which may be an analog or derivative obtained by artificial modification. The nucleic acid is preferably DNA or RNA such as single-stranded DNA, double-stranded DNA, siRNA, mRNA, or gRNA, or an analog or derivative thereof obtained by artificial modification.

In the present invention, the "siRNA" means double-stranded RNA of 10 to 30 bases, preferably 15 to 25 bases, or an analog thereof, containing complementary sequences. The siRNA has preferably 1 to 3, more preferably 2 overhang bases at the 3' end. The complementary sequence moiety may be completely complementary or may contain a noncomplementary base, and is preferably completely complementary.

The siRNA according to the present invention is not particularly limited, and, for example, siRNA for knocking down the gene expression of a disease-related gene can be used. The disease-related gene refers to an arbitrary gene or polynucleotide that yields a transcription or translation product at an abnormal level or in an abnormal form in a cell derived from an affected tissue as compared with a non-diseased control tissue or cell. Alternatively, siRNA for regulating the expression of the desired protein useful for research may be used as the siRNA according to the present invention.

In the present invention, the "mRNA" means RNA containing a nucleotide sequence translatable into a protein. The mRNA according to the present invention is not particularly limited as long as the mRNA can cause intracellular expression of the desired protein. The mRNA is preferably mRNA useful for a pharmaceutical application (e.g., an application to disease treatment) and/or an application for a research purpose. Examples of such mRNA include mRNA for the intracellular expression of a marker protein such as luciferase.

In the present invention, the "gRNA" means guide RNA adapted to a CRISPR system. The gRNA according to the present invention may be in the form of a single RNA strand composed of crRNA and tracrRNA linked to each other, i.e., chimeric RNA (also called single guide RNA, sgRNA, etc.), or may be in the form of individual single RNA strands that are not linked (a combination of two RNA strands or a combination of three or more RNA strands).

In the present invention, the "DNA" means DNA containing a nucleotide sequence transcribable into mRNA. The DNA according to the present invention is not particularly limited as long as the DNA is transcribed into the desired mRNA in a cell. The DNA is preferably DNA useful for a pharmaceutical application (e.g., an application to disease treatment) and/or an application for a research purpose. Examples of such DNA include plasmid DNA (pDNA), single-stranded DNA (ssDNA), Nanoplasmid, minicircle DNA (Minicircle), closed-ended DNA (ceDNA), Doggybone DNA (dbDNA), ministring DNA (msDNA), and linear DNA (linDNA). Examples of such DNA include DNA for the intracellular expression of a marker protein such as luciferase.

The DNA according to the present invention may contain an enhancer or a promoter. The enhancer or the promoter according to the present invention is not particularly limited as long as the enhancer or the promoter can control transcription into the desired mRNA in a cell. Examples of the promoter or the enhancer include ApoE/hAAT enhancer or promoter, CAG promoter, CMV (cytomegalovirus) promoter, RSV (Rous sarcoma virus) promoter or enhancer, SV40 promoter, DHFR (dihydrofolate reductase) promoter, EF1α promoter, EF and CBA (chicken β-actin) promoter, PGK (phosphoglycerate kinase) promoter, hSYN (human synapsin) promoter, MND promoter, RSV (Rous sarcoma virus LTR) promoter, chicken beta actin + intron promoter, TRE (tetracycline-responsive element) promoter, UBC (ubiquitin C) promoter, MSCV U3 (murine stem cell virus LTR) promoter, GALV U3 (gibbon ape leukemia virus LTR) promoter, GUSB (beta glucuronidase) promoter, MeCP2 promoter, GFAP (glial fibrillary acidic protein) promoter, human beta actin promoter, EBV (Epstein-Barr virus) promoter, and SFFV (spleen focus forming virus LTR) promoter. The enhancer or the promoter is preferably CMV promoter or CAG promoter.

Examples of the disease described above include, but are not particularly limited to, diseases described in the (1)-(7) below. Examples of the disease-related gene are shown within the parentheses "()" except for the case where specific examples of the disease are described. Examples of the nucleic acid according to the present invention also include nucleic acids regulating the expression levels of these disease-related genes (or proteins encoded thereby).

(1) Hematological diseases: anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, and ASAT), bare lymphocyte syndrome (TAPBP, TPSN, TAP2, ABCB3, PSF2, RING11, MHC2TA, C2TA, and RFX5), bleeding diseases (TBXA2R, P2RX1, and P2X1), factor H and factor H-like factor 1 deficiency(HF1, CFH, and HUS), factor V and factor VIII deficiency (MCFD2), factor VII deficiency (F7), factor X deficiency (F10), factor XI deficiency (F11), factor XII deficiency (F12 and HAF), factor XIIIA deficiency (F13A1 and F13A), factor XIIIB deficiency (F13B), fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FACE, FACE, FANCF, XRCC9, FANCG, BRIP1, BACH1, FANCJ, PHF9, FANCL, FANCM, and KIAA1596), hemophagocytic lymphohistiocytosis (PRF1, HPLH2, UNC13D, MUNC13-4, HPLH3, HLH3, and FHL3), hemophilia A (F8, F8C, and HEMA), hemophilia B (F9 and HEMB), bleeding disorders (PI, ATT, and F5), leukocyte defect (ITGB2, CD18, LCAMB, LAD, EIF2B1, EIF2BA, EIF2B2, EIF2B3, EIF2B5, LVWM, CACH, CLE, and EIF2B4), sickle-cell anemia (HBB), thalassemia (HBA2, HBB, HBD, LCRB, and HBA1), von Willebrand disease (VWF), hypoalbuminemia, hypovolemia, severe congenital protein C deficiency, prothrombin deficiency, etc.
(2) Inflammatory or immunological diseases: AIDS (KIR3DL1, NKAT3, NKB1, AMB11, KIR3DS1, IFNG, CXCL12, and SDF1), autoimmune lymphoproliferative syndrome (TNFRSF6, APT1, FAS, CD95, and ALPS1A), combined immunodeficiency disease (IL2RG, SCIDX1, SCIDX, and IMD4), HIV infection (CCL5, SCYA5, D17S135E, TCP228, IL10, CSIF, CMKBR2, CCR2, DMKBR5, CCCKR5, and CCR5), immunodeficiency disease (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, and TACI), inflammation (IL10, IL-1, IL-13, IL-17, IL-23, and CTLA4), severe combined immunodeficiency disease (JAK3, JAKL, DCLRE1C, ATREMIS, SCIDA, RAG1, RAG2, ADA, PTPRC, CD45, LCA, IL7R, CD3D, T3D, IL2RG, SCIDX1, SCIDX, and IMD4), primary immunodeficiency, secondary immunodeficiency, multifocal motor neuropathy, Guillain-Barre syndrome, chronic inflammatory demyelinating neuropathy, rheumatoid arthritis, psoriasis, inflammatory bowel disease (e.g., Crohn disease and colitis ulcerosa), Sjogren's syndrome, Behcet's disease, multiple sclerosis, systemic lupus erythematosus, lupus nephritis, discoid lupus erythematosus, Castleman's disease, ankylosing spondylitis, polymyositis, dermatomyositis, polyarteritis nodosa, mixed connective tissue disease, scleroderma, lupus erythematosus profundus, chronic thyroiditis, Graves' disease, autoimmune gastritis, type I and type II diabetes mellitus, autoimmune hemolytic anemia, autoimmune neutropenia, thrombocytopenia, atopic dermatitis, chronic active hepatitis, myasthenia gravis, graft versus host disease, Addison's disease, abnormal immune response, arthritis, dermatitis, radiodermatitis, primary biliary cirrhosis, etc.
(3) Metabolic, liver, or kidney diseases: amyloid neuropathy (TTR and PALB), amyloidosis (APOA1, APP, AAA, CVAP, AD1, GSN, FGA, LYZ, TTR, and PALB), non-alcoholic steatohepatitis and hepatic fibrosis (COL1A1), hepatic cirrhosis (KRT18, KRT8, CIRH1A, NAIC, TEX292, and KIAA1988), cystic fibrosis (CFTR, ABCC7, CF, and MRP7), glycogen storage disease (SLC2A2, GLUT2, G6PC, G6PT, G6PT1, GAA, LAMP2, LAMPB, AGL, GDE, GBE1, GYS2, PYGL, and PERM), hepatocellular adenoma (TCF1, HFN1A, and MODY3), hepatic failure (SCOD1 and SCO1), hepatic lipase deficiency (LIPC), hepatoblastoma (CTNNB1, PDFGRL, PDGRL, PRLTS, AXIN1, AXIN, TP53, P53, LFS1, IGF2R, MPRI, MET, CASP8, and MCH5), medullary cystic kidney disease (UMOD, HNFJ, FJHN, MCKD2, and ADMCKD2), phenyl ketonuria (PAH, PKU1, QDPR, DHPR, and PTS), polycystic kidney and liver diseases (FCYT, PKHD1, APRKD, PDK1, PDK2, PDK4, PDKTS, PRKCSH, G19P1, PCLD, and SEC63), Hunter syndrome, lysosome disease, Fabry's disease, Pompe's disease, Gaucher's disease, mucopolysaccharidosis, hypoparathyroidism, Wilson's disease, etc.
(4) Neurological diseases: ALS (SOD1, ALS2, STEX, FUS, TARDBP, and VEGF), Alzheimer's disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, and AD3), autism (BZRAP1, MDGA2, GLO1, MECP2, RTT, PPMX, MRX16, MRX79, NLGN3, NLGN4, KIAA1260, and AUTSX2), fragile X syndrome (FMR2, FXR1, FXR2, and mGLUR5), Huntington's disease (HD, IT15, PRNP, PRIP, JPH3, JP3, HDL2, TBP, and SCA17), Parkinson's disease (NR4A2, NURR1, NOT, TINUR, SNCAIP, TBP, SCA17, SNCA, NACP, PARK1, PARK4, DJ1, DBH, and NDUFV2), Rett syndrome (MECP2, RTT, PPMX, MRX16, MRX79, CDKL5, and STK9), schizophrenia (GSK3, 5-HTT, COMT, DRD, SLC6A3, DAOA, and DTNBP1), secretase-related disorder (APH-1), etc.
(5) Eye diseases: age-related macular degeneration (Abcr, Ccl2, cp, Timp3, cathepsin D, Vldlr, and Ccr2), cataract (CRYAA, CRYA1, CRYBB2, CRYB2, PITX3, BFSP2, CP49, CP47, PAX6, AN2, MGDA, CRYBA1, CRYB1, CRYGC, CRYG3, CCL, LIM2, MP19, CRYGD, CRYG4, BSFP2, CP49, CP47, HSF4, CTM, MIP, AQP0, CRYAB, CRYA2, CTPP2, CRYBB1, CRYGD, CRYG4, CRYA1, GJA8, CX50, CAE1, GJA3, CX46, CZP3, CAE3, CCM1, CAM, and KRIT1), corneal opacity (APOA1, TGFB1, CSD2, CDGG1, CSD, BIGH3, CDG2, TASTD2, TROP2, M1S1, VSX1, RINX, PPCD, PPD, KTCN, COL8A2, FECD, PPCD2, PIP5K3, and CFD), cornea plana congenita familiares (KERA and CNA2), glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, and GLC3A), Leber's congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, and LCA3), macular dystrophy (ELOVL4, ADMD, STGD2, STGD3, RDS, RP7, PRPH2, PRPH, AVMD, AOFMD, and VMD2), etc.
(6) Noplastic diseases: malignant tumor, neovascular glaucoma, infantile hemangioma, hereditary angioedema, multiple myeloma, chronic sarcoma, metastatic melanoma, Kaposi's sarcoma, vascular proliferation, cachexia, metastasis of breast cancer, etc., cancers (e.g., colorectal cancer (e.g., familial colorectal cancer, hereditary non-polyposis colorectal cancer, and gastrointestinal stromal tumor), lung cancer (e.g., non-small cell lung cancer, small-cell lung cancer, and malignant mesothelioma), mesothelioma, pancreatic cancer (e.g., ductal pancreatic cancer), stomach cancer (e.g., papillary adenocarcinoma, mucous adenocarcinoma, and adenosquamous carcinoma), breast cancer (e.g., invasive ductal breast cancer, noninvasive ductal breast cancer, and inflammatory breast cancer), ovarian cancer (e.g., epithelial ovarian cancer, extragonadal germ cell tumor, ovarian germ cell tumor, and ovarian tumor of low malignant potential), prostate cancer (e.g., hormone-dependent prostate cancer and hormone-independent prostate cancer), liver cancer (e.g., primary liver cancer and extrahepatic bile duct cancer), thyroid cancer (e.g., medullary thyroid cancer), kidney cancer (e.g., renal cell cancer and transitional cell cancer of the renal pelvis and ureter), uterine cancer, brain tumor (e.g., pineal astrocytoma, pilocytic astrocytoma, diffuse astrocytoma, and anaplastic astrocytoma), melanoma, sarcoma, bladder cancer, blood cancer, etc. including multiple myeloma, pituitary adenoma, glioma, acoustic schwannoma, retinal sarcoma, throat cancer, voice box cancer, tongue cancer, thymoma, esophageal cancer, duodenal cancer, colon cancer, rectal cancer, hepatocellular carcinoma, pancreatic endocrine tumor, bile duct cancer, gallbladder cancer, penis cancer, ureter cancer, testicular tumor, vulval cancer, uterine cervical cancer, uterine body cancer, uterine sarcoma, trophoblastic disease, vaginal cancer, skin cancer, mycosis fungoides, basal cell tumor, soft tissue sarcoma, malignant lymphoma, Hodgkin's disease, myelodysplastic syndrome, adult T cell leukemia, chronic myeloproliferative disease, pancreatic endocrine tumor, fibrous histiocytoma, leiomyosarcoma, rhabdomyosarcoma, and cancer of unknown primary origin), leukemia (e.g., acute leukemia (e.g., acute lymphatic leukemia and acute myeloid leukemia), chronic leukemia (e.g., chronic lymphatic leukemia and chronic myeloid leukemia), and myelodysplastic syndrome), uterine sarcoma (e.g., mixed mesodermal tumor, uterine leiomyosarcoma, and endometrial stromal tumor), myelofibrosis, etc.
(7) Other diseases: IgA nephropathy, aplastic anemia, sarcoidosis, Williams syndrome, Marfan syndrome, muscular dystrophy, spinocerebellar degeneration, hypoparathyroidism, pemphigus, pemphigoid, amyotrophic lateral sclerosis, schistorrhachis, hypertrophic cardiomyopathy, idiopathic thrombocytopenic purpura, ankylosing spondylitis, osteomalacia, dermatomyositis, IgG4-related disease, Usher syndrome, Apert syndrome, Alport's syndrome, Angelman syndrome, West syndrome, spinal muscular atrophy, Werner's syndrome, Osler-Weber-Rendu disease, Crouzon syndrome, Creutzfeldt-Jakob disease, POEMS syndrome, prion disease, Shy-Drager syndrome, Charcot-Marie-Tooth disease, Sturge-Weber syndrome, Stevens-Johnson syndrome, SMON, Sotos syndrome, Dravet syndrome, Noonan syndrome, Buerger's disease, Hirschsprung disease, Pfeiffer syndrome, tetralogy of Fallot, phenyl ketonuria, Prader-Willi syndrome, porphyria, mitochondrial disease, maple syrup urine disease, familial hypercholesterolemia, familial Mediterranean fever, Kabuki syndrome, fulminant hepatitis, tuberous sclerosis complex, polyarteritis nodosa, thrombotic thrombocytopenic purpura, microscopic polyangiitis, primary sclerosing cholangitis, primary biliary cholangitis, eosinophilic sinusitis, Takayasu arteritis, osteogenesis imperfecta, mixed connective-tissue disease, optic neuromyelitis, autoimmune hepatitis, autoimmune hemolytic anemia, xeroderma pigmentosum, progressive supranuclear palsy, adult Still's disease, syringomyelia, congenital myopathy, generalized scleroderma, multiple system atrophy, aortitis syndrome, corticobasal degeneration, biliary atresia, fatal familial insomnia, toxic epidermal necrolysis, idiopathic interstitial pneumonia, chondrodystrophia foetalis, pustular psoriasis, pulmonary arterial hypertension, inclusion body myositis, chronic inflammatory demyelinating polyneuropathy, chronic active Epstein-Barr virus infection, retinitis pigmentosa, Cushing disease, familial chronic pyoderma, autosomal dominant polycystic kidney, 1p36 deletion syndrome, 22q11.2 deletion syndrome, HTLV-1-associated myelopathy, Aicardi syndrome, Weaver syndrome, granulomatosis with polyangiitis, Ehlers-Danlos syndrome, Emanuel syndrome, Klippel-Trenanay-Weber syndrome, Cockayne syndrome, Costello syndrome, Coffin-Siris syndrome, Coffin-Lowry syndrome, Smith-Magenis syndrome, thanatophoric dysplasia, Tangier disease, CHARGE syndrome, Budd-Chiari syndrome, peroxisomal disease, epilepsy with myoclonic absence, Moebius syndrome, Menkes disease, lymphangioleiomyomatosis, Rubinstein-Taybi syndrome, Leber hereditary optic neuropathy, subacute sclerosing panencephalitis, ossification of ligamentum flavum, familial benign chronic pemphigus, oculocutaneous albinism, giant cell arteritis, ossification of posterior longitudinal ligament, coexisting cervical and lumbar spinal stenosis, hyper IgD syndrome, relapsing polychondritis, tricuspid valve atresia, congenital ichthyosis, polysplenia syndrome, pseudoxanthoma elasticum, delayed endolymphatic hydrops, Nakajo-Nishimura syndrome, hypophosphatasia, idiopathic portal hypertension, Nasu-Hakola disease, acute encephalitis with refractory, repetitive partial seizures, urea cycle defect, pulmonary alveolar proteinosis, paroxysmal nocturnal hemoglobinuria, pachydermoperiostosis, bronchiolitis obliterans, Arima syndrome, acute encephalopathy with biphasic seizures and late reduced diffusion, Epstein syndrome, fanconi anemia, 4p deletion syndrome, 5p deletion syndrome, Ullrich disease, Occipital horn syndrome, Carney complex, galactose-1-phosphate uridyltransferase deficiency, Galloway-Mowat syndrome, Mowat-Wilson syndrome, Young-Simpson syndrome, Landau-Kleffner syndrome, Rothmund-Thomson syndrome, pyogenic sterile arthritis, pyoderma gangrenosum and acne syndrome, interstitial cystitis, giant lymphatic malformation, eosinophilic granulomatosis with polyangiitis, autoimmune hemorrhaphilia XIII, congenital dyserythropoietic anemia, septo-optic dysplasia, branchio-oto-renal syndrome, etc.

The composition of the present invention as a medicament can be produced by a method known in the field of pharmaceutical formulation techniques using a pharmaceutically acceptable carrier. Examples of the dosage form of the medicament can include preparations for parenteral administration (e.g., solutions such as injections) supplemented with auxiliary agents commonly used such as a buffer and/or a stabilizer, and local preparations such as ointments, creams, solutions or plasters supplemented with pharmaceutical carriers commonly used.

The composition of the present invention can be used for transferring an active ingredient to many types of cells, tissues or organs. Examples of the cell to which the composition of the present invention can be applied include spleen cell, nerve cell, glia cell, pancreatic B cell, bone marrow cell, mesangial cell, Langerhans cell, epidermal cell, epithelial cell, endothelial cell, fibroblast, fiber cell, muscle cell (e.g., skeletal muscle cell, cardiac muscle cell, myoblast, and satellite cell), fat cell, immune cell (e.g., macrophage, T cell, B cell, natural killer cell, mast cell, neutrophil, basophil, eosinophil, monocyte, and megakaryocyte), synovial cell, cartilage cell, bone cell, osteoblast, osteoclast, mammary gland cell, liver cell or stromal cell, egg cell, sperm cell, or progenitor cell inducible to differentiate into these cells, stem cell (including e.g., induced pluripotent stem cell (iPS cell) and embryonic stem cell (ES cell)), blood cell, oocyte, and fertilized egg. Examples of the tissue or the organ to which the composition of the present invention can be applied include every tissue or organ where the cell described above are present, for example, brain, each site of brain (e.g., olfactory bulb, amygdaloid nucleus, cerebral basal ganglia, hippocampus, thalamus, hypothalamus, hypothalamic nucleus, cerebral cortex, oblong medulla, cerebellum, occipital lobe, frontal lobe, temporal lobe, putamen, caudate nucleus, corpus callosum, and substantia nigra), spinal cord, pituitary gland, stomach, pancreas, kidney, liver, genital gland, thyroid gland, gallbladder, bone marrow, adrenal gland, skin, muscle, lung, gastrointestinal tract (e.g., large intestine and small intestine), vascular vessel, heart, thymus gland, spleen, submandibular gland, peripheral blood, peripheral blood cell, prostate, testis, orchis, ovary, placenta, uterus, bone, joint and skeletal muscle. These cells, tissues or organs may be cancer cells, cancer tissues or the like resulting from malignant transformation.

The compound, the lipid particle and the composition of the present invention can be used with stability, low toxicity and safety. In the case of using the composition of the present invention *in vivo* or as a medicament, the composition can be administered to a recipient (e.g., a human or a nonhuman mammal (e.g., a mouse, a rat, a hamster, a rabbit, a cat, a dog, cattle, sheep, or a monkey) (preferably a human)) such that an effective amount of the nucleic acid is delivered to a targeted cell.

In the case of using the composition of the present invention *in vivo* or as a medicament, the composition can be safely administered as a pharmaceutical preparation, for example, tablets (including sugar-coated tablets, film-coated tablets, sublingual tablets, and orally disintegrating tablets), powders, granules, capsules (including soft capsules and microcapsules), solutions, troches, syrups, emulsions, suspensions, injections (e.g., subcutaneous injections, intravenous injections, intramuscular injections, and intraperitoneal injections), external preparations (e.g., transnasal administration preparations, transdermal preparations, ointments), suppositories (e.g., rectal suppositories and vaginal suppositories), pellets, transnasal agents, transpulmonary agents (inhalants), or drops, through an oral or parenteral (e.g., local, rectal, or intravenous administration) route. These preparations may be release-controlled preparations such as quick-release preparations or sustained-release preparations (e.g., sustained-release microcapsules).

Hereinafter, a method for producing the compound of the present invention will be described.

A starting material or a reagent used in each step in the production method given below and the obtained compound may each form a salt. Examples of such a salt include the same as the aforementioned salt of the compound of the present invention.

When the compound obtained in each step is a free compound, this compound can be converted to a salt of interest by a known method. On the contrary, when the compound obtained in each step is a salt, this salt can be converted to a free form or another type of salt of interest by a known method.

The compound obtained in each step may be used in the next reaction in the form of its reaction solution or after being obtained as a crude product. Alternatively, the compound obtained in each step can be isolated and/or purified from the reaction mixture by a separation approach such as concentration, crystallization, recrystallization, distillation, solvent extraction, fractionation, or chromatography according to a routine method.

If a starting material or a reagent compound for each step is commercially available, the commercially available product can be used directly.

In the reaction of each step, the reaction time may differ depending on the reagent or the solvent used and is usually 1 minute to 72 hours, preferably 10 minutes to 48 hours, unless otherwise specified.

In the reaction of each step, the reaction temperature may differ depending on the reagent or the solvent used and is usually -78°C to 300°C, preferably -78°C to 150°C, unless otherwise specified.

In the reaction of each step, the pressure may differ depending on the reagent or the solvent used and is usually 1 atm to 20 atm, preferably 1 atm to 3 atm, unless otherwise specified.

In the reaction of each step, a microwave synthesis apparatus, for example, Initiator manufactured by Biotage Japan Ltd., may be used. The reaction temperature may differ depending on the reagent or the solvent used and is usually room temperature to 300°C, preferably room temperature to 250°C, more preferably 50°C to 250°C, unless otherwise specified. The reaction time may differ depending on the reagent or the solvent used and is usually 1 minute to 48 hours, preferably 1 minute to 8 hours, unless otherwise specified.

In the reaction of each step, the reagent is used at 0.5 equivalents to 20 equivalents, preferably 0.8 equivalents to 5 equivalents, based on the substrate, unless otherwise specified. In the case of using the reagent as a catalyst, the reagent is used at 0.001 equivalents to 1 equivalent, preferably 0.01 equivalents to 0.2 equivalents, based on the substrate. When the reagent also serves as a reaction solvent, the reagent is used in the amount as the solvent.

In the reaction of each step, this reaction is carried out without a solvent or by dissolution or suspension in an appropriate solvent, unless otherwise specified. Specific examples of the solvent include solvents described in Examples and the following:
alcohols: methanol, ethanol, isopropanol, isobutanol, tert-butyl alcohol, 2-methoxyethanol, and the like;
ethers: diethyl ether, diisopropyl ether, diphenyl ether, tetrahydrofuran, 1,2-dimethoxyethane, cyclopentyl methyl ether, and the like;
aromatic hydrocarbons: chlorobenzene, toluene, xylene, and the like;
saturated hydrocarbons: cyclohexane, hexane, heptane, and the like;
amides: N,N-dimethylformamide, N-methylpyrrolidone, and the like;
halogenated hydrocarbons: dichloromethane, carbon tetrachloride, and the like;
nitriles: acetonitrile and the like;
sulfoxides: dimethyl sulfoxide and the like;
aromatic organic bases: pyridine and the like;
acid anhydrides: acetic anhydride and the like;
organic acids: formic acid, acetic acid, trifluoroacetic acid, and the like;
inorganic acids: hydrochloric acid, sulfuric acid, and the like;
esters: ethyl acetate, acetic acid isopropyl ester, and the like;
ketones: acetone, methyl ethyl ketone, and the like; and
water.

Two or more of these solvents may be used as a mixture at an appropriate ratio.

In the case of using a base in the reaction of each step, for example, the following base or a base described in Examples is used:
inorganic bases: sodium hydroxide, potassium hydroxide, magnesium hydroxide, and the like;
basic salts: sodium carbonate, calcium carbonate, sodium bicarbonate, and the like;
organic bases: triethylamine, diethylamine, N,N-diisopropylethylamine, pyridine, 4-dimethylaminopyridine, N,N-dimethylaniline, 1,4-diazabicyclo[2.2.2]octane, 1,8-diazabicyclo[5.4.0]-7-undecene, imidazole, piperidine, and the like;
metal alkoxides: sodium ethoxide, potassium tert-butoxide, sodium tert-butoxide, and the like;
alkali metal hydrides: sodium hydride, and the like;
metal amides: sodium amide, lithium diisopropylamide, lithium hexamethyldisilazide, and the like; and
organic lithiums: n-butyllithium, sec-butyllithium, and the like.

In the case of using an acid or an acidic catalyst in the reaction of each step, for example, the following acid or acidic catalyst or an acid or an acidic catalyst described in Examples is used:
inorganic acids: hydrochloric acid, sulfuric acid, nitric acid, hydrobromic acid, phosphoric acid, and the like;
organic acids: acetic acid, trifluoroacetic acid, citric acid, p-toluenesulfonic acid, 10-camphorsulfonic acid, and the like; and
Lewis acids: boron trifluoride-diethyl ether complex, zinc iodide, anhydrous aluminum chloride, anhydrous zinc chloride, anhydrous iron chloride, and the like.

The reaction of each step is carried out according to a known method, for example, a method described in The Fifth Series of Experimental Chemistry, Vol. 13 to Vol. 19 (edited by The Chemical Society of Japan); Shin Jikken Kagaku Koza (in Japanese, translated title: New Experimental Chemistry), Vol. 14 to Vol. 15 (edited by The Chemical Society of Japan); Seimitsu Yuki Kagaku (in Japanese, translated title: Precise Organic Chemistry, original title: Reaktionen und Synthesen im organisch-chemischen Praktikum und Forschungslaboratorium) Revised, 2nd Ed. (L. F. Tietze, Th. Eicher, Nankodo Co., Ltd.); Organic Named Reactions; The Reaction Mechanism and Essence, Revised (Hideo Tougo, Kodansha Ltd.); Organic Syntheses Collective Volume I to VII (John Wiley & Sons, Inc.); Modern Organic Synthesis in the Laboratory: A Collection of Standard Experimental Procedures (Jie Jack Li, Oxford University Press); Comprehensive Heterocyclic Chemistry III, Vol. 1 to Vol. 14 (Elsevier Japan KK); Strategic Applications of Named Reactions in Organic Synthesis (translated by Kiyoshi Tomioka, published by Kagaku-Dojin Publishing Company, Inc.); Comprehensive Organic Transformations (VCH Publishers, Inc.) (1989), etc., or a method described in Examples, unless otherwise specified.

In each step, the protection or deprotection reaction of a functional group is carried out according to a known method, for example, a method described in "Protective Groups in Organic Synthesis, 4th Ed." (Theodora W. Greene, Peter G. M. Wuts), Wiley-Interscience (2007); "Protecting Groups, 3rd Ed." (P.J. Kocienski), Thieme Medical Publishers (2004), etc., or a method described in Examples.

Examples of a protective group for a hydroxy group or a phenolic hydroxy group in an alcohol or the like include: ether-type protective groups such as methoxy methyl ether, benzyl ether, p-methoxy benzyl ether, t-butyl dimethyl silyl ether, t-butyl diphenyl silyl ether, and tetrahydropyranyl ether; carboxylic acid ester-type protective groups such as acetic acid ester; sulfonic acid ester-type protective groups such as methanesulfonic acid ester; and carbonic acid ester-type protective groups such as t-butyl carbonate.

Examples of a protective group for a carbonyl group in an aldehyde include: acetal-type protective groups such as dimethylacetal; and cyclic acetal-type protective groups such as cyclic 1,3-dioxane.

Examples of a protective group for a carbonyl group in a ketone include: ketal-type protective groups such as dimethylketal; cyclic ketal-type protective groups such as cyclic 1,3-dioxane; oxime-type protective groups such as O-methyloxime; and hydrazone-type protective groups such as N,N-dimethylhydrazone.

Examples of a protective group for a carboxyl group include: ester-type protective groups such as methyl ester and benzyl ester; and amide-type protective groups such as N,N-dimethylamide.

Examples of a protective group for a thiol include: ether-type protective groups such as benzyl thioether; and ester-type protective groups such as thioacetic acid ester, thiocarbonate, and thiocarbamate.

Examples of a protective group for an amino group or an aromatic heterocyclic ring such as imidazole, pyrrole, or indole include: carbamate-type protective groups such as benzyl carbamate; amide-type protective groups such as acetamide; alkylamine-type protective groups such as N-triphenylmethylamine; and sulfonamide-type protective groups such as methanesulfonamide.

These protective groups can be removed (deprotection) by use of a known method, for example, a method using an acid, a base, ultraviolet light, hydrazine, phenylhydrazine, sodium N-methyldithiocarbamate, tetrabutylammonium fluoride, palladium acetate, or trialkylsilyl halide (e.g., trimethylsilyl iodide and trimethylsilyl bromide), or a reduction method.

In the case of carrying out reduction reaction in each step, examples of the reducing agent used include: metal hydrides such as lithium aluminum hydride, sodium triacetoxyborohydride, sodium cyanoborohydride, diisobutyl aluminum hydride (DIBAL-H), sodium borohydride, and tetramethylammonium triacetoxyborohydride; boranes such as a borane-tetrahydrofuran complex; Raney nickel; Raney cobalt; hydrogen; and formic acid. A catalyst such as palladium-carbon, Raney nickel or Raney cobalt can be used in the presence of hydrogen or formic acid.

In the case of carrying out oxidation reaction in each step, examples of the oxidizing agent used include: peracids such as m-chloroperbenzoic acid (MCPBA), hydrogen peroxide, and t-butyl hydroperoxide; perchlorates such as tetrabutylammonium perchlorate; chlorates such as sodium chlorate; chlorites such as sodium chlorite; periodates such as sodium periodate; high-valent iodine reagents such as iodosylbenzene; reagents having manganese, such as manganese dioxide and potassium permanganate; leads such as lead tetraacetate; reagents having chromium, such as pyridinium chlorochromate (PCC), pyridinium dichromate (PDC), and Jones reagents; halogen compounds such as N-bromosuccinimide (NBS); oxygen; ozone; a sulfur trioxide-pyridine complex; osmium tetroxide; selenium dioxide; and 2,3-dichloro-5,6-dicyano-1,4-benzoquinone (DDQ).

In the case of carrying out radical cyclization reaction in each step, examples of the radical initiator used include: azo compounds such as azobisisobutyronitrile (AIBN); water-soluble radical initiators such as 4-4'-azobis-4-cyanopentanoic acid (ACPA); triethylboron in the presence of air or oxygen; and benzoyl peroxide. Examples of the radical reaction agent used include tributylstannane, tristrimethylsilylsilane, 1,1,2,2-tetraphenyldisilane, diphenylsilane, and samarium iodide.

In the case of carrying out Wittig reaction in each step, examples of the Wittig reagent used include alkylidenephosphoranes. The alkylidenephosphoranes can be prepared by a known method, for example, the reaction between a phosphonium salt and a strong base.

In the case of carrying out Horner-Emmons reaction in each step, examples of the reagent used include: phosphonoacetic acid esters such as methyl dimethylphosphonoacetate and ethyl diethylphosphonoacetate; and bases such as alkali metal hydrides and organic lithiums.

In the case of carrying out Friedel-Crafts reaction in each step, examples of the reagent used include a Lewis acid and an acid chloride or an alkylating agent (e.g., alkyl halides, alcohols, and olefins). Alternatively, an organic acid or an inorganic acid may be used instead of the Lewis acid, and an acid anhydride such as acetic anhydride may be used instead of the acid chloride.

In the case of carrying out aromatic nucleophilic substitution reaction in each step, a nucleophile (e.g., amines and imidazole) and a base (e.g., basic salts and organic bases) are used as reagents.

In the case of carrying out nucleophilic addition reaction using a carbanion, nucleophilic 1,4-addition reaction (Michael addition reaction) using a carbanion, or nucleophilic substitution reaction using a carbanion in each step, examples of the base used for generating the carbanion include organic lithiums, metal alkoxides, inorganic bases, and organic bases.

In the case of carrying out Grignard reaction in each step, examples of the Grignard reagent include: aryl magnesium halides such as phenyl magnesium bromide; and alkyl magnesium halides such as methyl magnesium bromide and isopropyl magnesium bromide. The Grignard reagent can be prepared by a known method, for example, the reaction between alkyl halide or aryl halide and metal magnesium with ether or tetrahydrofuran as a solvent.

In the case of carrying out Knoevenagel condensation reaction in each step, an active methylene compound flanked by two electron-attracting groups (e.g., malonic acid, diethyl malonate, and malononitrile) and a base (e.g., organic bases, metal alkoxides, and inorganic bases) are used as reagents.

In the case of carrying out Vilsmeier-Haack reaction in each step, phosphoryl chloride and an amide derivative (e.g., N,N-dimethylformamide) are used as reagents.

In the case of carrying out azidation reaction of alcohols, alkyl halides, or sulfonic acid esters in each step, examples of the azidating agent used include diphenylphosphorylazide (DPPA), trimethylsilylazide, and sodium azide. In the case of azidating, for example, alcohols, a method using diphenylphosphorylazide and 1,8-diazabicyclo[5.4.0]undec-7-ene (DBU), a method using trimethylsilylazide and a Lewis acid, or the like can be used.

In the case of carrying out reductive amination reaction in each step, examples of the reducing agent used include sodium triacetoxyborohydride, sodium cyanoborohydride, hydrogen, and formic acid. When the substrate is an amine compound, examples of the carbonyl compound used include p-formaldehyde as well as aldehydes such as acetaldehyde, and ketones such as cyclohexanone. When the substrate is a carbonyl compound, examples of the amines used include: primary amine such as ammonia and methylamine; and secondary amine such as dimethylamine.

In the case of carrying out Mitsunobu reaction in each step, azodicarboxylic acid esters (e.g., diethyl azodicarboxylate (DEAD) and diisopropyl azodicarboxylate (DIAD)) and triphenylphosphine are used as reagents.

In the case of carrying out esterification reaction, amidation reaction, or ureation reaction in each step, examples of the reagent used include: an acyl halide form of ester form, acid chloride, acid bromide, and the like; and activated carboxylic acids such as an acid anhydride, an active ester form, a sulfuric acid ester form, and the like. Examples of the activator for carboxylic acid include: carbodiimide condensing agents such as 1-ethyl-3-(3-dimethylaminopropyl)carbodiimide hydrochloride (EDCI), N,N'-dicyclohexylcarbodiimide (DCC); triazine condensing agents such as 4-(4,6-dimethoxy-1,3,5-triazin-2-yl)-4-methylmorpholinium chloride-n-hydrate (DMT-MM); carbonic acid ester condensing agents such as 1,1-carbonyldiimidazole (CDI); diphenylphosphorylazide (DPPA); benzotriazol-1-yloxy-trisdimethylaminophosphonium salt (BOP reagent); 2-chloro-1-methyl-pyridinium iodide (Mukaiyama reagent); thionyl chloride; lower alkyl haloformate such as ethyl chloroformate; O-(7-azabenzotriazol-1-yl)-N,N,N',N'-tetramethyluronium hexafluorophosphate (HATU); sulfuric acid; or combinations thereof. An additive such as 1-hydroxybenzotriazole (HOBt), N-hydroxysuccinimide (HOSu), or dimethylaminopyridine (DMAP) may be further added for the reaction.

In the case of carrying out coupling reaction in each step, examples of the metal catalyst used include: palladium compounds such as palladium(II) acetate, tetrakis(triphenylphosphine)palladium(0), dichlorobis(triphenylphosphine)palladium(II), dichlorobis(triethylphosphine)palladium(II), tris(dibenzylideneacetone)dipalladium(0), 1,1'-bis(diphenylphosphino)ferrocene palladium(II) chloride, and palladium(II) acetate; nickel compounds such as tetrakis(triphenylphosphine)nickel(0); rhodium compounds such as tris(triphenylphosphine)rhodium(III) chloride; cobalt compounds; copper compounds such as copper oxide and copper(I) iodide; and platinum compounds. A base may be further added for the reaction. Examples of such a base include inorganic bases and basic salts.

In the case of carrying out thiocarbonylation reaction in each step, diphosphorus pentasulfide is typically used as a thiocarbonylating agent. A reagent having a 1,3,2,4-dithiadiphosphetane-2,4-disulfide structure such as 2,4-bis(4-methoxyphenyl-1,3,2,4-dithiadiphosphetane-2,4-disulfide (Lawesson's reagent) may be used instead of diphosphorus pentasulfide.

In the case of carrying out Wohl-Ziegler reaction in each step, examples of the halogenating agent used include N-iodosuccinimide, N-bromosuccinimide (NBS), N-chlorosuccinimide (NCS), bromine, and sulfuryl chloride. The reaction can be accelerated by the further addition of heat, light, a radical initiator such as benzoyl peroxide or azobisisobutyronitrile for the reaction.

In the case of carrying out halogenation reaction of a hydroxy group in each step, examples of the halogenating agent used include a hydrohalic acid and an acid halide of an inorganic acid, specifically, hydrochloric acid, thionyl chloride, and phosphorus oxychloride for chlorination, and 48% hydrobromic acid for bromination. Also, a method for obtaining an alkyl halide form from an alcohol by the action of triphenylphosphine and carbon tetrachloride or carbon tetrabromide or the like may be used. Alternatively, a method for synthesizing an alkyl halide form through 2-stage reactions involving the conversion of an alcohol to sulfonic acid ester and the subsequent reaction with lithium bromide, lithium chloride, or sodium iodide may be used.

In the case of carrying out Arbuzov reaction in each step, examples of the reagent used include: alkyl halides such as ethyl bromoacetate; and phosphites such as triethyl phosphite and tri(isopropyl) phosphite.

In the case of carrying out sulfone-esterification reaction in each step, examples of the sulfonylating agent used include methanesulfonyl chloride, p-toluenesulfonyl chloride, methanesulfonic anhydride, p-toluenesulfonic anhydride, and trifluoromethanesulfonic anhydride.

In the case of carrying out hydrolysis reaction in each step, an acid or a base is used as a reagent. In the case of carrying out acid hydrolysis reaction of t-butyl ester, formic acid, triethylsilane, or the like may be added in order to reductively trap a by-product t-butyl cation.

In the case of carrying out dehydration reaction in each step, examples of the dehydrating agent used include sulfuric acid, diphosphorus pentoxide, phosphorus oxychloride, N,N'-dicyclohexylcarbodiimide, alumina, and polyphosphoric acid.

In the case of performing decarboxylation reaction in each step, an acid may be used. Examples of the acid include inorganic acids and organic acids.

In the case of performing nucleophilic substitution reaction in each step, a base may be used. Examples of the base include metal alkoxides, inorganic bases and organic bases.

The compound (I) can be produced by, for example, a production method given below. In the present invention, the compound (I) having the desired structure can be synthesized by using a starting material appropriate for the structure of the compound (I) of interest, particularly, for esterification. The salt of the compound (I) can be obtained by appropriate mixing with an inorganic base, an organic base, an organic acid, or a basic or acidic amino acid.

Scheme 1 given below shows an exemplary production method. In the scheme 1, P¹ represents a protective group, and the other symbols are the same as in the formula (I) (the same holds true for other schemes related to the scheme 1). In the scheme 1, L represents a leaving group, and R^{A}, R^{B} and R^{C} may be appropriately interchanged with each other.

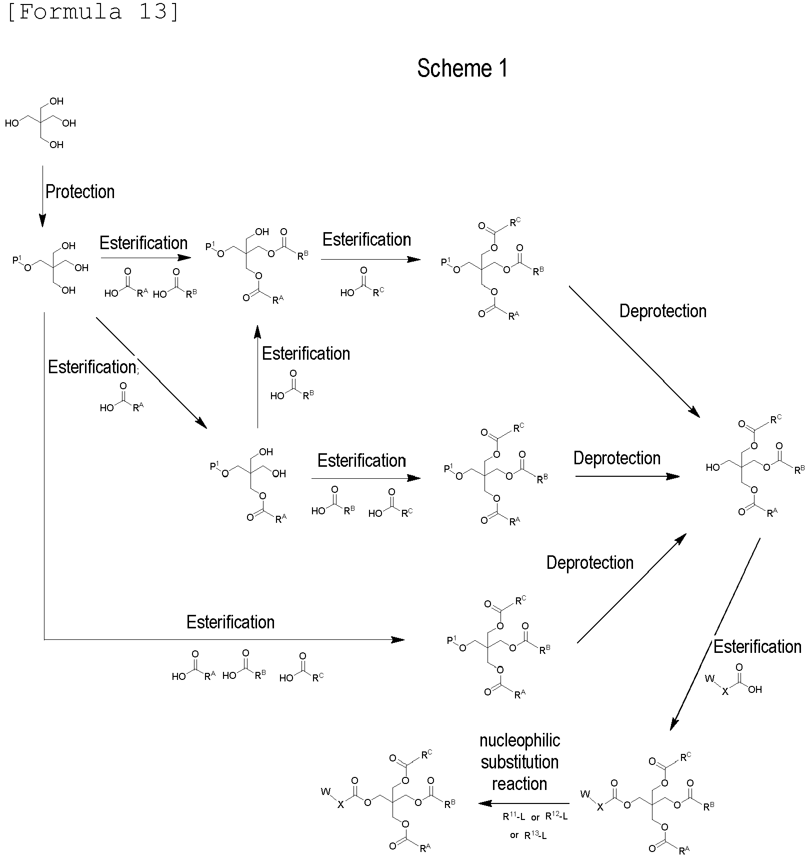

Scheme 2 given below shows a method for synthesizing a compound (R^{A1} or R^{B1}-COOH) for use in esterification when R^{A} and/or R^{B} of the compound (I) in the scheme 1 is R^{A/B} group 1 (an optionally substituted C₁₋₁₇ alkyl group, an optionally substituted C₃₋₁₇ alkenyl group, or an optionally substituted C₁₅₋₁₇ alkadienyl group), i.e., R^{A} and/or R^{B} is R^{A1} and/or R^{B1} described in the formula (II) or the formula (III). In the scheme 2, P², P³, P⁴ and P⁵ each represent a protective group, R^{a}, R^{b}, R^{c}, R^{d} and R^{g} each represent H, an optionally substituted alkyl group, an optionally substituted alkenyl group, or an optionally substituted alkadienyl group and partially constitute R^{A} and/or R^{B}. The numbers of carbon atoms, substituents and structures of R^{a}, R^{b}, R^{c}, R^{d} and R^{g} are appropriately adjusted according to the structure of R^{A} and/or R^{B} of interest.

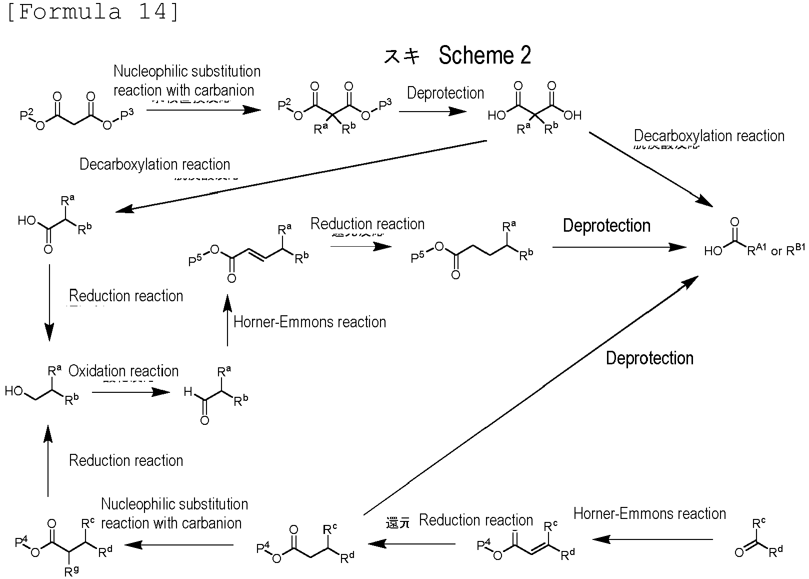

Scheme 3 given below shows a method for synthesizing a compound (R^{A2} or R^{B2}-COOH) for use in esterification when R^{A} and/or R^{B} of the compound (I) in the scheme 1 is R^{A/B} group 2 (-R³-C(O)O-R⁴ or -R³-OC(O)-R⁴), i.e., R^{A} and/or R^{B} is R^{A2} and/or R^{B2} described in the formula (II) or the formula (III), and a compound (R^{C}-COOH) for use in the esterification of R^{C} in the compound (I). In the scheme 3, P⁶ and P⁷ each represent a protective group.

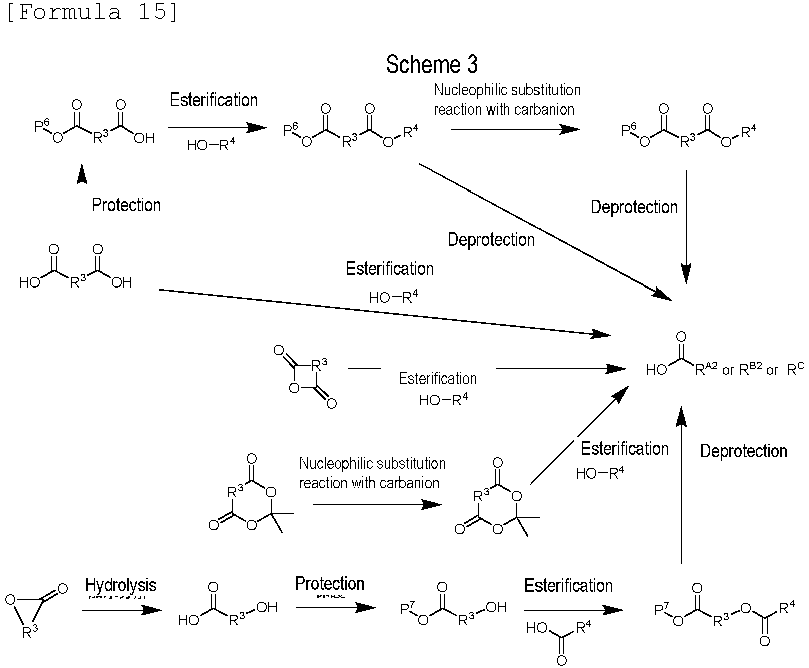

Scheme 4 given below shows a method for synthesizing a compound (R⁴-OH) for use in esterification in the scheme 3. In the scheme 4, P⁴ represents a protective group, and R^{a}, R^{b}, R^{c} and R^{d} each represent H, an optionally substituted alkyl group, an optionally substituted alkenyl group, or an optionally substituted alkadienyl group and partially constitute R⁴. The numbers of carbon atoms, substituents and structures of R^{a}, R^{b}, R^{c} and R^{d} are appropriately adjusted according to the structure of R⁴ of interest.

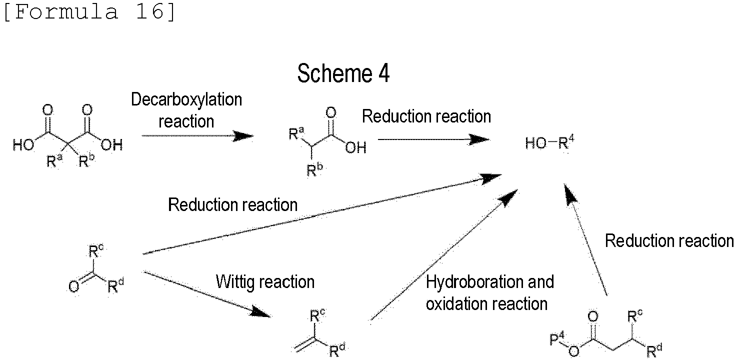

Scheme 5 given below shows a method for synthesizing a compound (R⁴-COOH) for use in esterification in the scheme 3. In the scheme 5, P⁴ and P⁵ each represent a protective group, and R^{a}, R^{b}, R^{c} and R^{d} each represent H, an optionally substituted alkyl group, an optionally substituted alkenyl group, or an optionally substituted alkadienyl group and partially constitute R⁴. The numbers of carbon atoms, substituents and structures of R^{a}, R^{b}, R^{c} and R^{d} are appropriately adjusted according to the structure of R⁴ of interest.

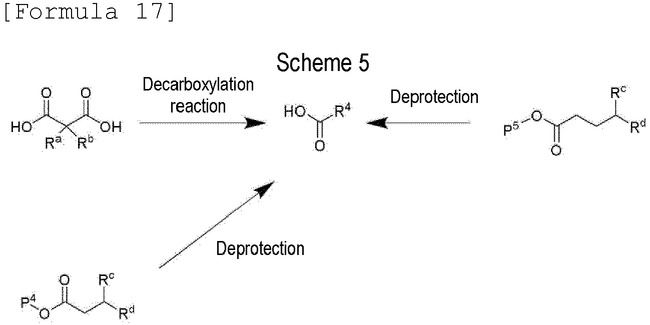

Scheme 6 given below shows a method for synthesizing a compound (W-X-COOH) for use in esterification in the scheme 1. In the scheme 6, R^{e} represents an optionally substituted alkylene group, L represents a leaving group, and P⁸ represents a protective group. The number of carbon atoms, substituent and structure of R^{e} are appropriately adjusted according to the structure of X of interest.

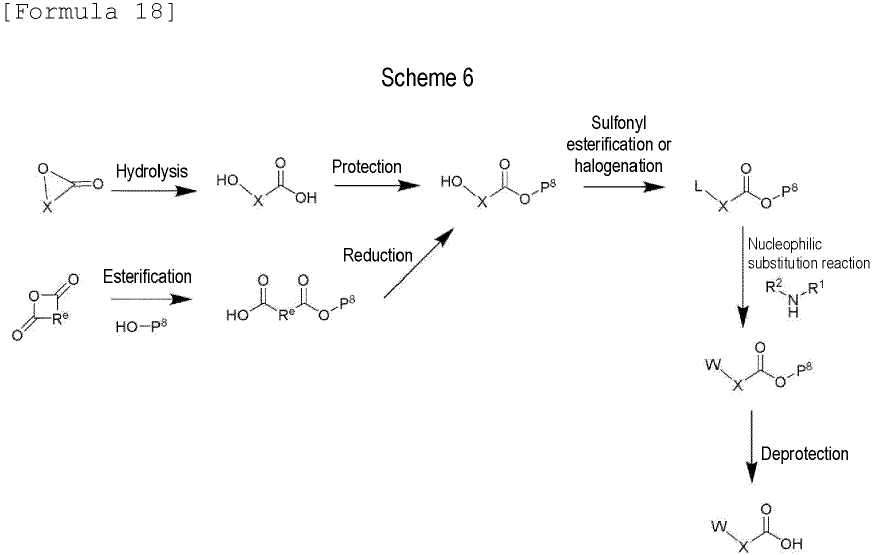

Scheme 7 given below shows a method for synthesizing the compound (I). In the scheme 7, R^{f} represents an optionally substituted alkylene group, and P⁹ represents a protective group. The number of carbon atoms, substituent and structure of R^{f} are appropriately adjusted according to the structure of W of interest.

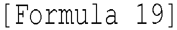

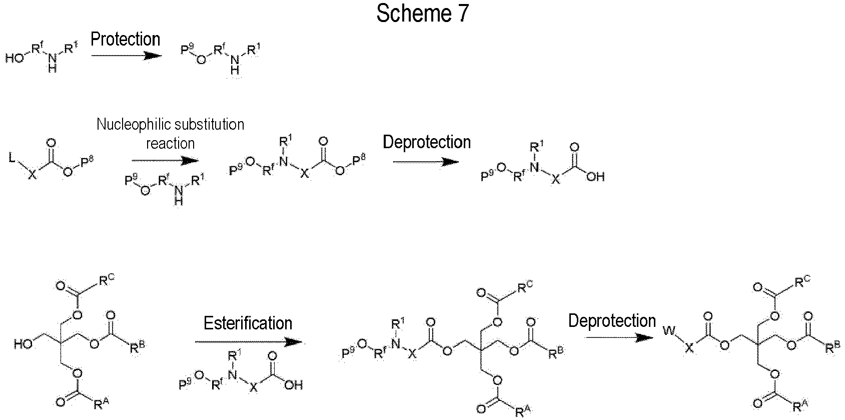

Hereinafter, methods for producing a lipid particle containing the compound of the present invention, and a composition for nucleic acid transfer (transfection) containing the lipid particle and a nucleic acid as an active ingredient will be described.

The lipid particle of the present invention can be produced by a known method for preparing lipid particles from lipid components after mixing of the compound (compound (I) or a salt thereof) of the present invention as a cationic lipid, if necessary, with an additional lipid component. For example, the (mixed) lipid component described above is dissolved in an organic solvent, and the resulting solution in the organic solvent can be mixed (e.g., by an emulsification method) with water or a buffer solution to produce a lipid particle dispersion. The mixing can be performed using a microfluidic mixing system (e.g., NanoAssemblr apparatus (Precision NanoSystems Inc.)). The obtained lipid particle may be subjected to desalting or dialysis and sterile filtration. If necessary, pH adjustment or osmotic pressure adjustment may be carried out.

The compound of the present invention may assume a plurality of structures by combinations of definitions of respective symbols (substituents, etc.) in the formula (I). In the production of the lipid particle, one type of compound (I) or salt thereof having a specific structure may be used as the compound of the present invention, or plural types of compounds (I) or salts thereof differing in structure may be used as a mixture.

Examples of the "additional lipid component" include the structured lipids mentioned above, for example, sterols, phospholipids, and polyethylene glycol lipids. The "additional lipid component" is used at, for example, 0.008 to 4 mol, based on 1 mol of the compound of the present invention. The compound of the present invention is preferably mixed, for use, with the additional lipid component (particularly, cholesterol, phosphatidylcholine and polyethylene glycol lipid). In the case of using the compound of the present invention and an additional lipid component in a mixture, a preferred embodiment is a mixture of 1 to 4 mol of the compound of the present invention, 0 to 3 mol of the sterol, 0 to 2 mol of the phospholipid and 0 to 1 mol of the polyethylene glycol lipid. In the case of using the compound of the present invention and an additional lipid component in a mixture, a more preferred embodiment is a mixture of 1 to 1.5 mol of the compound of the present invention, 0 to 1.25 mol of the sterol, 0 to 0.5 mol of the phospholipid and 0 to 0.125 mol of the polyethylene glycol lipid.

The concentration of the compound of the present invention or the mixture of the compound of the present invention with the additional lipid component in the solution in the organic solvent described above is preferably 0.5 to 100 mg/mL.

Examples of the organic solvent include methanol, ethanol, 1-propanol, 2-propanol, 1-butanol, tert-butanol, acetone, acetonitrile, N,N-dimethylformamide, dimethyl sulfoxide, and mixtures thereof. The organic solvent may contain 0 to 20% of water or a buffer solution.

Examples of the buffer solution include acidic buffer solutions (e.g., an acetate buffer solution, a citrate buffer solution, a 2-morpholinoethanesulfonic acid (MES) buffer solution, and a phosphate buffer solution), and neutral buffer solutions (e.g., a 4-(2-hydroxyethyl)-1-piperazineethanesulfonic acid (HEPES) buffer solution, a tris(hydroxymethyl)aminomethane (Tris) buffer solution, a phosphate buffer solution, and phosphate-buffered saline (PBS)).

In the case of carrying out the mixing using a microfluidic mixing system, 1 to 5 parts by volume of water or a buffer solution are preferably mixed with 1 part by volume of the solution in the organic solvent. In the system, the flow rate of the mixed solution (the mixed solution of the solution in the organic solvent with water or the buffer solution) is, for example, 0.01 to 20 mL/min, preferably 0.1 to 10 mL/min, and the temperature is, for example, 5 to 60°C, preferably 15 to 45°C.

The composition of the present invention can be produced as a lipid particle dispersion containing a nucleic acid by adding the nucleic acid to water or a buffer solution for the production of the lipid particle or a lipid particle dispersion. The nucleic acid is preferably added such that the concentration of the nucleic acid in water or the buffer solution is, for example, 0.01 to 20 mg/mL, preferably 0.05 to 2.0 mg/mL.

Alternatively, the composition of the present invention may be produced as a lipid particle dispersion containing an active ingredient by mixing the lipid particle or a lipid particle dispersion with the nucleic acid or an aqueous solution thereof by a known method. The lipid particle dispersion can be prepared by dispersing the lipid particle in an appropriate dispersion medium. The aqueous solution of the active ingredient can be prepared by dissolving the active ingredient in an appropriate solvent.

The content of the compound of the present invention in the composition of the present invention excluding the dispersion medium and the solvent is usually 10 to 70% by weight, preferably 40 to 70% by weight.

The content of the nucleic acid in the composition of the present invention excluding the dispersion medium and the solvent is usually 0.1 to 25% by weight, preferably 1 to 20% by weight.

The dispersion medium in the lipid particle dispersion or the dispersion containing the composition can be replaced with water or a buffer solution by dialysis. The dialysis is carried out at 4°C to room temperature using an ultrafiltration membrane having a molecular weight cutoff of 10 to 20 K. The dialysis may be performed repetitively. The replacement of the dispersion medium may employ tangential flow filtration (TFF). After the replacement of the dispersion medium, if necessary, pH adjustment or osmotic pressure adjustment may be carried out. Examples of the pH adjuster include sodium hydroxide, citric acid, acetic acid, triethanolamine, sodium hydrogen phosphate, sodium dihydrogen phosphate, and potassium dihydrogen phosphate. Examples of the osmotic pressure adjuster include: inorganic salts such as sodium chloride, potassium chloride, sodium hydrogen phosphate, potassium hydrogen phosphate, sodium dihydrogen phosphate, and potassium dihydrogen phosphate; polyols such as glycerol, mannitol, and sorbitol; and sugars such as glucose, fructose, lactose, and sucrose. The pH is usually adjusted to 6.5 to 8.0, preferably 7.0 to 7.8. The osmotic pressure is preferably adjusted to 250 to 350 Osm/kg.

The composition of the present invention may contain, if necessary, a component other than the lipid particle and the nucleic acid. Examples of such a component include appropriate amounts of a stabilizer and an antioxidant.

Examples of the stabilizer include, but are not particularly limited to, sugars such as glycerol, mannitol, sorbitol, lactose, and sucrose.

Examples of the antioxidant include ascorbic acid, uric acid, cysteine, tocopherol homologs (vitamin E, four isomers tocopherol α, β, γ, and δ, etc.), EDTA, and cysteine.

Hereinafter, methods for analyzing a lipid particle containing the compound of the present invention, and a composition containing the lipid particle and a nucleic acid as an active ingredient will be described.

The particle size of the lipid particle (in the composition) can be measured by a known approach. For example, the particle size can be calculated as a Z-average particle size by the cumulant analysis of an autocorrelation function using a particle size measurement apparatus Zetasizer Nano ZS (Malvern Instruments) based on a dynamic light scattering measurement technique. The particle size (average particle size) of the lipid particle (in the composition) is, for example, 10 to 200 nm, preferably 60 to 170 nm.

The concentration and rate of encapsulation of the nucleic acid (e.g., siRNA or mRNA) in the composition of the present invention can be measured by a known approach. For example, the nucleic acid is fluorescently labeled using Quant-iT(TM) RiboGreen(R) (Invitrogen Corp.), and the fluorescence intensity can be measured to determine the concentration and the rate of encapsulation. The concentration of the nucleic acid in the composition can be calculated using a calibration curve prepared from aqueous nucleic acid solutions having known concentrations. The rate of encapsulation can be calculated on the basis of the difference in fluorescence intensity between the presence and absence of addition of Triton-X 100 (surfactant for disrupting the lipid particle). The concentration of the nucleic acid in the composition refers to the total concentration of a nucleic acid encapsulated in the lipid particle and an unencapsulated nucleic acid. The rate of encapsulation refers to the ratio of the nucleic acid encapsulated in the lipid particle to all nucleic acids in the composition.

### Examples

The present invention will be described in more detail with reference to Examples, Production Examples and Test Examples given below. However, the present invention is not limited by these examples. Changes or modifications may be made therein without departing from the scope of the present invention.

In the examples given below, the term "room temperature" usually refers to a temperature of approximately 10°C to approximately 35°C. The ratio shown in a mixed solvent refers to a volume ratio, unless otherwise specified. The term "%" refers to % by weight, unless otherwise specified.

In the examples, elution for column chromatography was performed under observation by TLC (thin layer chromatography), unless otherwise specified. In the TLC observation, a solvent used as an eluting solvent in column chromatography was used as a developing solvent. Detection adopted a UV detector, and a TLC chromogenic reagent was used, if necessary, for observation. In silica gel column chromatography, the term "NH" means that an aminopropylsilane-bound silica gel was used, and the term "Diol" means that a 3-(2,3-dihydroxypropoxy)propylsilane-bound silica gel was used. In preparative HPLC (high-performance liquid chromatography), the term "C18" means that an octadecyl-bound silica gel was used. The ratio shown in an eluting solvent refers to a volume ratio, unless otherwise specified.

¹H NMR was measured by Fourier transform NMR. ¹H NMR was analyzed using ACD/SpecManager (trade name) software or the like. Very gentle peaks of protons of, for example, a hydroxyl group and an amino group may not be described.

MS was measured by LC/MS or MALDI/TOFMS. ESI, APCI, or MALDI was used as an ionization method. CHCA was used as a matrix. Measured values (Found) are shown in data. A molecular ion peak is usually observed, however, the peak observed may be of a polyvalent ion or a fragment ion. For a salt, the peak observed is usually of a free molecular ion, a cationic species, an anionic species or a fragment ion.

The following abbreviations are used in the examples given below.
MS: mass spectrum
M: molar concentration
N: normality
CDCl₃: deuterated chloroform
DMSO-d₆: deuterated dimethyl sulfoxide
¹H NMR: proton nuclear magnetic resonance
LC/MS: liquid chromatograph-mass spectrometer
ESI: electrospray ionization
APCI: atmospheric pressure chemical ionization
MALDI: matrix-assisted laser desorption/ionization
TOFMS: time-of-flight mass spectrometry
CHCA: α-cyano-4-hydroxycinnamic acid
DCM: dichloromethane
DMA: N,N-dimethylacetamide
DMF: N,N-dimethylformamide
THF: tetrahydrofuran
MeOH: methanol
EtOH: ethanol
DMAP: 4-dimethylaminopyridine
TBAF: tetrabutylammonium fluoride
TBAB: tetrabutylammonium bromide
DIBAL-H: diisobutyl aluminum hydride
DBU:1,8-diazabicyclo[5,4,0]undec-7-ene
TEA: triethylamine
EDCI: 1-ethyl-3-(3-dimethylaminopropyl)carbodiimide hydrochloride
DCC: N,N'-dicyclohexylcarbodiimide
HOBt: 1-hydroxybenzotriazole

Compounds of Examples 1 to 173 (compounds 1 to 173) shown in Table 1 below were produced in accordance with the production methods described in the present specification. More specific production methods will be given below as to Examples 3, 13, 22, 33, 39, 51, 70, 73, 93, 115, 119, 121, 132 and 173 among these Examples.

**[Table 1-1]**

| Example No. | IUPAC name | Structural formula | MS:m/z ([M+H]⁺ or [M+2H]²⁺ or [M]⁺) |
|---|---|---|---|
| 1 | 1,1'-[2-{[(9-Butoxy-9-oxononanoyl)oxy]methyl}-2-({[4-(dimethylamino)butanoyl]oxy}m ethyl)propane-1,3-diyl] 9,9'-dibutyl dinonanedioate | 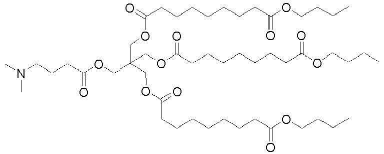 | 928.6 |
| 2 | 1,1'-[2-{[(9-Butoxy-9-oxononanoyl)oxy]methyl}-2-({[5-(dimethylamino)pentanoyl]oxy} methyl)propane-1,3-diyl] 9,9'-dibutyl dinonanedioate | 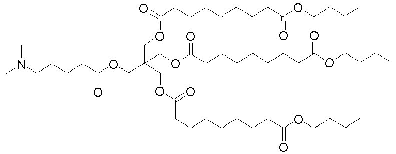 | 942.7 |
| 3 | 1,1'-2-{[(N,N-Dimethyl-beta-alanyl)oxy]methyl}-2-({[6-(heptyloxy)-6-oxohexanoyl]oxy}methyl)propan e-1,3-diyl] 6,6'-diheptyl dihexanedioate | 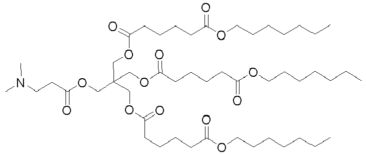 | 914.6 |
| 4 | 1,1'-12-({[4-(Dimethylamino)butanoyl]oxy}m ethyl)-2-({[6-(heptyloxy)-6-oxohexanoyl]oxy}methyl)propan e-1,3-diyl] 6,6'-diheptyl dihexanedioate | 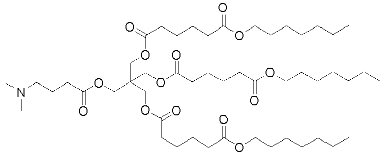 | 928.6 |
| 5 | 1,1'-[2-(1[4-(Diethylamino)butanoyl]oxy}met hyl)-2-({[6-(heptyloxy)-6-oxohexanoyl]oxy}methyl)propan e-1,3-diyl] 6,6'-diheptyl dihexanedioate | 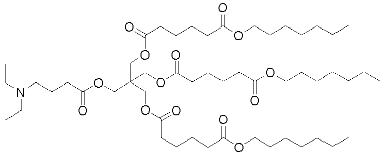 | 479.0 |
| 6 | 1,1'-2-{[(N,N-Dimethyl-beta-alanyl)oxy]methyl}-2-({[6-(octyloxy)-6-oxohexanoyl]oxy}methyl)propan e-1,3-diyl] 6,6'-dioctyl dihexanedioate | 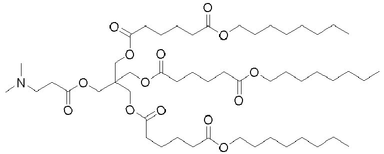 | 478.9 |

**[Table 1-2]**

| | | | |
|---|---|---|---|
| 7 | 1,1'-[2-({[4-(Dimethylamino)butanoyl]oxy}m ethyl)-2-({[6-(octyloxy)-6-oxohexanoyl]oxy}methyl)propan e-1,3-diyl] 6,6'-dioctyl dihexanedioate | 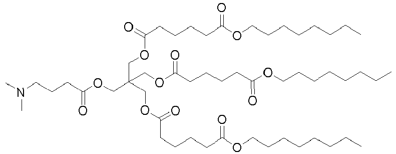 | 486.0 |
| 8 | 1,1'-[2-(1[4-(Diethylamino)butanoyl]oxy}met hyl)-2-({[6-(octyloxy)-6-oxohexanoyl]oxy}methyl)propan e-1,3-diyl] 6,6'-dioctyl dihexanedioate | 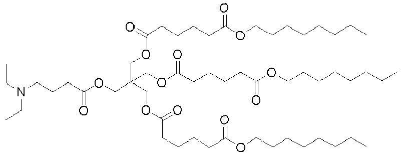 | 5000 |
| 9 | 1,1'-(2-1[(N,N-Dimethyl-beta-alanyl)oxy]methyl}-2-{[(12-methoxy-12-oxododecanoyl)oxy]methyl}prop ane-1,3-diyl) 12,12'-dimethyl didodecanedioate | 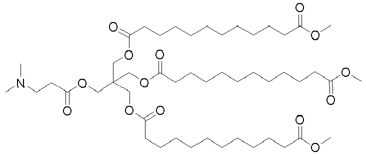 | 914.6 |
| 10 | 1,1'-[2-({[4-(Dimethylamino)butanoyl]oxy}m ethyl)-2-{[(12-methoxy-12-oxododecanoyl)oxy]methyl}prop ane-1,3-diyl] 12,12'-dimethyl didodecanedioate | 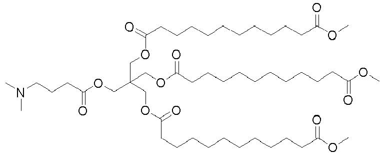 | 928.6 |
| 11 | 1,1'-[2-(1[4-(Diethylamino)butanoyl]oxy}met hyl)-2-{[(12-methoxy-12-oxododecanoyl)oxy]methyl}prop ane-1,3-diyl] 12,12'-dimethyl didodecanedioate | 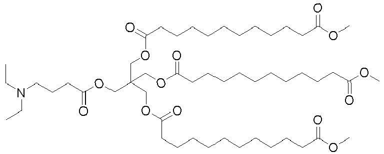 | 956.6 |
| 12 | 1,1'-(2-{[(N,N-Dimethyl-beta-alanyl)oxy]methyl}-2-{[(3-pentyloctanoyl)oxy]methyl}prop ane-1,3-diyl) 6,6'-diheptyl dihexanedioate | 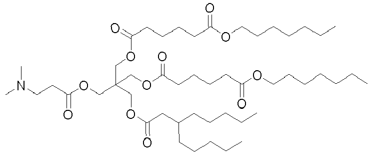 | 442.9 |
| 13 | 1,1'-[2-({[4-(Dimethylamino)butanoyl]oxy}m ethyl)-2-{[(3-pentyloctanoyl)oxy]methyl}prop ane-1,3-diyl] 6,6'-diheptyl dihexanedioate | 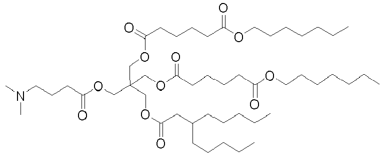 | 449.9 |

**[Table 1-3]**

| | | | |
|---|---|---|---|
| 14 | 1,1'-[2-({[4-(Diethylamino)butanoyl]oxy}met hyl)-2-{[(3-pentyloctanoyl)oxy]methyl}prop ane-1,3-diyl] 6,6'-diheptyl dihexanedioate | 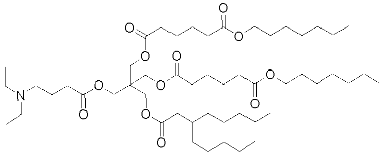 | 464.0 |
| 15 | 2-{[(N,N-Dimethyl-beta-alanyl)oxy]methyl}-3-[(3-pentyloctanoyl)oxy]-2-{[(3-pentyloctanoyl)oxy]methyl}propy l heptyl hexanedioate | 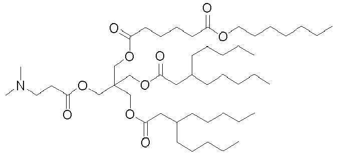 | 854.7 |
| 16 | 2-({[4-(Dimethylamino)butanoyl]oxy}m ethyl)-3-[(3-pentyloctanoyl)oxy]-2-{[(3-pentyloctanoyl)oxy]methyl}propy l heptyl hexanedioate | 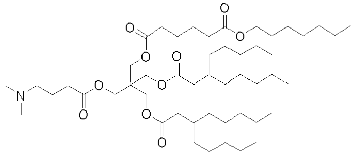 | 868.7 |
| 17 | 2-({[4-(Diethylamino)butanoyl]oxy}met hyl)-3-[(3-pentyloctanoyl)oxy]-2-{[(3-pentyloctanoyl)oxy]methyl}propy l heptyl hexanedioate | 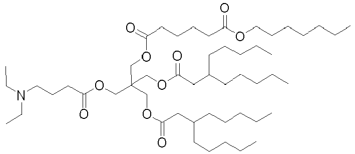 | 896.7 |
| 18 | 2-({[5-(Dimethylamino)pentanoyl]oxy} methyl)-3-[(3-pentyloctanoyl)oxy]-2-{[(3-pentyloctanoyl)oxy]methyl}propy l heptyl hexanedioate | 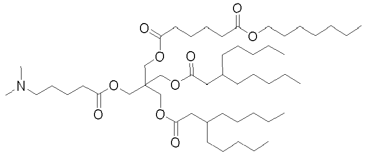 | 882.7 |
| 19 | Butyl 2-{[(N,N-dimethyl-beta-alanyl)oxy]methyl}-3-[(3-pentyloctanoyl)oxy]-2-{[(3-pentyloctanoyl)oxy]methyl}propy l hexanedioate | 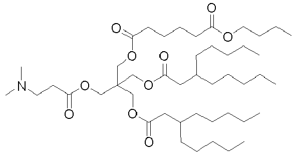 | 812.6 |
| 20 | Butyl 2-({[4-(dimethylamino)butanoyl]oxy}m ethyl)-3-[(3-pentyloctanoyl)oxy]-pentyloctanoyl)oxy]methyl}propy l hexanedioate | 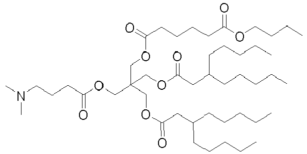 | 826.7 |

**[Table 1-4]**

| | | | |
|---|---|---|---|
| 21 | Butyl 2-({[4-(diethylamino)butanoyl]oxy}met hyl)-3-[(3-pentyloctanoyl)oxy]-2-{[(3-pentyloctanoyl)oxy]methyl}propy l hexanedioate | 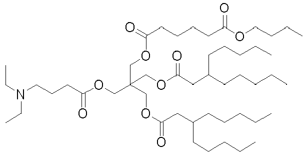 | 854.7 |
| 22 | {[2-{[(N,N-Dimethyl-beta-alanyl)oxy]methyl}-2-({[5-(octanoyloxy)pentanoyl]oxy}met hyl)propane-1,3-diyl]bis(oxy)-5-oxopentane-5,1-diyl} dioctanoate | 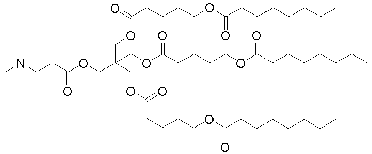 | 914.6 |
| 23 | {[2-({[4-(Dimethylamino)butanoyl]oxy}m ethyl)-2-({[5-(octanoyloxy)pentanoyl]oxy}met hyl)propane-1,3-diyl]bis(oxy)-5-oxopentane-5,1-diyl} dioctanoate | 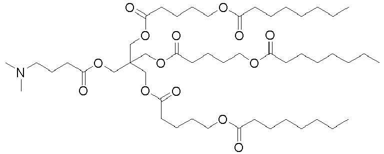 | 928.6 |
| 24 | {[2-({[4-(Diethylamino)butanoyl]oxy}met hyl)-2-({[5-(octanoyloxy)pentanoyl]oxy}met hyl)propane-1,3-diyl]bis(oxy)-5-oxopentane-5,1-diyl} dioctanoate | 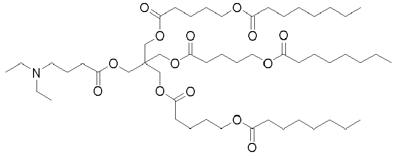 | 956.6 |
| 25 | {[2-({[4-(Dimethylamino)butanoyl]oxy}m ethyl)-2-{[(3-pentyloctanoyl)oxy]methyl}prop ane-1,3-diyl]bis(oxy)-5-oxopentane-5,1-diyl} dioctanoate | 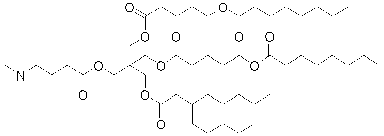 | 898.7 |
| 26 | 2-({[4-(Dimethylamino)butanoyl]oxy}m ethyl)-2-({[5-(octanoyloxy)pentanoyl]oxy}met hyl)propane-1,3-diyl bis(3-pentyloctanoate) | 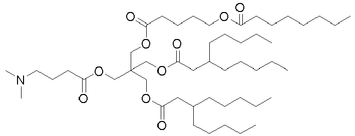 | 868.7 |
| 27 | 1,1'-(2-{[(N,N-Dimethyl-beta-alanyl)oxy]methyl}-2-{[(3-propyldecanoyl)oxy]methyl}prop ane-1,3-diyl) 6,6'-diheptyl dihexanedioate | 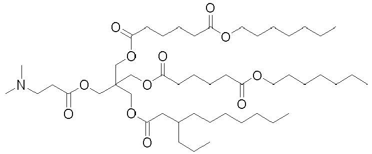 | 884.7 |

**[Table 1-5]**

| | | | |
|---|---|---|---|
| 28 | 1,1'-[2-({[4-(Dimethylamino)butanoyl]oxy}m ethyl)-2-{[(3-propyldecanoyl)oxy]methyl}prop ane-1,3-diyl] 6,6'-diheptyl dihexanedioate | 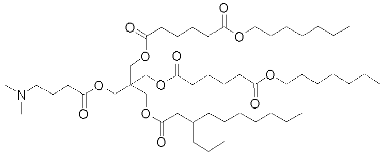 | 898.7 |
| 29 | 1,1'-[2-({[4-(Diethylamino)butanoyl]oxy}met hyl)-2-{[(3-propyldecanoyl)oxy]methyl}prop ane-1,3-diyl] 6,6'-diheptyl dihexanedioate | 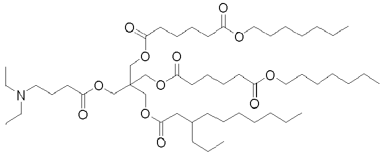 | 926.7 |
| 30 | 2-{[(N,N-Dimethyl-beta-alanyl)oxy]methyl}-3-[(3-propyldecanoyl)oxy]-2-{[(3-propyldecanoyl)oxy]methyl}prop yl heptyl hexanedioate | 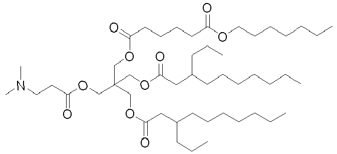 | 854.7 |
| 31 | 2-({[4-(Dimethylamino)butanoyl]oxy}m ethyl)-3-[(3-propyldecanoyl)oxy]-2-{[(3-propyldecanoyl)oxy]methyl}prop yl heptyl hexanedioate | 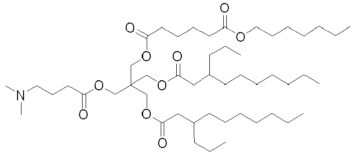 | 868.7 |
| 32 | 2-({[4-(Diethylamino)butanoyl]oxy}met hyl)-3-[(3-propyldecanoyl)oxy]-propyldecanoyl)oxy]methyl}prop yl heptyl hexanedioate | 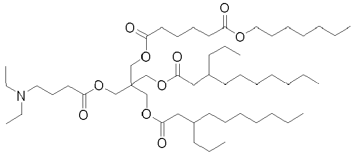 | 896.7 |
| 33 | 1,1'-Dibutyl 9,9'-[2-({[4-(dimethylamino)butanoyl]oxy}m ethyl)-2-{[(3-pentyloctanoyl)oxy]methyl}prop ane-1,3-diyl] dinonanedioate | 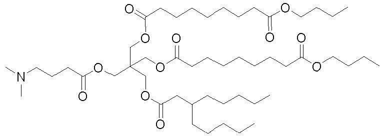 | 898.7 |
| 34 | Butyl 2-({[4-(dimethylamino)butanoyl]oxy}m ethyl)-3-[(3-pentyloctanoyl)oxy]-2-{[(3-pentyloctanoyl)oxy]methyl}propy I nonanedioate | 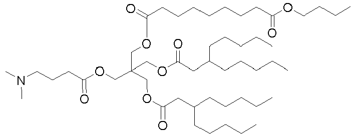 | 868.7 |
| 35 | 1,1'-[2-({[4-(Dimethylamino)butanoyl]oxy}m ethyl)-2-{[(3-pentyloctanoyl)oxy]methyl}prop ane-1,3-diyl] 9,9'-dimethyl dinonanedioate | 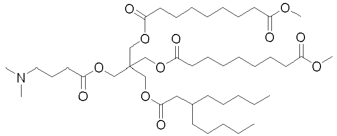 | 814.6 |

**[Table 1-6]**

| | | | |
|---|---|---|---|
| 36 | 2-({[4-(Dimethylamino)butanoyl]oxy}m ethyl)-3-[(3-pentyloctanoyl)oxy]-pentyloctanoyl)oxy]methyl}propy l methyl nonanedioate | 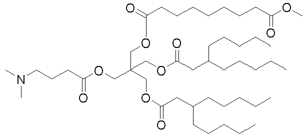 | 826.7 |
| 37 | 1,1'-{2-({[4-(Dimethylamino)butanoyl]oxy}m ethyl)-2-[({4-oxo-4-[(2-pentylheptyl)oxy]butanoyl}oxy)m ethyl]propane-1,3-diyl} 4,4'-bis(2-pentylheptyl) dibutanedioate | 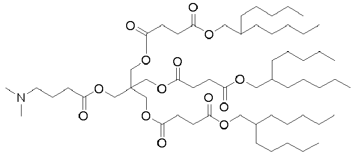 | 1054.8 |
| 38 | 1,1'-{2-({[5-(Dimethylamino)pentanoyl]oxy} methyl)-2-[({4-oxo-4-[(2-pentylheptyl)oxy]butanoyl}oxy)m ethyl]propane-1,3-diyl} 4,4'-bis(2-pentylheptyl) dibutanedioate | 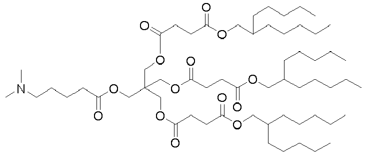 | 1068.8 |
| 39 | 1,1'-{2-({[4-(Dimethylamino)butanoyl]oxy}m ethyl)-2-[({4-oxo-4-[(undecan-6-yl)oxy]butanoyl}oxy)methyl]prop ane-1,3-diyl} 4,4'-diundecan-6-yl dibutanedioate | 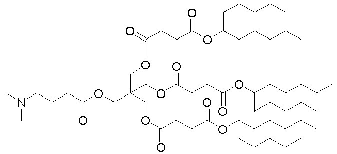 | 1012.7 |
| 40 | 1,1'-{2-({[5-(Dimethylamino)pentanoyl]oxy} methyl)-2-[({4-oxo-4-[(undecan-6-yl)oxy]butanoyl}oxy)methyl]prop ane-1,3-diyl} 4,4'-diundecan-6-yl dibutanedioate | 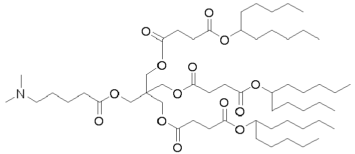 | 1026.7 |
| 41 | 1,1'-[2-({[4-(Dimethylamino)butanoyl]oxy}m ethyl)-2-({[6-(nonyloxy)-6-oxohexanoyl]oxy}methyl)propan e-1,3-diyl] 6,6'-dinonyl dihexanedioate | 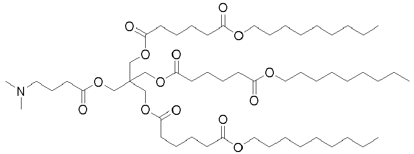 | 1012.7 |
| 42 | 1,1'-Didecyl 6,6'-[2-({[6-(dexyloxy)-6-oxohexanoyl]oxy}methyl)-2-({[4-(dimethylamino)butanoyl]oxy}m ethyl)propane-1,3-diyl] dihexanedioate | 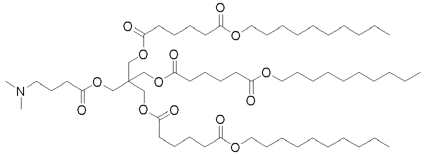 | 528.0 |

**[Table 1-7]**

| | | | |
|---|---|---|---|
| 43 | 1,1'-[2-({[4-(Dimethylamino)butanoyl]oxy}m ethyl)-2-{[(3-pentyloctanoyl)oxy]methyl}prop ane-1,3-diyl] 6,6'-dioctyl dihexanedioate | 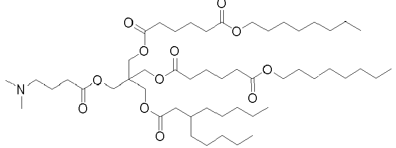 | 926.7 |
| 44 | 1,1'-[2-(1[4-(Dimethylamino)butanoyl]oxy}m ethyl)-2-{[(3-pentyloctanoyl)oxy]methyl}prop ane-1,3-diyl]6,6'-dinonyl dihexanedioate | 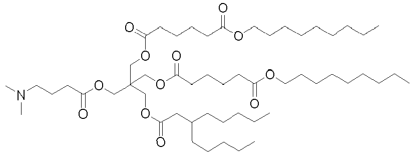 | 954.7 |
| 45 | 1,1'-Didecyl 6,6'-[2-({[4-(dimethylamino)butanoyl]oxy}m ethyl)-2-{[(3-pentyloctanoyl)oxy]methyl}prop ane-1,3-diyl] dihexanedioate | 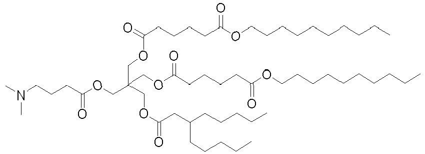 | 982.7 |
| 46 | 1,1'-[2-({[4-(Dimethylamino)butanoyl]oxy}m ethyl)-2-{[(3-pentyloctanoyl)oxy]methyl}prop ane-1,3-diyl] 6,6'-dihexyl dihexanedioate | 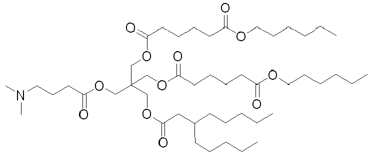 | 870.6 |
| 47 | 1,1'-[2-({[4-(Dimethylamino)butanoyl]oxy}m ethyl)-2-{[(3-pentyloctanoyl)oxy]methyl}prop ane-1,3-diyl] 6,6'-di-(4Z)-hept-4-en-1-yl dihexanedioate | 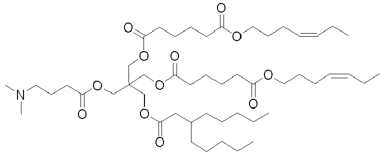 | 894.6 |
| 48 | 1,1'-[2-({[4-(Dimethylamino)butanoyl]oxy}m ethyl)-2-{[(3-pentyloctanoyl)oxy]methyl}prop ane-1,3-diyl] 6,6'-di-(3Z)-hept-3-en-1-yl dihexanedioate | 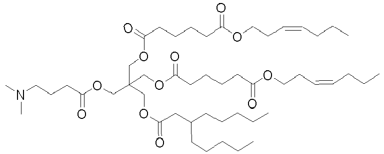 | 894.6 |
| 49 | 2-({[4-(Dimethylamino)butanoyl]oxy}m ethyl)-3-[(3-pentyloctanoyl)oxy]-pentyloctanoyl)oxy]methyl}propy l octyl hexanedioate | 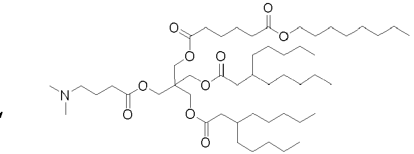 | 882.7 |

**[Table 1-8]**

| | | | |
|---|---|---|---|
| 50 | 2-({[4-(Dimethylamino)butanoyl]oxy}m ethyl)-3-[(3-pentyloctanoyl)oxy]-2-{[(3-pentyloctanoyl)oxy]methyl}propy l nonyl hexanedioate | 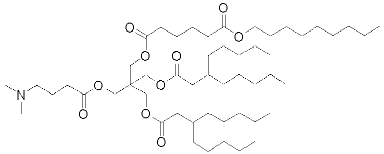 | 896.7 |
| 51 | Decyl 2-({[4-(dimethylamino)butanoyl]oxy}m ethyl)-3-[(3-pentyloctanoyl)oxy]-2-{[(3-pentyloctanoyl)oxy]methyl}propy l hexanedioate | 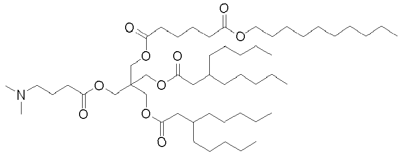 | 9107 |
| 52 | 2-({[4-(Dimethylamino)butanoyl]oxy}m ethyl)-3-[(3-pentyloctanoyl)oxy]-2-{[(3-pentyloctanoyl)oxy]methyl}propy l hexyl hexanedioate | 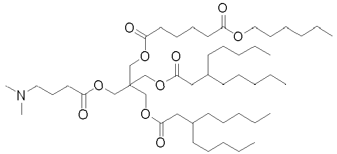 | 854.7 |
| 53 | 2-({[4-(Dimethylamino)butanoyl]oxy}m ethyl)-3-[(3-pentyloctanoyl)oxy]-2-{[(3-pentyloctanoyl)oxy]methyl}propy l (4Z)-hept-4-en-1-yl hexanedioate | 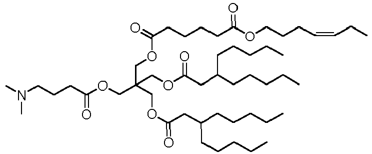 | 866.7 |
| 54 | 2-({[4-(Dimethylamino)butanoyl]oxy}m ethyl)-3-[(3-pentyloctanoyl)oxy]-2-{[(3-pentyloctanoyl)oxy]methyl}propy l (3Z)-hept-3-en-1-yl hexanedioate | 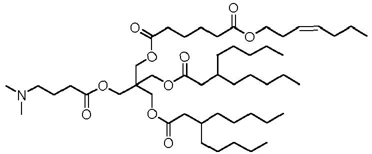 | 8667 |
| 55 | 1,1'-[2-({[4-(Dimethylamino)butanoyl]oxy}m ethyl)-2-({[9-oxo-9-(pentyloxy)nonanoyl]oxy}methyl )propane-1,3-diyl] 9,9'-dipentyl dinonanedioate | 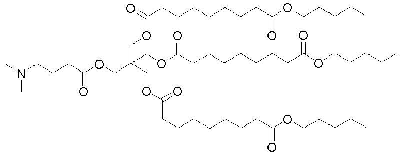 | 970.7 |
| 56 | 1,1'-[2-({[4-(Dimethylamino)butanoyl]oxy}m ethyl)-2-({[9-(hexyloxy)-9-oxononanoyl]oxy}methyl)propan e-1,3-diyl] 9,9'-dihexyl dinonanedioate | 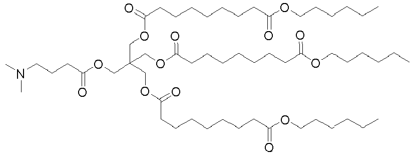 | 507.0 |

**[Table 1-9]**

| | | | |
|---|---|---|---|
| 57 | 1,1'-[2-({[4-(Dimethylamino)butanoyl]oxy }methyl)-2-({[9-(heptyloxy)-9-oxononanoyl]oxy}methyl)pro pane-1,3-diyl] 9,9'-diheptyl dinonanedioate | 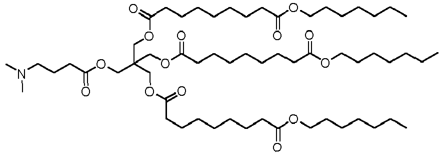 | 1054.7 |
| 58 | 1,1'-[2-({[4-(Dimethylamino)butanoyl]oxy }methyl)-2-{[(3-pentyloctanoyl)oxy]methyl }pr opane-1,3-diyl] 9,9'-dipentyl dinonanedioate | 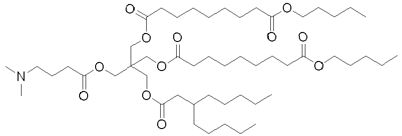 | 926.7 |
| 59 | 1,1'-[2-({[4-(Dimethylamino)butanoyl]oxy }methyl)-2-{[(3-pentyloctanoyl)oxy]methyl }pr opane-1,3-diyl] 9,9'-dihexyl dinonanedioate | 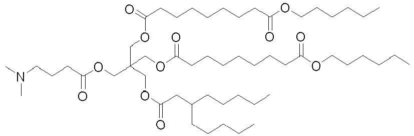 | 954.7 |
| 60 | 1,1'-[2-({[4-(Dimethylamino)butanoyl]oxy }methyl)-2-{[(3-pentyloctanoyl)oxy]methyl}pr opane-1,3-diyl] 9,9'-diheptyl dinonanedioate | 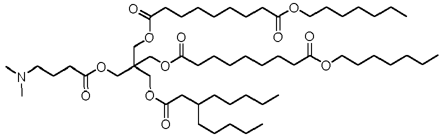 | 982.7 |
| 61 | 1,1'-[2-({[4-(Dimethylamino)butanoyl]oxy }methyl)-2-{[(3-pentyloctanoyl)oxy]methyl }pr opane-1,3-diyl] 9,9'-dipropyl dinonanedioate | 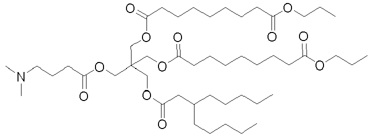 | 870.6 |
| 62 | 2-({[4-(Dimethylamino)butanoyl]oxy }methyl)-3-[(3-pentyloctanoyl)oxy]-2-{[(3-pentyloctanoyl)oxy]methyl }pr opyl pentyl nonanedioate | 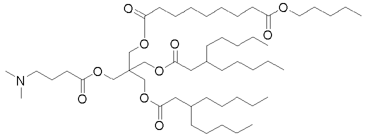 | 882.7 |
| 63 | 2-({[4-(Dimethylamino)butanoyl]oxy }methyl)-3-[(3-pentyloctanoyl)oxy]-2-{[(3-pentyloctanoyl)oxy]methyl}pr opyl hexyl nonanedioate | 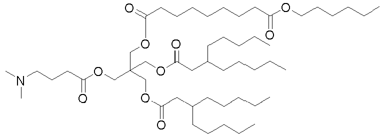 | 896.7 |
| 64 | 2-({[4-(Dimethylamino)butanoyl]oxy }methyl)-3-[(3-pentyloctanoyl)oxy]-2-{[(3-pentyloctanoyl)oxy]methyl }pr opyl heptyl nonanedioate | 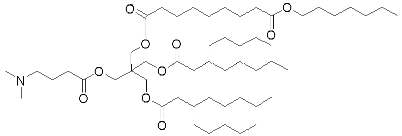 | 910.7 |

**[Table 1-10]**

| | | | |
|---|---|---|---|
| 65 | 2-({[4-(Dimethylamino)butanoyl]oxy }methyl)-3-[(3-pentyloctanoyl)oxy]-2-{[(3-pentyloctanoyl)oxy]methyl }pr opyl propyl nonanedioate | 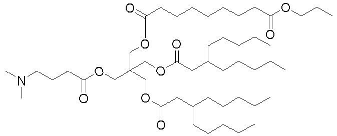 | 854.7 |
| 66 | 1,1'-[2-({[4-(Dimethylamino)butanoyl]oxy }methyl)-2-({[3-(octyloxy)propanoyl]oxy}met hyl)propane-1,3-diyl] 3,3'-dioctyl dipropanedioate | 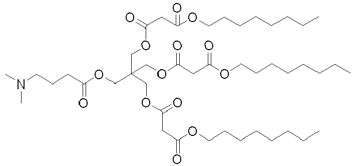 | 844.6 |
| 67 | 1,1'-[2-({[4-(Dimethylamino)butanoyl]oxy }methyl)-2-({[3-(nonyloxy)-3-oxopropanoyl]oxy}methyl)pro pane-1,3-diyl] 3,3'-dinonyl dipropanedioate | 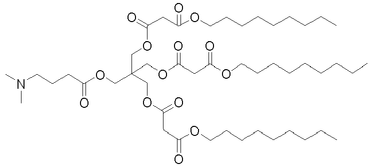 | 886.6 |
| 68 | 1, 1'-Didecyl 3,3'-[2-({[3-(decyloxy)-3-oxopropanoyl]oxy}methyl)-2-({[4-(dimethylamino)butanoyl]oxy }methyl)propane-1,3-diyl] dipropanedioate | 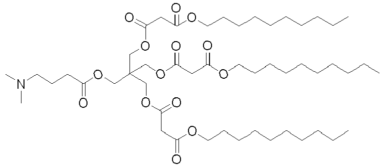 | 928.6 |
| 69 | 1,1'-[2-({[4-(Dimethylamino)butanoyl]oxy }methyl)-2-{[(3-pentyloctanoyl)oxy]methyl}pr opane-1,3-diyl] 3,3'-dioctyl dipropanedioate | 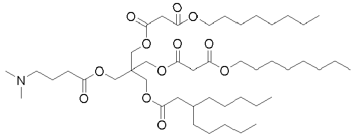 | 842.6 |
| 70 | 1,1'-[2-({[4-(Dimethylamino)butanoyl]oxy }methyl)-2-{[(3-pentyloctanoyl)oxy]methyl }pr opane-1,3-diyl] 3,3'-dinonyl dipropanedioate | 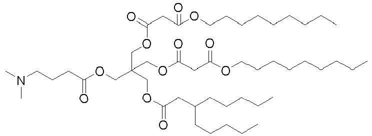 | 870.7 |
| 71 | 1, 1'-Didecyl 3,3'-[2-({[4-(dimethylamino)butanoyl]oxy }methyl)-2-{[(3-pentyloctanoyl)oxy]methyl }pr opane-1,3-diyl] dipropanedioate | 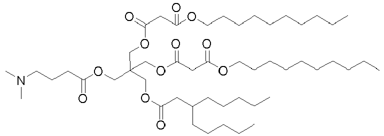 | 8987 |

**[Table 1-11]**

| | | | |
|---|---|---|---|
| 72 | 2-({[4-(Dimethylamino)butanoyl]oxy }methyl)-3-[(3-pentyloctanoyl)oxy]-2-{[(3-pentyloctanoyl)oxy]methyl}pr opyl octyl propanedioate | 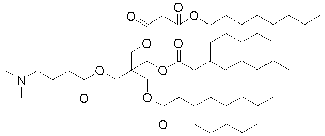 | 840.7 |
| 73 | 2-({[4-(Dimethylamino)butanoyl]oxy }methyl)-3-[(3-pentyloctanoyl)oxy]-2-{[(3-pentyloctanoyl)oxy]methyl }pr opyl nonyl propanedioate | 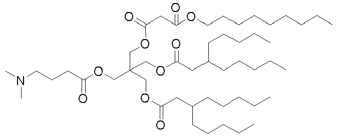 | 854.7 |
| 74 | Decyl 2-({[4-(dimethylamino)butanoyl]oxy }methyl)-3-[(3-pentyloctanoyl)oxy]-2-{[(3-pentyloctanoyl)oxy]methyl }pr opyl propanedioate | 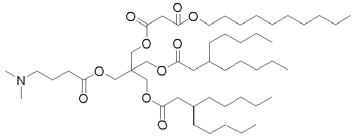 | 868.7 |
| 75 | 1,1'-[2-({[4-(Dimethylamino)butanoyl]oxy }methyl)-2-{[(12-ethoxy-12-oxododecanoyl)oxy]methyl}p ropane-1,3-diyl] 12,12'-diethyl didodecanedioate | 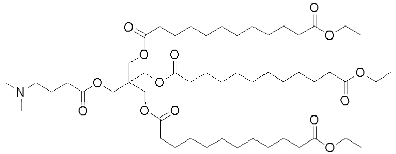 | 970.7 |
| 76 | 1,1'-[2-({[4-(Dimethylamino)butanoyl]oxy }methyl)-2-{[(12-oxo-12-propoxydodecanoyl)oxy] met hyl}propane-1,3-diyl] 12,12'-dipropyl didodecanedioate | 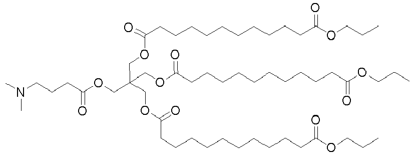 | 1012.7 |
| 77 | 1,1'-[2-({[4-(Dimethylamino)butanoyl]oxy }methyl)-2-{[(3-pentyloctanoyl)oxy]methyl }pr opane-1,3-diyl] 12,12'-dimethyl didodecanedioate | 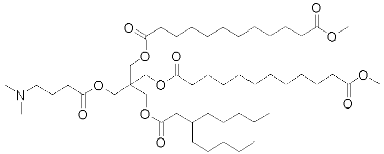 | 898.7 |
| 78 | 1,1'-[2-({[4-(Dimethylamino)butanoyl]oxy }methyl)-2-{[(3-pentyloctanoyl)oxy]methyl}pr opane-1,3-diyl] 12,12'-diethyl didodecanedioate | 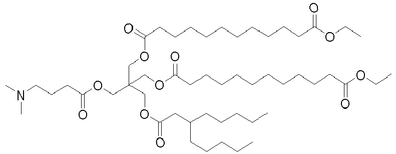 | 926. 7 |

**[Table 1-12]**

| | | | |
|---|---|---|---|
| 79 | 1,1'-[2-({[4-(Dimethylamino)butanoyl]oxy }methyl)-2-{[(3-pentyloctanoyl)oxy]methyl}pr opane-1,3-diyl] 12,12'-dipropyl didodecanedioate | 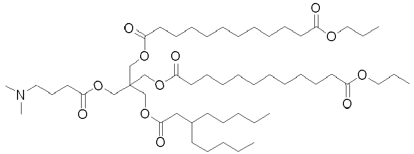 | 954.7 |
| 80 | 2-({[4-(Dimethylamino)butanoyl]oxy }methyl)-3-[(3-pentyloctanoyl)oxy]-2-{[(3-pentyloctanoyl)oxy]methyl }pr opyl methyl dodecanedioate | 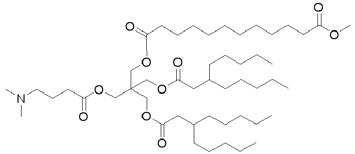 | 868.7 |
| 81 | 2-({[4-(Dimethylamino)butanoyl]oxy }methyl)-3-[(3-pentyloctanoyl)oxy]-2-{[(3-pentyloctanoyl)oxy]methyl }pr opyl ethyl dodecanedioate | 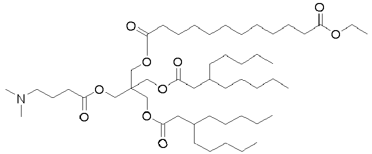 | 882.7 |
| 82 | 2-({[4-(Dimethylamino)butanoyl]oxy }methyl)-3-[(3-pentyloctanoyl)oxy]-2-{[(3-pentyloctanoyl)oxy]methyl }pr opyl propyl dodecanedioate | 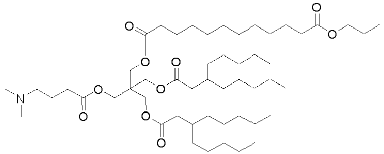 | 896.7 |
| 83 | 1,1'-[2-({[4-(Dimethylamino)butanoyl]oxy }methyl)-2-{[(2-pentylheptanoyl)oxy]methyl}p ropane-1,3-diyl] 6,6'-diheptyl dihexanedioate | 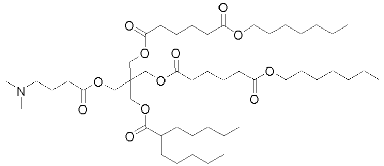 | 884.7 |
| 84 | 1,1'-[2-{[(3-Butyl heptanoyl)oxy]methyl}-2-({[4-(dimethylamino)butanoyl]oxy }methyl)propane-1,3-diyl] 6,6'-diheptyl dihexanedioate | 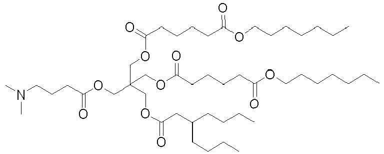 | 870.6 |
| 85 | 1,1'-[2-({[4-(Dimethylamino)butanoyl]oxy }methyl)-2-{[(3-hexylnonanoyl)oxy]methyl}pr opane-1,3-diyl] 6,6'-diheptyl dihexanedioate | 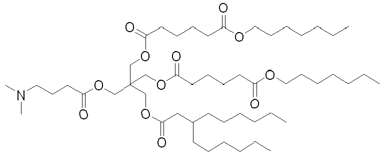 | 926. 7 |

**[Table 1-13]**

| | | | |
|---|---|---|---|
| 86 | 2-({[4-(Dimethylamino)butanoyl]oxy }methyl)-3-[(2-pentylheptanoyl)oxy]-2-{[(2-pentylheptanoyl)oxy]methyl}p ropyl heptyl hexanedioate | 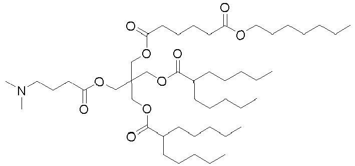 | 840.7 |
| 87 | 2,2-Bis{[(3-butylheptanoyl)oxy]methyl}-3-({[4-(dimethylamino)butanoyl]oxy }propyl heptyl hexanedioate | 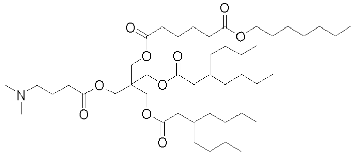 | 812.6 |
| 88 | 2-({[4-(Dimethylamino)butanoyl]oxy }methyl)-3-[(3-hexylnonanoyl)oxy]-2-{[(3-hexylnonanoyl)oxy]methyl}pr opyl heptyl hexanedioate | 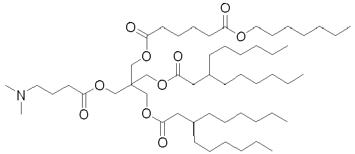 | 924.8 |
| 89 | 1,1'-(2-[({4-[Ethyl(methyl)amino]butanoyl }oxy)methyl]-2-{[(3-pentyloctanoyl)oxy]methyl}pr opane-1,3-diyl] 6,6'-diheptyl dihexanedioate | 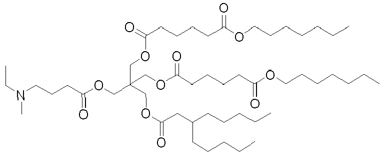 | 912.7 |
| 90 | 1,1'-Diheptyl 6,6'-(2-[({4-[methyl(propan-2-yl)amino]butanoyl}oxy)methyl ]-2-{[(3-pentyloctanoyl)oxy]methyl}pr opane-1,3-diyl) dihexanedioate | 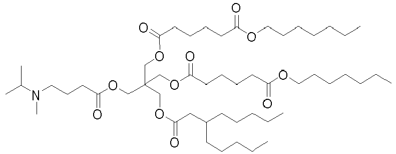 | 926.7 |
| 91 | 1,1'-(2-[({4-[Ethyl(propan-2-yl)amino]butanoyl}oxy)methyl ]-2-{[(3-pentyloctanoyl)oxy]methyl }pr opane-1,3-diyl] 6,6'-diheptyl dihexanedioate | 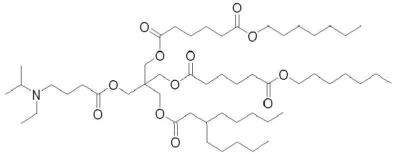 | 940.7 |
| 92 | 1,1'-(2-[({4-[di(Propan-2-yl)amino]butanoyl}oxy)methyl ]-2-{[(3-pentyloctanoyl)oxy]methyl}pr opane-1,3-diyl] 6,6'-diheptyl dihexanedioate | 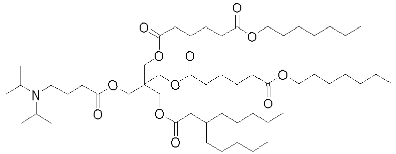 | 954.7 |

**[Table 1-14]**

| | | | |
|---|---|---|---|
| 93 | 1,1'-Diheptyl 6,6'-(2-[({4-[(2-hydroxyethyl)(methyl)amino] butanoyl}oxy)methyl]-2-{[(3-pentyloctanoyl)oxy]methyl}pr opane-1,3-diyl) dihexanedioate | 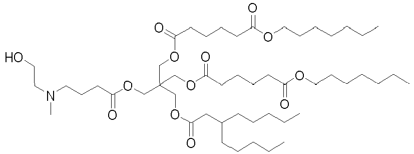 | 928.7 |
| 94 | 1,1'-Diheptyl 6,6'-(2-[({4-[(3-hydroxypropyl)(methyl)amino ]butanoyl}oxy)methyl]-2-{[(3-pentyloctanoyl)oxy]methyl }pr opane-1,3-diyl) dihexanedioate | 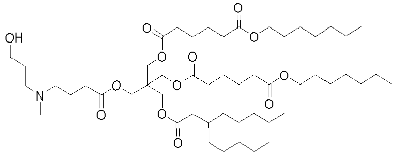 | 942.70 |
| 95 | 1,1'-[2-({[4-(Dimethylamino)butanoyl]oxy }methyl)-2-{[(3-pentyloctanoyl)oxy]methyl }pr opane-1,3-diyl] 4,4'-bis(2-pentylheptyl) dibutanedioate | 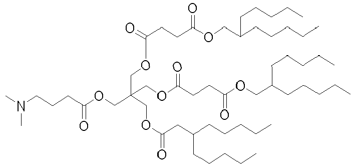 | 982.8 |
| 96 | 2-({[4-(Dimethylamino)butanoyl]oxy }methyl)-3-[(3-pentyloctanoyl)oxy]-2-{[(3-pentyloctanoyl)oxy]methyl }pr opyl 2-pentylheptyl butanedioate | 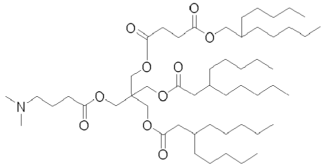 | 910.7 |
| 97 | 1,1'-[2-({[4-(Dimethylamino)butanoyl]oxy }methyl)-2-{[(3-pentyloctanoyl)oxy]methyl}pr opane-1,3-diyl] 4,4'-dinonyl dibutanedioate | 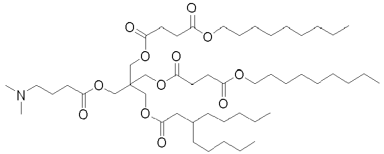 | 898.7 |
| 98 | 1,1'-[2-({[4-(Dimethylamino)butanoyl]oxy }methyl)-2-{[(3-pentyloctanoyl)oxy]methyl}pr opane-1,3-diyl] 5,5'-dioctyl dipentanedioate | 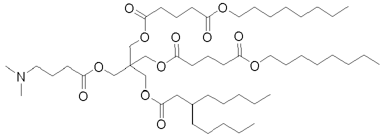 | 898.7 |
| 99 | 1,1'-[2-({[4-(Dimethylamino)butanoyl]oxy }methyl)-2-{[(3-pentyloctanoyl)oxy]methyl}pr opane-1,3-diyl] 7,7'-dihexyl diheptanedioate | 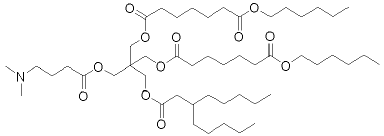 | 898.7 |

**[Table 1-15]**

| | | | |
|---|---|---|---|
| 100 | 1,1'-(2-[({4-[Ethyl(propan-2-yl)amino]butanoyl}oxy)methyl ]-2-{[(3-pentyloctanoyl)oxy]methyl}pr opane-1,3-diyl) 4,4'-dinonyl dibutanedioate | 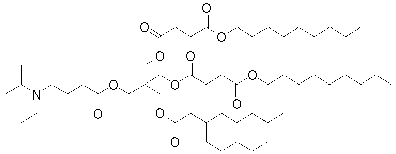 | 940.7 |
| 101 | 1,1'-(2-[({4-[Ethyl(propan-2-yl)amino]butanoyl}oxy)methyl ]-2-{[(3-pentyloctanoyl)oxy]methyl}pr opane-1,3-diyl) 5,5'-dioctyl dipentanedioate | 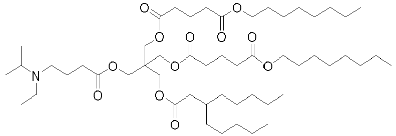 | 940.7 |
| 102 | 1,1'-(2-[({4-[Ethyl(propan-2-yl)amino]butanoyl}oxy)methyl ]-2-{[(3-pentyloctanoyl)oxy]methyl }pr opane-1,3-diyl) 7,7'-dihexyl diheptanedioate | 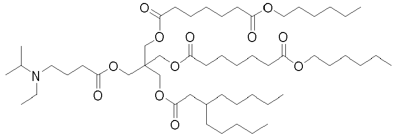 | 940.7 |
| 103 | 2-({[4-(Dimethylamino)butanoyl]oxy }methyl)-3-[(3-pentyloctanoyl)oxy]-2-{[(3-pentyloctanoyl)oxy]methyl }pr opyl nonyl butanedioate | 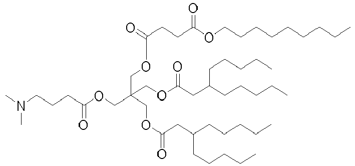 | 868.7 |
| 104 | 2-({[4-(Dimethylamino)butanoyl]oxy }methyl)-3-[(3-pentyloctanoyl)oxy]-2-{[(3-pentyloctanoyl)oxy]methyl }pr opyl octyl pentanedioate | 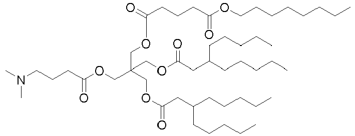 | 868.7 |
| 105 | 2-({[4-(Dimethylamino)butanoyl]oxy }methyl)-3-[(3-pentyloctanoyl)oxy]-2-{[(3-pentyloctanoyl)oxy]methyl }pr opyl hexyl heptanedioate | 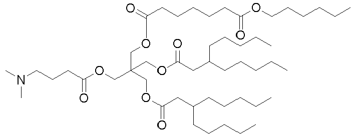 | 868.7 |
| 106 | 1,1'-(2-[({4-[Ethyl(propan-2-yl)amino]butanoyl}oxy)methyl ]-2-1[(3-pentyloctanoyl)oxy]methyl}pr opane-1,3-diyl) 6,6'-dioctyl dihexanedioate | 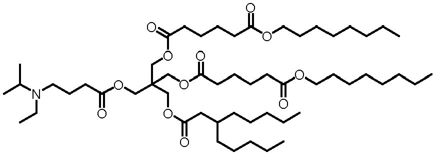 | 968.7 |

**[Table 1-16]**

| | | | |
|---|---|---|---|
| 107 | 1,1'-(2-[({4-[Ethyl(propan-2-yl)amino]butanoyl}oxy)methyl ]-2-{[(3-pentyloctanoyl)oxy]methyl}pr opane-1,3-diyl) 6,6'-dinonyl dihexanedioate | 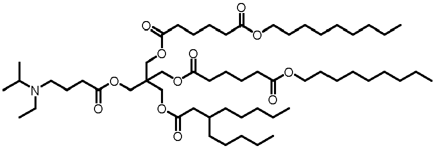 | 996.7 |
| 108 | 1, 1'-Didecyl 6,6'-(2-[({4-[ethyl(propan-2-yl)amino]butanoyl}oxy)methyl ]-2-{[(3-pentyloctanoyl)oxy]methyl }pr opane-1,3-diyl) dihexanedioate | 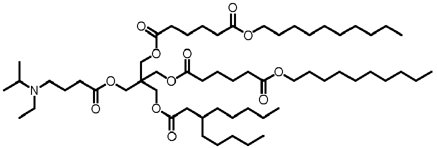 | 1024.8 |
| 109 | 1,1'-(2-[({4-[Ethyl(propan-2-yl)amino]butanoyl}oxy)methyl ]-2-{[(3-pentyloctanoyl)oxy]methyl }pr opane-1,3-diyl) 6,6'-dihexyl dihexanedioate | 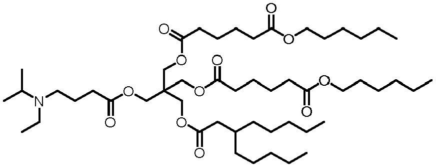 | 912.7 |
| 110 | 1,1'-(2-[({4-[Ethyl(propan-2-yl)amino]butanoyl}oxy)methyl ]-2-{[(3-pentyloctanoyl)oxy]methyl}pr opane-1,3-diyl) 6,6'-di-(4Z)-hept-4-en-1-yl dihexanedioate | 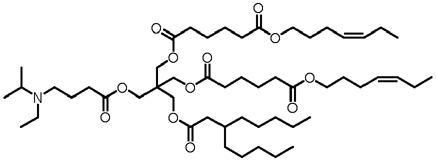 | 936.7 |
| 111 | 1,1'-(2-[({4-[Ethyl(propan-2-yl)amino]butanoyl}oxy)methyl ]-2-{[(3-pentyloctanoyl)oxy]methyl}pr opane-1,3-diyl) 6,6'-di-(3Z)-hept-3-en-1-yl dihexanedioate | 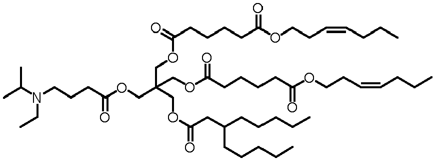 | 936.7 |
| 112 | 1,1'-(2-[({4-[Ethyl(propan-2-yl)amino]butanoyl}oxy)methyl ]-2-{[(2-pentylheptanoyl)oxy]methyl}p ropane-1,3-diyl) 6,6'-diheptyl dihexanedioate | 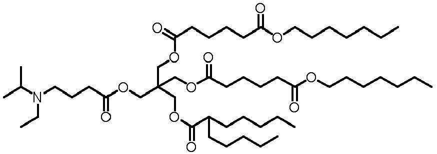 | 926.7 |
| 113 | 1,1'-(2-{[(3-Butylheptanoyl)oxy]methyl}-2-[({4-[ethyl(propan-2-yl)amino]butanoyl}oxy)methyl ]propane-1,3-diyl) 6,6'-diheptyl dihexanedioate | 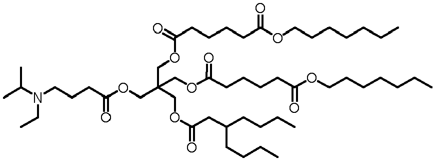 | 912.7 |

**[Table 1-17]**

| | | | |
|---|---|---|---|
| 114 | 1,1'-(2-[({4-[Ethyl(propan-2-yl)amino]butanoyl}oxy)methyl ]-2-{[(3-hexylnonanoyl)oxy]methyl}pr opane-1,3-diyl) 6,6'-diheptyl dihexanedioate | 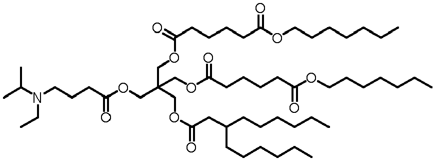 | 968.7 |
| 115 | 1,1'-[2-({[4-(Dimethylamino)butanoyl]oxy }methyl)-2-{[(3-pentyloctanoyl)oxy]methyl}pr opane-1,3-diyl] 6,6'-bis(2-propylpentyl) dihexanedioate | 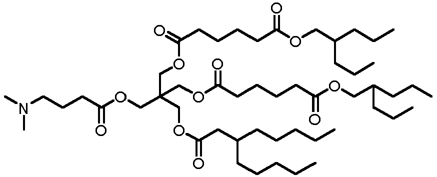 | 926.7 |
| 116 | 1,1'-(2-[({4-[Ethyl(propan-2-yl)amino]butanoyl}oxy)methyl ]-2-{[(3-pentyloctanoyl)oxy]methyl}pr opane-1,3-diyl) 6,6'-bis(2-propylpentyl) dihexanedioate | 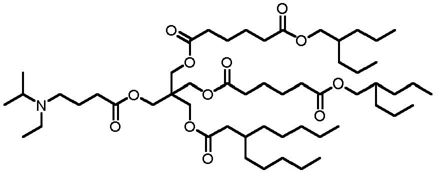 | 968.7 |
| 117 | 1,1'-Bis(2-butylhexyl) 6,6'-[2-({[4-(dimethylamino)butanoyl]oxy }methyl)-2-{[(3-pentyloctanoyl)oxy]methyl }pr opane-1,3-diyl] dihexanedioate | 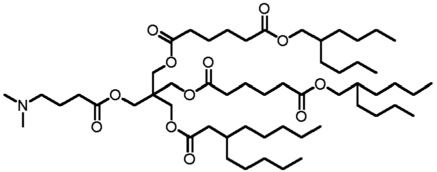 | 982.8 |
| 118 | 1,1'-Bis(2-butylhexyl) 6,6'-(2-[({4-[ethyl(propan-2-yl)amino]butanoyl}oxy)methyl ]-2-{[(3-pentyloctanoyl)oxy]methyl}pr opane-1,3-diyl) dihexanedioate | 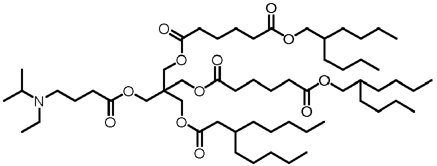 | 1024.8 |
| 119 | 1,1'-{2-({[4-(Dimethylamino)butanoyl]oxy }methyl)-2-[({3-oxo-3-[(2-pentylheptyl)oxy]propanoyl}o xy)methyl]propane-1,3-diyl} 3,3'-bis(2-pentylheptyl) dipropanedioate | 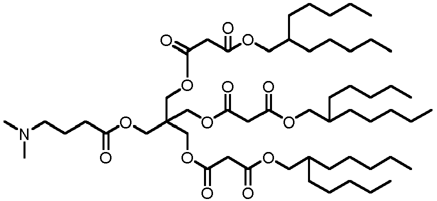 | 1012.7 |

**[Table 1-18]**

| | | | |
|---|---|---|---|
| 120 | 1,1'-{2-[({4-[Ethyl(propan-2-yl)amino]butanoyl}oxy)methyl ]-2-[({3-oxo-3-[(2-pentylheptyl)oxy]propanoyl}o xy)methyl]propane-1,3-diyl} 3,3'-bis(2-pentylheptyl) dipropanedioate | 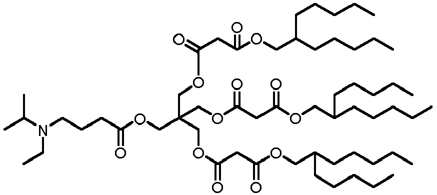 | 1054.7 |
| 121 | 1,1'-[2-({[4-(Dimethylamino)butanoyl]oxy }methyl)-2-{[(3-pentyloctanoyl)oxy]methyl}pr opane-1,3-diyl] 3,3'-dihexyl bis(hexylpropanedioate) | 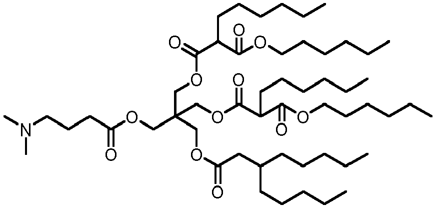 | 954.7 |
| 122 | 1,1'-[2-({[5-(Dimethylamino)pentanoyl]ox y}methyl)-2-{[(3-pentyloctanoyl)oxy]methyl}pr opane-1,3-diyl] 3,3'-dihexyl bis(hexylpropanedioate) | 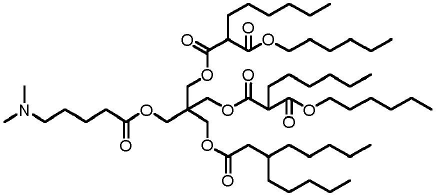 | 968.7 |
| 123 | 2-({[4-(Dimethylamino)butanoyl]oxy }methyl)-3-[(3-pentyloctanoyl)oxy]-2-{[(3-pentyloctanoyl)oxy]methyl }pr opyl hexyl hexylpropanedioate | 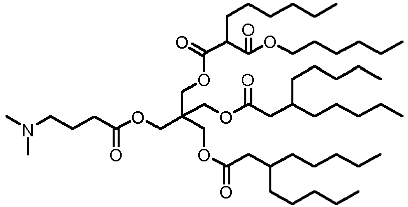 | 896.7 |
| 124 | 2-({[5-(Dimethylamino)pentanoyl]ox y}methyl)-3-[(3-pentyloctanoyl)oxy]-2-{[(3-pentyloctanoyl)oxy]methyl }pr opyl hexyl hexylpropanedioate | 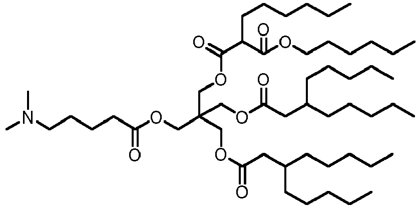 | 910.7 |

**[Table 1-19]**

| | | | |
|---|---|---|---|
| 125 | 1,1'-{2-({[4-(Dimethylamino)butanoyl]oxy }methyl)-2-[(heptanoyloxy)methyl]propa ne-1,3-diyl}3,3'-dihexyl bis(hexylpropanedioate) | 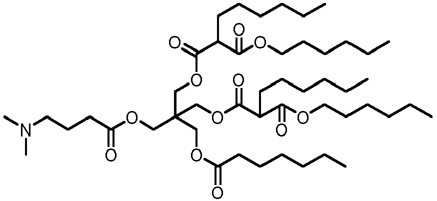 | 870.6 |
| 126 | 1,1'-(2-[({4-[Ethyl(propan-2-yl)amino]butanoyl}oxy)methyl ]-2-{[(3-pentyloctanoyl)oxy]methyl}pr opane-1,3-diyl) 3,3'-dioctyl dipropanedioate | 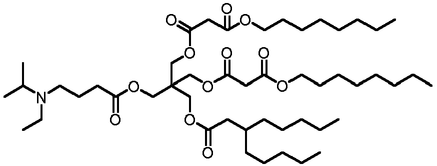 | 884.6 |
| 127 | 1,1'-(2-[({4-[Ethyl(propan-2-yl)amino]butanoyl}oxy)methyl ]-2-{[(3-pentyloctanoyl)oxy]methyl}pr opane-1,3-diyl) 3,3'-dinonyl dipropanedioate | 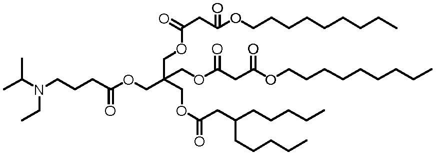 | 912.6 |
| 128 | 1,1'-Didecyl 3,3'-(2-[({4-[ethyl(propan-2-yl)amino]butanoyl}oxy)methyl ]-2-{[(3-pentyloctanoyl)oxy]methyl}pr opane-1,3-diyl) dipropanedioate | 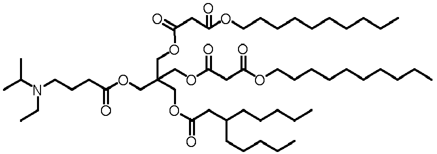 | 940.7 |
| 129 | 1,1'-[2-({[4-(Dimethylamino)butanoyl]oxy }methyl)-2-{[(3-pentyloctanoyl)oxy]methyl}pr opane-1,3-diyl] 8,8'-dipentyl dioctanedioate | 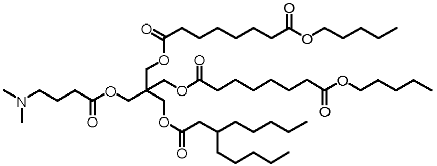 | 898.5 |
| 130 | 1,1'-[2-({[4-(Diethylamino)butanoyl]oxy} methyl)-2-{[(3-pentyloctanoyl)oxy]methyl}pr opane-1,3-diyl] 8,8'-dipentyl dioctanedioate | 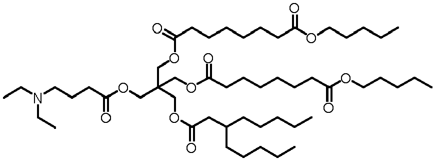 | 926. 7 |

**[Table 1-20]**

| | | | |
|---|---|---|---|
| 131 | 1,1'-(2-[({4-[Ethyl(propan-2-yl)amino]butanoyl}oxy)methyl ]-2-{[(3-pentyloctanoyl)oxy]methyl}pr opane-1,3-diyl) 8,8'-dipentyl dioctanedioate | 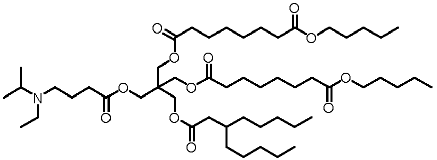 | 940.5 |
| 132 | 1,1'-[2-({[4-(Dimethylamino)butanoyl]oxy }methyl)-2-{[(3-pentyloctanoyl)oxy]methyl}pr opane-1,3-diyl] 3,3'-dimethyl bis(octylpropanedioate) | 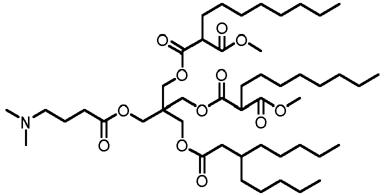 | 870.6 |
| 133 | 1,1'-(2-[({4-[Ethyl(propan-2-yl)amino]butanoyl}oxy)methyl ]-2-{[(3-pentyloctanoyl)oxy]methyl}pr opane-1,3-diyl) 3,3'-dimethyl bis(octylpropanedioate) | 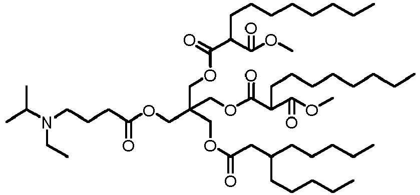 | 912.7 |
| 134 | 1,1'-[2-({[4-(Dimethylamino)butanoyl]oxy }methyl)-2-{[(3-pentyloctanoyl)oxy]methyl}pr opane-1,3-diyl] 3,3'-dimethyl bis(decylpropanedioate) | 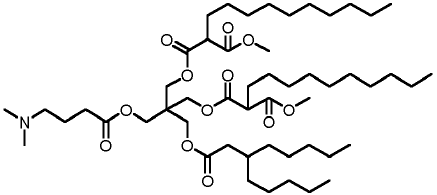 | 926.7 |
| 135 | 1,1'-(2-[({4-[Ethyl(propan-2-yl)amino]butanoyl}oxy)methyl ]-2-{[(3-pentyloctanoyl)oxy]methyl}pr opane-1,3-diyl) 3,3'-dimethyl bis(decylpropanedioate) | 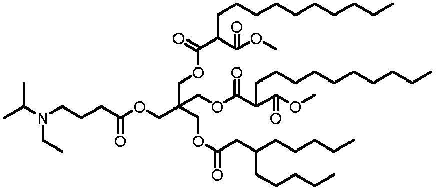 | 968.7 |

**[Table 1-21]**

| | | | |
|---|---|---|---|
| 136 | 2-({[4-(Dimethylamino)butanoyl]oxy }methyl)-3-[(3-pentyloctanoyl)oxy]-2-{[(3-pentyloctanoyl)oxy]methyl}pr opyl methyl octylpropanedioate | 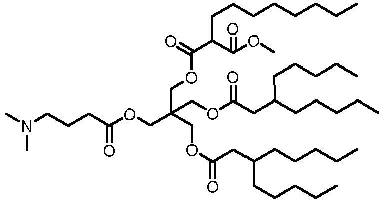 | 854.7 |
| 137 | 2-({[4-(Dimethylamino)butanoyl]oxy }methyl)-3-[(3-pentyloctanoyl)oxy]-2-{[(3-pentyloctanoyl)oxy]methyl }pr opyl methyl decylpropanedioate | 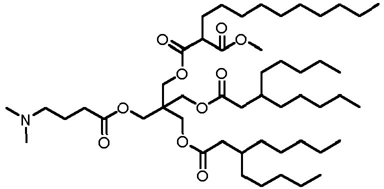 | 882.7 |
| 138 | 1,1'-[2-({[4-(Dimethylamino)butanoyl]oxy }methyl)-2-{[(3-pentyloctanoyl)oxy]methyl }pr opane-1,3-diyl] 5,5'-dinonyl dipentanedioate | 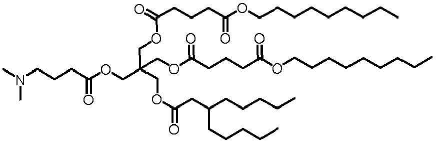 | 926.7 |
| 139 | 1,1'-(2-[({4-[Ethyl(propan-2-yl)amino]butanoyl}oxy)methyl ]-2-{[(3-pentyloctanoyl)oxy]methyl}pr opane-1,3-diyl) 5,5'-dinonyl dipentanedioate | 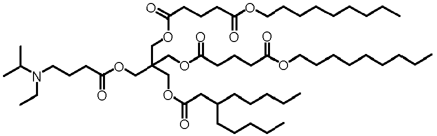 | 968.7 |
| 140 | 1, 1'-Didecyl 5,5'-[2-({[4-(dimethylamino)butanoyl]oxy }methyl)-2-{[(3-pentyloctanoyl)oxy]methyl}pr opane-1,3-diyl] dipentanedioate | 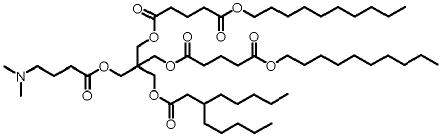 | 954.7 |
| 141 | 1, 1'-Didecyl 5,5'-(2-[({4-[ethyl(propan-2-yl)amino]butanoyl}oxy)methyl ]-2-{[(3-pentyloctanoyl)oxy]methyl}pr opane-1,3-diyl) dipentanedioate | 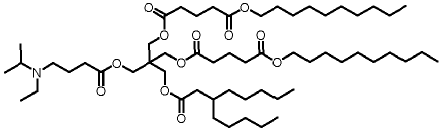 | 996.8 |
| 142 | 1,1'-[2-({[4-(Dimethylamino)butanoyl]oxy }methyl)-2-{[(3-pentyloctanoyl)oxy]methyl}pr opane-1,3-diyl] 5,5'-diundecyl dipentanedioate | 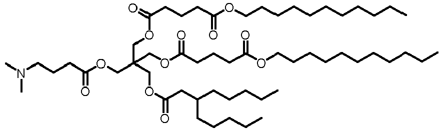 | 982.7 |

**[Table 1-22]**

| | | | |
|---|---|---|---|
| 143 | 1,1'-(2-[({4-[Ethyl(propan-2-yl)amino]butanoyl}oxy)methyl ]-2-{[(3-pentyloctanoyl)oxy]methyl}pr opane-1,3-diyl) 5,5'-diundecyl dipentanedioate | 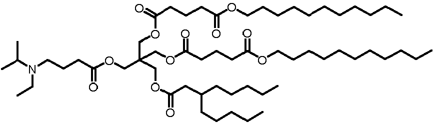 | 1024.8 |
| 144 | 1,1'-[2-({[4-(Dimethylamino)butanoyl]oxy }methyl)-2-{[(3-pentyloctanoyl)oxy]methyl}pr opane-1,3-diyl]5,5'-bis(2-propylpentyl) dipentanedioate | 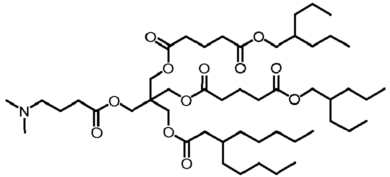 | 8987 |
| 145 | 1,1'-(2-[({4-[Ethyl(propan-2-yl)amino]butanoyl}oxy)methyl ]-2-{[(3-pentyloctanoyl)oxy]methyl}pr opane-1,3-diyl) 5,5'-bis(2-propylpentyl) dipentanedioate | 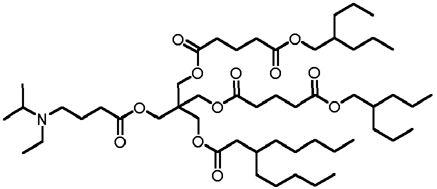 | 940.7 |
| 146 | 1,1'-Bis(2-butylhexyl) 5,5'-[2-({[4-(dimethylamino)butanoyl]oxy }methyl)-2-{[(3-pentyloctanoyl)oxy]methyl}pr opane-1,3-diyl] dipentanedioate | 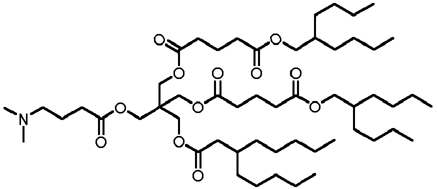 | 954.7 |
| 147 | 1,1'-Bis(2-butylhexyl) 5,5'-(2-[({4-[ethyl(propan-2-yl)amino]butanoyl}oxy)methyl ]-2-{[(3-pentyloctanoyl)oxy]methyl}pr opane-1,3-diyl) dipentanedioate | 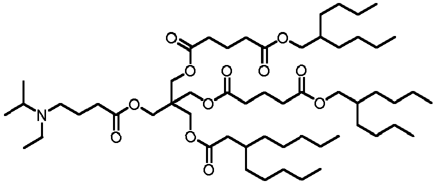 | 996.8 |
| 148 | 1,1'-[2-({[4-(Dimethylamino)butanoyl]oxy }methyl)-2-{[(3-propyldecanoyl)oxy]methyl}p ropane-1 ,3-diyl] 5,5'-dioctyl dipentanedioate | 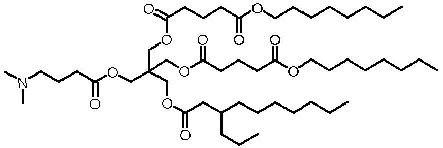 | 8986 |
| 149 | 1,1'-(2-[({4-[Ethyl(propan-2-yl)amino]butanoyl}oxy)methyl ]-2-{[(3-propyldecanoyl)oxy]methyl}p ropane-1 ,3-diyl) 5,5'-dioctyl dipentanedioate | 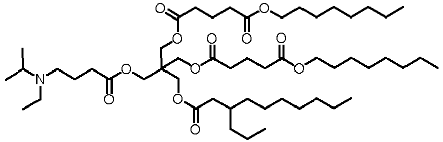 | 940.7 |

**[Table 1-23]**

| | | | |
|---|---|---|---|
| 150 | 1,1'-[2-{[(3-Butylheptanoyl)oxy]methyl}-2-({[4-(dimethylamino)butanoyl]oxy }methyl)propane-1,3-diyl] 5,5'-dioctyl dipentanedioate | 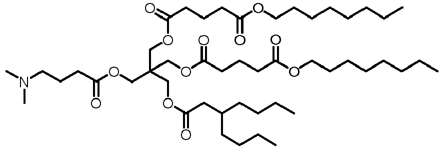 | 870.6 |
| 151 | 1,1'-(2-{[(3-Butylheptanoyl)oxy]methyl}-2-[({4-[ethyl(propan-2-yl)amino]butanoyl}oxy)methyl ]propane-1,3-diyl) 5,5'-dioctyl dipentanedioate | 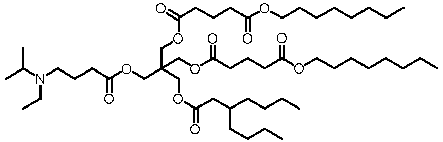 | 912.7 |
| 152 | 1,1'-[2-({[4-(Dimethylamino)butanoyl]oxy }methyl)-2-{[(3-hexylnonanoyl)oxy]methyl}pr opane-1,3-diyl] 5,5'-dioctyl dipentanedioate | 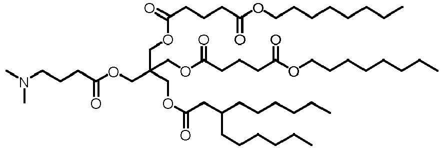 | 926.7 |
| 153 | 1,1'-(2-[({4-[Ethyl(propan-2-yl)amino]butanoyl}oxy)methyl ]-2-{[(3-hexylnonanoyl)oxy]methyl}pr opane-1,3-diyl) 5,5'-dioctyl dipentanedioate | 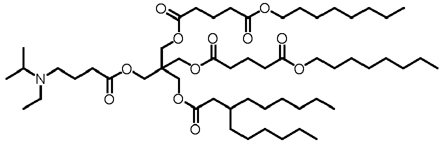 | 968.7 |
| 154 | 1,1'-[2-({[4-(Dimethylamino)butanoyl]oxy }methyl)-2-{[(2-pentylheptanoyl)oxy]methyl}p ropane-1 ,3-diyl] 5,5'-dioctyl dipentanedioate | 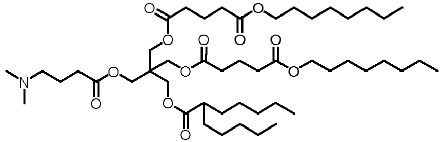 | 884.6 |
| 155 | 1,1'-(2-[({4-[Ethyl(propan-2-yl)amino]butanoyl}oxy)methyl ]-2-{[(2-pentylheptanoyl)oxy]methyl}p ropane-1 ,3-diyl) 5,5'-dioctyl dipentanedioate | 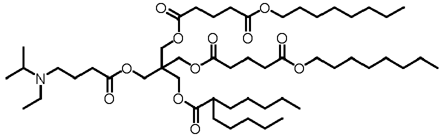 | 926.7 |
| 156 | 1,1'-{2-({[4-(Dimethylamino)butanoyl]oxy }methyl)-2-[({2-[(hexyloxy)carbonyl]octanoyl} oxy)methyl]propane-1,3-diyl} 3,3'-dihexyl bis(hexylpropanedioate) | 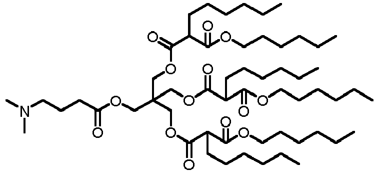 | 1012.8 |

**[Table 1-24]**

| | | | |
|---|---|---|---|
| 157 | 1,1'-{2-({[5-(Dimethylamino)pentanoyl]ox y}methyl)-2-[({2-[(hexyloxy)carbonyl]octanoyl} oxy)methyl]propane-1,3-diyl} 3,3'-dihexyl bis(hexylpropanedioate) | 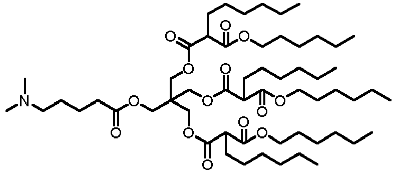 | 1026.8 |
| 158 | 1,1'-{2-({[5-(Dimethylamino)pentanoyl]ox y}methyl)-2-[({3-oxo-3-[(2-pentylheptyl)oxy]propanoyl}o xy)methyl]propane-1,3-diyl} 3,3'-bis(2-pentylheptyl) dipropanedioate | 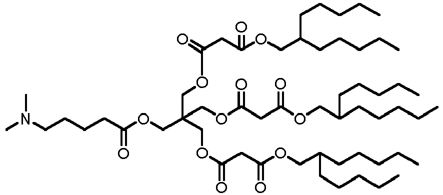 | 1026.7 |
| 159 | 1,1'-[2-({[4-(Dimethylamino)butanoyl]oxy }methyl)-2-{[(3-pentyloctanoyl)oxy]methyl}pr opane-1,3-diyl] 3,3'-bis(2-pentylheptyl) dipropanedioate | 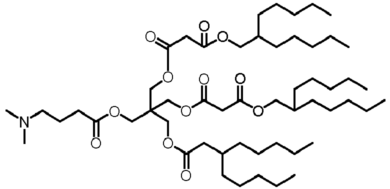 | 954.8 |
| 160 | 1,1'-[2-({[5-(Dimethylamino)pentanoyl]ox y}methyl)-2-{[(3-pentyloctanoyl)oxy]methyl}pr opane-1,3-diyl] 3,3'-bis(2-pentylheptyl) dipropanedioate | 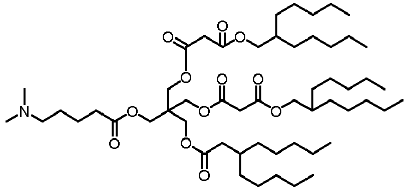 | 968.8 |
| 161 | 2-({[4-(Dimethylamino)butanoyl]oxy }methyl)-3-[(3-pentyloctanoyl)oxy]-2-{[(3-pentyloctanoyl)oxy]methyl}pr opyl 2-pentylheptyl propanedioate | 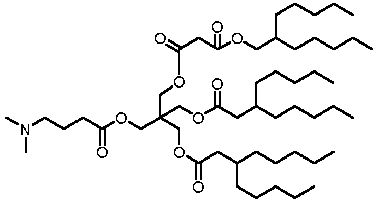 | 896.8 |

**[Table 1-25]**

| | | | |
|---|---|---|---|
| 162 | 2-({[5-(Dimethylamino)pentanoyl]ox y}methyl)-3-[(3-pentyloctanoyl)oxy]-2-{[(3-pentyloctanoyl)oxy]methyl }pr opyl 2-pentylheptyl propanedioate | 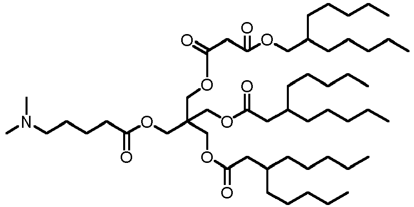 | 9108 |
| 163 | 1,1'-{2-({[4-(Dimethylamino)butanoyl]oxy }methyl)-2-[({3-oxo-3-[(undecane-6-yl)oxy]propanoyl}oxy)methyl] propane-1,3-diyl} 3,3'-diundecane-6-yl dipropanedioate | 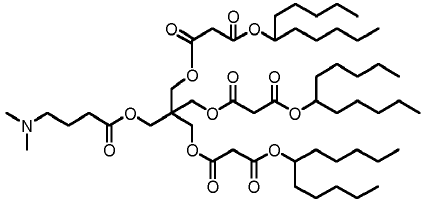 | 970.9 |
| 164 | 1,1'-{2-({[5-(Dimethylamino)pentanoyl]ox y}methyl)-2-[({3-oxo-3-[(undecane-6-yl)oxy]propanoyl}oxy)methyl] propane-1,3-diyl} 3,3'-diundecane-6-yl dipropanedioate | 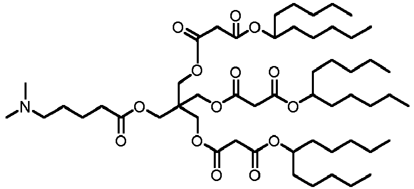 | 984.8 |
| 165 | 1,1'-{2-({[4-(Dimethylamino)butanoyl]oxy }methyl)-2-[({3-oxo-3-[(tridecan-7-yl)oxy]propanoyl}oxy)methyl] propane-1,3-diyl} 3,3'-ditridecane-7-yl dipropanedioate | 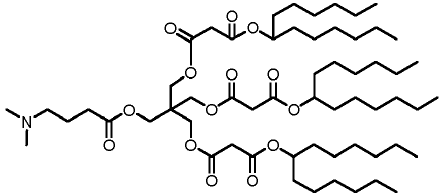 | 1054.5 |
| 166 | 1,1'-{2-({[5-(Dimethylamino)pentanoyl]ox y}methyl)-2-[({3-oxo-3-[(tridecan-7-yl)oxy]propanoyl}oxy)methyl] propane-1,3-diyl} 3,3'-ditridecane-7-yl dipropanedioate | 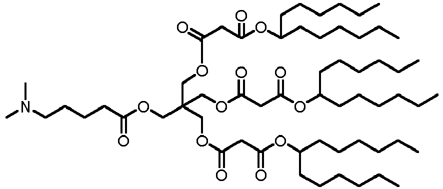 | 1068.6 |

**[Table 1-26]**

| | | | |
|---|---|---|---|
| 167 | 1,1'-Bis(2-butylhexyl) 3,3'-{2-[({3-[(2-butylhexyl)oxy]-3-oxopropanoyl}oxy)methyl]-2-({[4-(dimethylamino)butanoyl]oxy }methyl)propane-1,3-diyl} dipropanedioate | 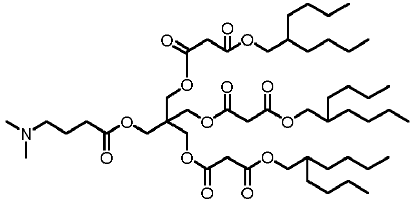 | 928.8 |
| 168 | 1,1'-Bis(2-butylhexyl) 3,3'-{2-[({3-[(2-butylhexyl)oxy]-3-oxopropanoyl}oxy)methyl]-2-({[5-(dimethylamino)pentanoyl]ox y}methyl)propane-1,3-diyl} dipropanedioate | 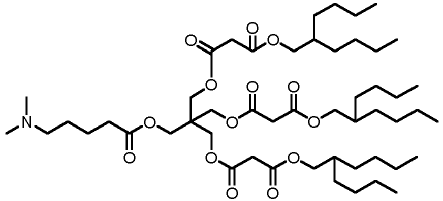 | 942.5 |
| 169 | 1,1'-{2-({[4-(Dimethylamino)butanoyl]oxy }methyl)-2-[({3-[(2-hexyloctyl)oxy]-3-oxopropanoyl}oxy)methyl]pro pane-1,3-diyl} 3,3'-bis(2-hexyloctyl) dipropanedioate | 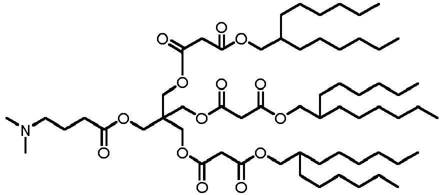 | 1097.0 |
| 170 | 1,1'-{2-({[5-(Dimethylamino)pentanoyl]ox y}methyl)-2-[({3-[(2-hexyloctyl)oxy]-3-oxopropanoyl}oxy)methyl]pro pane-1,3-diyl} 3,3'-bis(2-hexyloctyl) dipropanedioate | 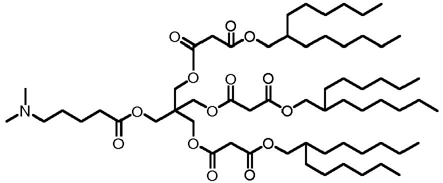 | 11111 |
| 171 | 1,1'-Bis(2-butyloctyl) 3,3'-{2-[({3-[(2-butyloctyl)oxy]-3-oxopropanoyl}oxy)methyl]-2-({[4-(dimethylamino)butanoyl]oxy }methyl)propane-1,3-diyl} dipropanedioate | 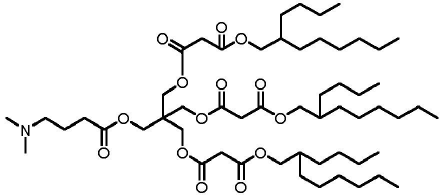 | 1012.5 |

**[Table 1-27]**

| | | | |
|---|---|---|---|
| 172 | 1,1'-Bis(2-butyloctyl) 3,3'-{2-[({3-[(2-butyloctyl)oxy]-3-oxopropanoyl}oxy)methyl]-2-({[5-(dimethylamino)pentanoyl]ox y}methyl)propane-1,3-diyl} dipropanedioate | 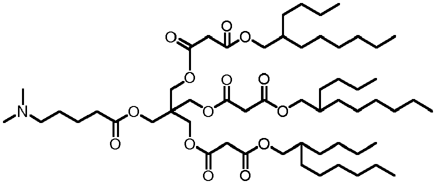 | 1026.5 |
| 173 | N,N,N-trimethyl-4-oxo-4-{3-({3-oxo-3-[(2-pentylheptyl)oxy]propanoyl}o xy)-2,2-bis[({3-oxo-3-[(2-pentylheptyl)oxy]propanoyl}o xy)methyl]propoxy}butane-1-aminium | 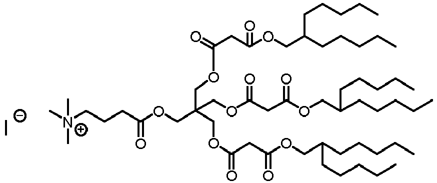 | 1026.9 |

### [Example 3]

### 1,1'-[2-{[(N,N-Dimethyl-beta-alanyl)oxy]methyl}-2-({[6-(heptyloxy)-6-oxohexanoyl]oxy}methyl)propane-1,3-diyl] 6,6'-diheptyl dihexanedioate

### A) 2-({[tert-Butyl(diphenyl)silyl]oxy]methyl}-2-(hydroxymethyl)propane-1,3-diol

A mixture of 2,2-bis(hydroxymethyl)propane-1,3-diol (49.5 g), triethylamine (36.8 g) and DMA (300 mL) was stirred at room temperature, and a mixture of tert-butyldiphenylchlorosilane (50 g) and DMA (200 mL) was added dropwise thereto over 1 hour. 6 hours later, ethyl acetate, water, and heptane were added to the mixture, and the mixture was stirred for 10 minutes. The reaction mixture was separated into an aqueous layer (A) and an organic layer (B). A was subjected to extraction with a mixed solvent of ethyl acetate and heptane. The extracts were washed with a 20% aqueous citric acid solution, a 5% aqueous sodium bicarbonate solution, saturated brine and water and then concentrated under reduced pressure (C). B was subjected to extraction with a mixed solvent of water and DMA. The obtained aqueous layer was subjected to extraction with a mixed solvent of ethyl acetate and heptane. Then, the extracts were washed with a 20% aqueous citric acid solution, a 5% aqueous sodium bicarbonate solution, saturated brine and water and concentrated under reduced pressure (D). C and D were mixed to obtain the title compound (78 g).
¹H NMR (400 MHz, CDCl₃) δ 7.66 (dd, J=1.3, 7.8 Hz, 4H), 7.50 - 7.38 (m, 6H), 3.75 (s, 6H), 3.67 (s, 2H), 2.46 - 2.24 (br s, 3H), 1.08 (s, 9H).

### B) 6-(Heptyloxy)-6-oxohexanoic acid

To a mixture of oxepane-2,7-dione (10.0 g), heptan-1-ol (10.0 g) and DCM (120 mL), DMAP (953 mg) and TEA (15.8 g) were added, and the mixture was stirred at room temperature for 3 hours. 1 M hydrochloric acid was added to the mixture, followed by extraction with DCM. The organic layer was washed with saturated brine, then dried over sodium sulfate, and concentrated under reduced pressure. The residue was purified by silica gel column chromatography (ethyl acetate/petroleum ether) to obtain the title compound (11.1 g).
¹H NMR (400 MHz, DMSO-d6) δ 12.04 (br s, 1H) 4.00 (t, J=6.53 Hz, 2H) 2.29 (t,J=6.90 Hz, 2H) 2.21 (t, J=6.90 Hz, 2H) 1.60 - 1.43 (m, 6H) 1.34 - 1.20 (m, 8H) 0.90 - 0.82 (m, 3H).

### C) 1,1'-Diheptyl 6,6'-[2-({ [6-(heptyloxy)-6-oxohexanoyl]oxy}methyl)-2-(hydroxymethyl)propane-1,3-diyl] dihexanedioate

To a mixture of 2-({[tert-butyl(diphenyl)silyl]oxy}methyl)-2-(hydroxymethyl)propane-1,3-diol (2.0 g), 6-(heptyloxy)-6-oxohexanoic acid (4.0 g) and DMF (30 mL), EDCI (6.1 g) and DMAP (652 mg) were added, and the mixture was stirred at room temperature for 15 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain 1,1'-[2-({[tert-butyl(diphenyl)silyl]oxy}methyl)-2-({[6-(heptyloxy)-6-oxohexanoyl]oxy}methyl)propane-1,3-diyl] 6,6'-diheptyl dihexanedioate (4.7 g). To a mixture of 1,1'-[2-({[tert-butyl(diphenyl)silyl]oxy}methyl)-2-({[6-(heptyloxy)-6-oxohexanoyl]oxy}methyl)propane-1,3-diyl] 6,6'-diheptyl dihexanedioate (4.7 g) and THF (45 mL), a solution of TBAF in THF (1 M, 5.8 mL) was added, and the mixture was stirred at room temperature for 12 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (2.4 g). ¹H NMR (400 MHz, CDCl₃) δ 4.13 (s, 6H) 4.08 (t, J=6.79 Hz, 6H) 3.53 (s, 2H) 2.44 - 2.30 (m, 12H) 1.71 - 1.60 (m, 18H) 1.37 - 1.26 (m, 24H) 0.97 - 0.86 (m, 9H).

### D) 1,1'-[2-[{(N,N-Dimethyl-beta-alanyl)oxy]methyl}-2-({[6-(heptyloxy)-6-oxohexanoylJoxy}methyl)propane-1,3-diyl] 6,6'-diheptyl dihexanedioate

To a mixture of 1,1'-diheptyl 6,6'-[2-({[6-(heptyloxy)-6-oxohexanoyl]oxy}methyl)-2-(hydroxymethyl)propane-1,3-diyl] dihexanedioate (0.4 g), 3-(dimethylamino)propionic acid hydrochloride (152 mg) and DMF (5 mL), EDCI (235 mg) and DMAP (30 mg) were added, and the mixture was stirred at room temperature for 15 hours. Water was added to the mixture, followed by extraction with ethyl acetate. The organic layer was washed with saturated brine, then dried over sodium sulfate, and concentrated under reduced pressure. The residue was purified by silica gel column chromatography (MeOH/DCM) to obtain the title compound (210 mg).
¹H NMR (400 MHz, CDCl₃) δ 4.19 - 4.10 (m, 8H), 4.07 (t, J=6.79 Hz, 6H), 2.63 - 2.56 (m, 2H), 2.53 - 2.46 (m, 2H), 2.39 - 2.29 (m, 12H), 2.24 (s, 6H), 1.74 - 1.55 (m, 18H), 1.39 - 1.23 (m, 24H), 0.94 - 0.84 (m, 9H).
MS: [M+H]⁺ 914.6.

### [Example 13]

### 1,1'-[2-({[4-(Dimethylamino)butanoyl]oxy}methyl)-2-{[(3-pentyloctanoyl)oxy]methyl}propane-1,3-diyl] 6,6'-diheptyl dihexanedioate

### A) 3-Pentyloctanoic acid

To a mixture of 60% sodium hydride (1.12 g) and THF (100 mL), ethyl 2-diethoxyphosphoryl acetate (6.73 g) was added at 0°C, and the mixture was stirred for 10 minutes. Undecan-6-one (3.41 g) was added thereto at room temperature, and the mixture was stirred at 50°C for 12 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain ethyl 3-pentyloct-2-enoate (4.1 g). To a mixture of ethyl 3-pentyloct-2-enoate (0.8 g) and EtOH (10 mL), 10% Pd/C (50 mg) was added, and the mixture was stirred at room temperature for 18 hours in a hydrogen atmosphere (15 psi). The mixture was filtered, and the filtrate was dried under reduced pressure to obtain ethyl 3-pentyloctanoate (0.8 g). To a mixture of ethyl 3-pentyloctanoate (0.8 g) and EtOH (10 mL), a solution of sodium hydroxide (0.66 g) in water (3 mL) was added, and the mixture was stirred at 50°C for 1 hour. The mixture was concentrated under reduced pressure. The residue was diluted with water and washed with petroleum ether. The aqueous layer was rendered acidic (pH = 3 to 4) with 1 M hydrochloric acid, followed by extraction with ethyl acetate. The organic layer was washed with saturated brine, then dried over sodium sulfate, and concentrated under reduced pressure to obtain the title compound (0.5 g) .
¹H NMR (400 MHz, CDCl₃) δ 2.30 (d, J=6.9 Hz, 2H), 1.88 (m, 1H), 1.37 - 1.25 (m,16H), 0.91 (t, J=6.9 Hz, 6H).

### B) 2-({[tert-Butyl(diphenyl)silyl]oxy}methyl)-3-hydroxy-2-(hydroxymethyl)propyl 3-pentyloctanoate

To a mixture of 3-pentyloctanoic acid (3.43 g) and DMF (100 mL), EDCI (4.61 g), 2-({[tert-butyl(diphenyl)silyl]oxy}methyl)-2-(hydroxymethyl)propane-1,3-diol (10.0 g) and DMAP (815 mg) were added, and the mixture was stirred at room temperature for 16 hours. The mixture was poured to water, followed by extraction with ethyl acetate/petroleum ether. The organic layer was washed with saturated brine, then dried over sodium sulfate, and concentrated under reduced pressure. The residue was purified by silica gel column chromatography (ethyl acetate/petroleum ether) to obtain the title compound (3.9 g). ¹H NMR (400 MHz, CDCl₃) δ 7.70 - 7.60 (m, 4H), 7.49 - 7.37 (m, 6H), 4.26 (s, 2H), 3.73 - 3.52 (m, 6H), 2.23 (d, J=6.8 Hz, 2H), 1.85 - 1.75 (m, 1H), 1.35 - 1.17(m, 16H), 1.07 (s, 9H), 0.87 (t, J=7.0 Hz, 6H).

### C) 1,1'-[2-({[tert-Butyl(diphenyl)silyl]oxy}methyl)-2-{[(3-pentyloctanoyl)oxy]methyl}propane-1,3-diyl] 6,6'-diheptyl dihexanedioate

To a mixture of 2-({[tert-butyl(diphenyl)silyl]oxy}methyl)-3-hydroxy-2-(hydroxymethyl)propyl 3-pentyloctanoate (3.9 g) and DMF (60 mL), 6-(heptyloxy)-6-oxohexanoic acid (4.0 g), DMAP (419 mg) and EDCI (3.9 g) were added, and the mixture was stirred at room temperature for 16 hours. The mixture was poured to water, followed by extraction with ethyl acetate/petroleum ether. The organic layer was washed with saturated brine, then dried over sodium sulfate, and concentrated under reduced pressure. The residue was purified by silica gel column chromatography (ethyl acetate/petroleum ether) to obtain the title compound (6.8 g). ¹H NMR (400 MHz, CDCl₃) δ 7.54 (d, J=7.0 Hz, 4H), 7.41 - 7.26 (m, 6H), 4.15 (s,6H), 4.07 (t, J=6.8 Hz, 4H), 3.62 (s, 2H), 2.25 - 2.14 (m, 8H), 2.12 (d, J=6.8 Hz, 2H), 1.80 - 1.72 (m, 1H), 1.61 - 1.48 (m, 12H), 1.32 - 1.08 (m, 32H), 0.97 (s, 9H), 0.86 - 0.72 (m, 12H).

### D) 1,1'-Diheptyl 6,6'-[2-(hydroxymethyl)-2-{[(3-pentyloctanoyl)oxy]methyl}propane-1,3-diyl] dihexanedioate

To a mixture of 1,1'-[2-({[tert-butyl(diphenyl)silyl]oxy}methyl)-2-{[(3-pentyloctanoyl)oxy}methyl]propane-1,3-diyl] 6,6'-diheptyl dihexanedioate (6.8 g) and THF (120 mL), a solution of TBAF in THF (1 M, 8.6 mL) was added, and the mixture was stirred at room temperature for 24 hours. The solution was rendered neutral by the addition of acetic acid to the mixture and stirred for 10 minutes. This solution was poured to water, followed by extraction with ethyl acetate/petroleum ether. The organic layer was washed with saturated brine, then dried over sodium sulfate, and concentrated under reduced pressure. The residue was purified by silica gel column chromatography (ethyl acetate/petroleum ether) to obtain the title compound (3.8 g). ¹H NMR (400 MHz, CDCl₃) δ 4.12 - 4.00 (m, 10H), 3.50 (s, 2H), 2.41 - 2.22 (m, 10H), 1.85-1.80 (m, 1H), 1.72 - 1.55 (m, 12H), 1.36 - 1.18 (m, 32H), 0.92 - 0.81 (m, 12H).

### E) 1,1'-[2-({[4-(Dimethylamino)butanoyl]oxy}methyl)-2-{[(3-pentyloctanoyl)oxy]methyl}propane-1,3-diyl] 6,6'-diheptyl dihexanedioate

To a mixture of 1,1'-diheptyl 6,6'-[2-(hydroxymethyl)-2-{[(3-pentyloctanoyl)oxy]methyl}propane-1,3-diyl] dihexanedioate (0.4 g) and DMF (20 mL), 4-(dimethylamino)butyric acid hydrochloride (120 mg), EDCI (176 mg) and DMAP (31 mg) were added, and the mixture was stirred at room temperature for 16 hours. The mixture was poured to water, followed by extraction with ethyl acetate. The organic layer was washed with a 5% aqueous sodium bicarbonate solution, then dried over sodium sulfate, and concentrated under reduced pressure. The residue was purified by silica gel column chromatography (MeOH/DCM) to obtain the title compound (0.3 g).
¹H NMR (400 MHz, CDCl₃) δ 4.11 (s, 8H), 4.05 (t, J=6.8 Hz, 4H), 2.56 - 2.45 (m,2H), 2.42 - 2.36 (m, 8H), 2.36 - 2.28 (m, 8H), 2.28 - 2.20 (m, 2H), 1.91 - 1.85(m, 2H), 1.85 - 1.80 (m, 1H), 1.68 - 1.57 (m, 12H), 1.36 - 1.20 (m, 32H), 0.92 - 0.85 (m, 12H) .
MS: [M+2H]²⁺ 449.9.

### [Example 22]

### {[2-{[(N,N-Dimethyl-beta-alanyl)oxy]methyl}-2-({[5-(octanoyloxy)pentanoyl]oxy}methyl)propane-1,3-diyl]bis(oxy)-5-oxopentane-5,1-diyl} dioctanoate

### A) Benzyl 5-hydroxypentanoate

To a mixture of delta-valerolactone (20 g) and EtOH (200 mL), a solution of sodium hydroxide (8.8 g) in water (50 mL) was added, and the mixture was stirred at 80°C for 0.5 hours and further stirred at room temperature for 12 hours. The mixture was concentrated under reduced pressure. To a mixture of the residue and acetone (200 mL), benzyl bromide (40.7 g) and TBAB (3.2 g) were added, and the mixture was stirred at 60°C for 12 hours. The mixture was dried under reduced pressure. The residue was diluted with ethyl acetate, washed with an aqueous sodium bisulfate solution, a 5% aqueous sodium bicarbonate solution and saturated brine, then dried over sodium sulfate, and concentrated under reduced pressure. The residue was purified by silica gel column chromatography (ethyl acetate/petroleum ether) to obtain the title compound (33 g).
¹H NMR (400 MHz, CDCl₃) δ 7.39-7.32 (m, 5H), 5.12 (s, 2H), 3.64 (t, J=6.4 Hz, 2H), 2.41 (t, J=7.4 Hz, 2H), 1.77-1.73 (m, 2H), 1.62-1.58 (m, 2H).

### B) 5-(Benzyloxy)-5-oxopentyl octanoate

To a mixture of benzyl 5-hydroxypentanoate (33 g) and DCM (300 mL), octanoic acid (45.7 g), DMAP (38.7 g) and EDCI (91.1 g) were added, and the mixture was stirred at room temperature for 12 hours. DCM was added to the mixture, and the mixture was washed with water and saturated brine, then dried over sodium sulfate, and concentrated under reduced pressure. The residue was purified by silica gel column chromatography (ethyl acetate/petroleum ether) to obtain the title compound (43 g). ¹H NMR (400 MHz, CDCl₃) δ 7.35 - 7.27 (m, 5H), 5.05 (s, 2H), 3.99 (t, J=6.4 Hz,2H), 2.33 (t, J=6.8 Hz, 2H), 2.21 (t, J=6.8 Hz, 2H), 1.64 - 1.54 (m, 6H), 1.24-1.21 (m, 8H), 0.81 (t, J=6.8 Hz, 3H) .

### C) 5-(Octanoyloxy)pentanoic acid

A mixture of 5-(benzyloxy)-5-oxopentyl octanoate (1 g), 10% Pd/C (100 mg) and EtOH (10 mL) was stirred at room temperature for 1 hour in a hydrogen atmosphere (15 psi). The mixture was filtered, and the filtrate was concentrated under reduced pressure to obtain the title compound (0.7 g).
¹H NMR (400 MHz, DMSO-d6) δ = 4.00 (t, J=6.1 Hz, 2H), 2.32 - 2.18 (m, 4H), 1.63- 1.46 (m, 6H), 1.32 - 1.17 (m, 8H), 0.91 - 0.81 (m, 3H).

### D) {[2-({[tert-Butyl(diphenyl)silyl]oxy}methyl)-2-({[5-(octanoyloxy)pentanoyl]oxy}methyl)propane-1,3-diyl]bis(oxy)-5-oxopentane-5,1-diyl} dioctanoate

A mixture of 2-({[tert-butyl(diphenyl)silyl]oxy}methyl)-2-(hydroxymethyl)propane-1,3-diol (570 mg), 5-(octanoyloxy)pentanoic acid (1.49 g), EDCI (1.02 g), DMAP (186 mg) and DMF (10 mL) was stirred at room temperature for 12 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (1.3 g).
¹H NMR (400 MHz, CDCl₃) δ 7.63 (m, 4H), 7.50 - 7.37 (m, 6H), 4.15 (s, 6H), 4.11- 4.02 (m, 6H), 3.63 (s, 2H), 2.35 - 2.24 (m, 12H), 1.69 - 1.60 (m, 18H), 1.38 - 1.25 (m, 24H), 1.06 (s, 9H), 0.95 - 0.85 (m, 9H).

### E) {[2-(Hydroxymethyl)-2-({[5-(octanoyloxy)pentanoyl]oxy}methyl)propane-1,3-diyl]bis(oxy)-5-oxopentane-5,1-diyl} dioctanoate

A mixture of {[2-({[tert-butyl(diphenyl)silyl]oxy}methyl)-2-({[5-(octanoyloxy)pentanoyl]oxy}methyl)propane-1,3-diyl]bis(oxy)-5-oxopentane-5,1-diyl} dioctanoate (3.6 g) and THF (80 mL) was stirred, and a solution of TBAF in THF (1 M, 4.1 mL) was added dropwise thereto. 18 hours later, water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (1.8 g). ¹H NMR (400 MHz, DMSO-d6) δ 4.90 (t, J=5.4 Hz, 1H), 4.07 - 3.93 (m, 12H), 3.42 (d, J=5.4 Hz, 2H), 2.38 - 2.20 (m, 12H), 1.63 - 1.44 (m, 18H), 1.34 - 1.14 (m, 24H), 0.93 - 0.79 (m, 9H).

### F) {[2-{[(N,N-Dimethyl-beta-alanyl)oxy]methyl}-2-({[5-(octanoyloxy)pentanoyl]oxy}methyl)propane-1,3-diyl]bis(oxy)-5-oxopentane-5,1-diyl} dioctanoate

To a mixture of {[2-(hydroxymethyl)-2-({[5-(octanoyloxy)pentanoyl]oxy}methyl)propane-1,3-diyl]bis(oxy)-5-oxopentane-5,1-diyl} dioctanoate (300 mg) and DMF (10 mL), 3-(dimethylamino)propionic acid hydrochloride (113 mg), DMAP (22 mg) and EDCI (176 mg) were added, and the mixture was stirred at room temperature for 18 hours. Water was added to the mixture, followed by extraction with ethyl acetate. The organic layer was washed with a 5% aqueous sodium bicarbonate solution and saturated brine, then dried over sodium sulfate, and concentrated under reduced pressure. The residue was purified by silica gel column chromatography (MeOH/DCM) to obtain the title compound (230 mg).
¹H NMR (400 MHz, CDCl₃) δ 4.12 - 4.03 (m, 8H), 4.00 - 3.90 (m, 6H), 2.85 - 2.67(m, 2H), 2.60 - 2.50 (m, 2H), 2.44 - 2.25 (m, 12H), 2.24 - 2.18 (m, 6H), 1.69 -1.47 (m, 18H), 1.28 - 1.14 (m, 24H), 0.81 (t, J=6.8 Hz, 9H).
MS: [M+H]⁺ 914.6.

### [Example 33]

### 1,1'-Dibutyl 9,9'-[2-({[4-(dimethylamino)butanoyl]oxy}methyl)-2-{[(3-pentyloctanoyl)oxy]methyl}propane-1,3-diyl] dinonanedioate

### A) 9-Butoxy-9-oxononanoic acid

To a mixture of nonanedioic acid (20 g) and DCM (600 mL), DMAP (2.6 g), EDCI (20.4 g) and butan-1-ol (7.9 g) were added, and the mixture was stirred at room temperature for 12 hours. The mixture was washed with water, then dried over sodium sulfate, and concentrated under reduced pressure. The residue was purified by silica gel column chromatography (ethyl acetate/petroleum ether) to obtain the title compound (11 g). ¹H NMR (400 MHz, CDCl₃) δ 4.08 (t, J=6.4 Hz, 2H), 2.36 (t, J=7.6 Hz, 2H), 2.31 (t, J=7.6 Hz, 2H), 1.65-1.60 (m, 6H), 1.39-1.34 (m, 8H), 0.95 (t, J=7.2 Hz, 2H).

### B) 1,1'-Dibutyl 9,9'-[2-({ [tert-butyl(diphenyl)silyl]oxy}methyl)-2-{[(3-pentyloctanoyl)oxy]methyl}propane-1,3-diyl] dinonanedioate

To a mixture of 2-({[tert-butyl(diphenyl)silyl]oxy}methyl)-3-hydroxy-2-(hydroxymethyl)propyl 3-pentyloctanoate (1 g), 9-butoxy-9-oxononanoic acid (1.01 g) and DMF (10 mL), EDCI (1.01 g) and DMAP (107 mg) were added, and the mixture was stirred at room temperature for 12 hours. Ethyl acetate was added to the mixture, and the mixture was washed with water and saturated brine, then dried over sodium sulfate, and concentrated under reduced pressure. The residue was purified by silica gel column chromatography (ethyl acetate/petroleum ether) to obtain the title compound (1.5 g).
¹H NMR (400 MHz, CDCl₃) δ 7.55 - 7.53 (m, 4H), 7.39-7.28 (m, 6H), 4.05 (s, 6H),4.00 (t, J=6.8 Hz, 4H), 3.55 (s, 2H), 2.23-2.14 (m, 10H), 1.79 - 1.66 (m, 1H), 1.55-1.54 (m, 12H), 1.30-1.22 (m, 4H), 1.22-1.16 (m, 28H), 0.97 (s, 9H), 0.86 (t, J=7.2 Hz, 6H), 0.80 (t, J=6.8 Hz, 6H).

### C) 1,1'-Dibutyl 9,9'-[2-(hydroxymethyl)-2-{[(3-pentyloctanoyl)oxy]methyl}propane-1,3-diyl] dinonanedioate

To a mixture of 1,1'-dibutyl 9,9'-[2-({[tert-butyl(diphenyl)silyl]oxy}methyl)-2-{[(3-pentyloctanoyl)oxy]methyl}propane-1,3-diyl] dinonanedioate (1 g) and THF (30 mL), a solution of TBAF in THF (1 M, 1.17 mL) was added, and the mixture was stirred at room temperature for 12 hours. Ethyl acetate was added to the mixture, and the mixture was washed with saturated brine, then dried over sodium sulfate, and concentrated under reduced pressure. The residue was purified by silica gel column chromatography (ethyl acetate/petroleum ether) to obtain the title compound (0.6 g). ¹H NMR (400 MHz, CDCl₃) δ 4.13 - 4.09 (m, 10H), 3.51 (s, 2H), 2.60 (br s, 1H), 2.36-2.29 (m, 10H), 1.87 - 1.65 (m, 1H), 1.61-1.50 (m, 12H), 1.34-1.28 (m, 4H), 1.28-1.26 (m, 28H), 0.97 - 0.90 (m, 12H).

### D) 1,1'-Dibutyl 9,9'-[2-({[4-(dimethylamino)butanoyl]oxy}methyl)-2-{[(3-pentyloctanoyl)oxy]methyl}propane-1,3-diyl] dinonanedioate

To a mixture of 1,1'-dibutyl 9,9'-[2-(hydroxymethyl)-2-{[(3-pentyloctanoyl)oxy]methyl}propane-1,3-diyl] dinonanedioate (0.4 g), 4-(dimethylamino)butyric acid hydrochloride (102 mg) and DMF (10 mL), EDCI (146 mg) and DMAP (31 mg) were added, and the mixture was stirred at room temperature for 12 hours. Water was added to the mixture, followed by extraction with ethyl acetate. The organic layer was washed with a saturated aqueous solution of sodium bicarbonate and saturated brine, then dried over sodium sulfate, and concentrated under reduced pressure. The residue was purified by silica gel column chromatography (MeOH/DCM) to obtain the title compound (131 mg).
¹H NMR (400 MHz, CDCl₃) δ 4.03 - 3.97 (m, 12H), 2.55-2.20 (m, 20H), 1.90-1.45 (m, 15H), 1.24-1.18 (m, 32H), 0.88 - 0.79 (m, 12H) .
MS: [M+H]⁺ 898.7.

### [Example 39]

### 1,1'-{2-({[4-(Dimethylamino)butanoyl]oxy}methyl)-2-[({4-oxo-4-[(undecan-6-yl)oxy]butanoyl}oxy)methyl]propane-1,3-diyl} 4,4'-diundecan-6-yl dibutanedioate

### A) Undecane-6-ol

To a mixture of undecan-6-one (5 g) and THF (70 mL), sodium borohydride (1.3 g) was added at room temperature. After stirring for 2 hours, water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (4.91 g).
¹H NMR (400 MHz, CDCl₃) δ 3.56 - 3.47 (m, 1H), 1.45 - 1.17 (m, 16H), 0.82 (t, J=6.88 Hz, 6H).

### B) 4-(Benzyloxy)-4-oxobutanoic acid

To a mixture of succinic anhydride (30 g), benzyl alcohol (32.4 g) and DCM (800 mL), DMAP (3.7 g) and TEA (60.7 g) were added at 0°C, and the mixture was stirred for 3 hours. 1M Hydrochloric acid was added to the mixture, followed by extraction with DCM. The organic layer was dried over sodium sulfate and concentrated under reduced pressure. The residue was purified by silica gel column chromatography (ethyl acetate/petroleum ether) to obtain the title compound (54.1 g). ¹H NMR (400 MHz, CDCl₃) δ 7.47 - 7.31 (m, 5H) 5.17 (s, 2H) 2.80 - 2.63 (m, 4H).

### C) Undecane-6-ylbenzyl butanedioate

To a mixture of 4-(benzyloxy)-4-oxobutanoic acid (8.9 g), undecan-6-ol (4.9 g) and DMF (20 mL), EDCI (8.2 g) and DMAP (1.04 g) were added at room temperature, and the mixture was stirred for 1 hour. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (9.41 g).
¹H NMR (400 MHz, CDCl₃) δ 7.40 - 7.21 (m, 5H), 5.06 (s, 2H), 4.83 - 4.77 (m, 1H), 2.65 - 2.52 (m, 4H), 1.46 - 1.38 (m, 4H), 1.28 - 1.15 (m, 12H), 0.85 - 0.76 (m, 6H).

### D) 4,4'-Diundecane-6-yl dibutanedioate 1,1'-{2-({[tert-butyl(diphenyl)silyl]oxy]methyl)-2-[({4-oxo-4-[(undecane-6-yl)oxy]butanoyl}oxy)methyl]propane-1,3-diyl}

To a mixture of undecane-6-ylbenzyl butanedioate (9.4 g) and MeOH (120 mL), 10% Pd/C (2 g) was added, and the mixture was stirred under a hydrogen atmosphere (15 psi) at room temperature for 18 hours. The mixture was filtered and concentrated under reduced pressure. The residue was purified by silica gel column chromatography (MeOH/DCM) to obtain 4-oxo-4-[(undecane-6-yl)oxy]butanoic acid (6.47 g). To a mixture of 2-({[tert-butyl(diphenyl)silyl]oxy}methyl)-2-(hydroxymethyl)propane-1,3-diol (0.5 g), 4-oxo-4-[(undecane-6-yl)oxy]butanoic acid (1.16 g) and DMF (30 mL), EDCI (1.02 g) and DMAP (163 mg) were added and at room temperature, and the mixture was stirred for 2 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (1.4 g).
¹H NMR (400 MHz, CDCl₃) δ 7.70 - 7.58 (m, 4H) 7.53 - 7.35 (m, 6H) 4.92 - 4.85 (m, 3H) 4.14 (s, 6H) 3.65 (s, 2H) 2.58 (s, 12H) 1.57 - 1.48 (m, 12H) 1.34 - 1.25 (m, 36H) 1.06 (s, 9H) 0.91- 0.88 (m, 18H) .

### E) 4,4'-Diundecane-6-yl dibutanedioate 1,1'-{2-(hydroxymethyl)-2-[({4-oxo-4-[(undecane-6-yl)oxy]butanoyl)}oxy)methyl]propane-1,3-diyl}

To a mixture of 4,4'-diundecane-6-yl dibutanedioate 1,1'-{2-({[tert-butyl(diphenyl)silyl]oxy}methyl)-2-[({4-oxo-4-[(undecane-6-yl)oxy]butanoyl}oxy)methyl]propane-1,3-diyl} (1.4 g) and THF (15 mL), a THF solution of TBAF (1 M, 1.35 mL) was added, and the mixture was stirred for 16 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (614 mg).
¹H NMR (400 MHz, CDCl₃) δ 4.98 - 4.79 (m, 3H), 4.17 (s, 6H), 3.55 (s, 2H), 2.68 (s, 12H), 1.61 - 1.49 (m, 12H), 1.36 - 1.23 (m, 36H), 0.90 (t, J=6.75 Hz, 18H).

### F) 1,1'-12-(1[4-(Dimethylamino)butanoyl]oxy}methyl)-2-[({4-oxo-4-[(undecane-6-yl)oxy]butanoyl}oxy)methyl]propane-1,3-diyl} 4,4'-diundecane-6-yl dibutanedioate

To a mixture of 4-(dimethylamino)butyric acid hydrochloride (80 mg), 4,4'-diundecane-6-yl dibutanedioate 1,1'-{2-(hydroxymethyl)-2-[({4-oxo-4-[(undecane-6-yl)oxy]butanoyl}oxy)methyl]propane-1,3-diyl} (214 mg) and DMF (5 mL), EDCI (114 mg) and DMAP (14.5 mg) was added, and the mixture was stirred at room temperature for 15 hours. Water was added to the mixture, followed by extraction with ethyl acetate. The organic layer was washed with saturated aqueous sodium hydrogencarbonate solution and saturated brine, then dried over sodium sulfate, and concentrated under reduced pressure. The residue was purified by silica gel column chromatography (MeOH/DCM) to obtain the title compound (231 mg).
¹H NMR (400 MHz, CDCl₃) δ = 4.82 - 4.76 (m, 3H), 4.12 - 4.00 (m, 8H), 2.75 - 2.64 (m, 2H), 2.59 - 2.48 (m, 18H), 2.37 (t, J=6.94 Hz, 2H), 1.99 - 1.89 (m, 2H), 1.50 - 1.39 (m, 12H), 1.29 - 1.20 (m, 36H), 0.81 (t, J=6.75 Hz, 18H).
MS: [M+H]⁺ 1012.7.

### [Example 51]

### Decyl 2-({[4-(dimethylamino)butanoyl]oxy}methyl)-3-[(3-pentyloctanoyl)oxy]-2-{[(3-pentyloctanoyl)oxy]methyl}propyl hexanedioate

### A) 6-(Decyloxy)-6-oxohexanoic acid

To a mixture of oxepane-2,7-dione (5 g), decan-1-ol (6.8 g) and DCM (50 mL), DMAP (477 mg) and TEA (7.9 g) were added at 0°C, and the mixture was stirred for 3 hours. 1M Hydrochloric acid was added to the mixture, followed by extraction with DCM. The organic layer was dried over sodium sulfate and concentrated under reduced pressure. The residue was purified by silica gel column chromatography (MeOH/DCM) to obtain the title compound (3.8 g).
¹H NMR (400 MHz, CDCl₃) δ 12.02 (br s, 1H), 3.99 (t, J=6.5 Hz, 2H), 2.28 (t, J=6.9 Hz, 2H), 2.20 (t, J=6.9 Hz, 2H), 1.58 - 1.46 (m, 6H), 1.42 - 1.13 (m, 14H), 0.85 (t, J=6.7 Hz, 3H).

### B) Decyl hexanedioate 2-({[tert-butyl(diphenyl)silyl]oxy}methyl)-3-[(3-pentyloctanoyl)oxy]-2-{[(3-pentyloctanoyl)oxy] methyl} propyl

To a mixture of bis(3-pentyloctanoic acid) 2-({[tert-butyl(diphenyl)silyl]oxy}methyl)-2-(hydroxymethyl)propane-1,3-diyl (700 mg), 6-(decyloxy)-6 - oxohexanoic acid (340 mg) and DMF (15 mL), DMAP (56 mg) and EDCI (350 mg) were added at room temperature, and the mixture was stirred for 12 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtaion the title compound (880 mg).
¹H NMR (400 MHz, CDCl₃) δ 7.61 (dd, J=1.4, 7.9 Hz, 4H), 7.46 - 7.35 (m, 6H), 4.12 (s, 6H), 4.08 - 4.02 (m, 2H), 3.62 (s, 2H), 2.32 - 2.15 (m, 8H), 1.85 - 1.70 (m, 2H), 1.64 - 1.57 (m, 6H), 1.32 - 1.17 (m, 46H), 1.04 (s, 9H), 0.90 - 0.84 (m, 15H).

### C) Hexanedioate 2-(hydroxymethyl)-3-[(3-pentyloctanoyl)oxy]-2-{[(3-pentyloctanoyl)oxy]methyl}propyldecyl

To a mixture of decyl hexanedioate 2-({[tert-butyl(diphenyl)silyl]oxy}methyl)-3-[(3-pentyloctanoyl)oxy]-2-{[(3-pentyloctanoyl)oxy]methyl} propyl (300 mg) and THF (12 mL), acetic acid (35 mg) was added, and the mixture was stirred at room temperature for 5 minutes. A solution of TBAF in THF (1 M, 0.72 mL) was added to the mixture, and the mixture was stirred at room temperature for 12 hours. A saturated aqueous sodium hydrogencarbonate solution was added to the mixture, followed by extraction with ethyl acetate. The organic layer was dried over sodium sulfate and concentrated under reduced pressure. The residue was purified by silica gel column chromatography (ethyl acetate/petroleum ether) to obtain the title compound (185 mg). ¹H NMR (400 MHz, CDCl₃) δ 4.10 (d, J=2.3 Hz, 6H), 4.05 (t, J=6.8 Hz, 2H), 3.48 (s, 2H), 2.60 (br s, 1H), 2.37 - 2.24 (m, 8H), 1.85 - 1.70 (m, 2H), 1.67 - 1.59 (m, 6H), 1.32 - 1.21 (m, 46H), 0.88 (t, J=7.0 Hz, 15H).

### D) Decyl 2-({[4-(dimethylamino)butanoyl]oxy}methyl)-3-[(3-pentyloctanoyl)oxy]-2-{[(3-pentyloctanoyl)oxy]methyl}propyl hexanedioate

To a mixture of hexanedioate 2-(hydroxymethyl)-3-[(3-pentyloctanoyl)oxy]-2-{[(3-pentyloctanoyl)oxy]methyl}propyldecyl (249 mg) and DMF (6 mL), 4-(dimethylamino)butyric acid hydrochloride (105 mg), DMAP (19 mg) and EDCI (150 mg) were added, and the mixture was stirred at room temperature for 18 hours. Water and saturated brine were added to the mixture, followed by extraction with ethyl acetate. The organic layer was washed with saturated aqueous sodium hydrogencarbonate solution and saturated brine, then dried over sodium sulfate, and concentrated under reduced pressure. The residue was purified by silica gel column chromatography (MeOH/DCM) to obtain the title compound (229 mg).
¹H NMR (400 MHz, CDCl₃) δ = 4.10 (s, 8H), 4.05 (t, J=6.8 Hz, 2H), 2.42 - 2.28 (m, 14H), 2.24 (d, J=6.8 Hz, 4H), 1.87 - 1.81 (m, 4H), 1.68 - 1.55 (m, 6H), 1.32 - 1.21 (m, 46H), 0.87 (t, J=6.9 Hz, 15H).
MS: [M+H]⁺ 910.7.

### [Example 70]

### 1,1'-[2-({[4-(Dimethylamino)butanoyl]oxy}methyl)-2-{[(3-pentyloctanoyl)oxy]methyl}propane-1,3-diyl] 3,3'-dinonyl dipropanedioate

### A) 3-(Nonyloxy)-3-oxopropanoic acid

To a mixture of 2,2-dimethyl-1,3-dioxane-4,6-dione (1 g) and toluene (10 mL), 1-nonanol (1 g) was added, and the mixture was stirred at 120°C for 3 hours. The mixture was cooled to room temperature, and water was added thereto, followed by extraction with ethyl acetate. The organic layer was washed with saturated brine, then dried over sodium sulfate, and concentrated under reduced pressure. The residue was purified by silica gel column chromatography (MeOH/DCM) to obtain the title compound (0.98 g).
¹H NMR (400 MHz, CDCl₃) δ 4.19 (t, J=6.8 Hz, 2H), 3.44 (s, 2H), 1.72 - 1.62 (m,2H), 1.38 -1.22 (m, 12H), 0.92 - 0.84 (m, 3H).

### B) 1,1'-[2-({[tert-Butyl(diphenyl)silyl]oxy}methyl)-2-{[(3-pentyloctanoyl)oxy]methyl}propane-1,3-diyl] 3,3'-dinonyl dipropanedioate

To a mixture of 2-({[tert-butyl(diphenyl)silyl]oxy}methyl)-3-hydroxy-2-(hydroxymethyl)propyl 3-pentyloctanoate (600 mg) and DCM (10 mL), 3-(nonyloxy)-3-oxopropanoic acid (605 mg) and DCC (650 mg) were added, and the mixture was stirred at room temperature for 18 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (810 mg). ¹H NMR (400 MHz, CDCl₃) δ 7.64-7.62 (m, 4H), 7.49 - 7.37 (m, 6H), 4.23 (s, 4H),4.16 - 4.07 (m, 6H), 3.65 (s, 2H), 3.32 (s, 4H), 2.21 (dd, J=1.7, 6.8 Hz, 2H), 1.81 (m, 1H), 1.67 - 1.60 (m, 4H), 1.36 - 1.23 (m, 40H), 1.06 (s, 9H), 0.94 - 0.85 (m, 12H).

### C) 1,1'-[2-(Hydroxymethyl)-2-{[(3-pentyloctanoyl)oxy]methyl]propane-1,3-diyl] 3,3'-dinonyl dipropanedioate

To a mixture of 1,1'-[2-({[tert-butyl(diphenyl)silyl]oxy}methyl)-2-{[(3-pentyloctanoyl)oxy]methyl}propane-1,3-diyl] 3,3'-dinonyl dipropanedioate (810 mg) and THF (20 mL), a solution of acetic acid (98 mg) and TBAF in THF (1 M, 2.03 mL) was added, and the mixture was stirred at room temperature for 12 hours. An aqueous sodium bicarbonate solution was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (425 mg). ¹H NMR (400 MHz, CDCl₃) δ 4.26 - 4.20 (m, 4H), 4.18 - 4.11 (m, 6H), 3.58 (s, 2H), 3.47 - 3.37 (m, 4H), 2.28 (d, J=6.5 Hz, 2H), 1.86 (m, 1H), 1.70-1.62 (m, 4H), 1.38 - 1.25 (m, 40H), 0.94 - 0.86 (m, 12H).

### D) 1,1'-[2-(1[4-(Dimethylamino)butanoyl]oxy}methyl)-2-{[(3-pentyloctanoyl)oxy]methyl}propane-1,3-diyl] 3,3'-dinonyl dipropanedioate

To a mixture of 1,1'-[2-(hydroxymethyl)-2-{[(3-pentyloctanoyl)oxy]methyl}propane-1,3-diyl] 3,3'-dinonyl dipropanedioate (425 mg) and DMF (10 mL), 4-(dimethylamino)butyric acid hydrochloride (141 mg), DMAP (34 mg) and EDCI (215 mg) were added, and the mixture was stirred at room temperature for 12 hours. Water was added to the mixture, followed by extraction with ethyl acetate. The organic layer was washed with a saturated aqueous solution of sodium bicarbonate and saturated brine, then dried over sodium sulfate, and concentrated under reduced pressure. The residue was purified by silica gel column chromatography (MeOH/DCM) to obtain the title compound (240 mg).
¹H NMR (400 MHz, CDCl₃) δ 4.21 (s, 4H), 4.18 - 4.08 (m, 8H), 3.40 (s, 4H), 2.66(m, 2H), 2.57 - 2.39 (m, 8H), 2.30 - 2.21 (m, 2H), 2.04 - 1.91 (m, 2H), 1.83 (m, 1H), 1.70-1.62 (m, 4H), 1.37 - 1.22 (m, 40H), 0.95 - 0.82 (m, 12H).
MS: [M+H]⁺ 870.7.

### [Example 73]

### 2-({[4-(Dimethylamino)butanoyl]oxy}methyl)-3-[(3-pentyloctanoyl)oxy]-2-{[(3-pentyloctanoyl)oxy]methyl}propyl nonyl propanedioate

### A) 2-({[tert-Butyl(diphenyl)silyl]oxy}methyl)-2-(hydroxymethyl)propane-1,3-diyl bis(3-pentyloctanoate)

To a mixture of 2-({[tert-butyl(diphenyl)silyl]oxy]methyl)-2-(hydroxymethyl)propane-1,3-diol (10.0 g), 3-pentyloctanoic acid (9.16 g) and DMF (150 mL), EDCI (10.24 g) and DMAP (979 mg) were added, and the mixture was stirred at room temperature for 15 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (6.57 g).
¹H NMR (400 MHz, CDCl₃) δ 7.65 (d, J=6.63 Hz, 4H), 7.49 - 7.34 (m, 6H),4.16 (s, 4H), 3.65 (s, 2H), 3.54 (s, 2H), 2.49 (br s, 1H), 2.21 (d, J=6.88 Hz, 4H), 1.81-1.65 (m, 2H), 1.37 - 1.17 (m, 32H), 1.06 (s, 9H), 0.88 (t, J=6.88 Hz, 12H).

### B) Nonyl 2-({[tert-butyl(diphenyl)silyl]oxy}methyl)-3-[(3-pentyloctanoyl)oxy]-2-{[(3-pentyloctanoyl)oxy]methyl}propyl propanedioate

To a mixture of 2-({[tert-butyl(diphenyl)silyl]oxy}methyl)-2-(hydroxymethyl)propane-1,3-diyl bis(3-pentyloctanoate) (600 mg) and DCM (10 mL), DCC (484 mg) and 3-(nonyloxy)-3-oxopropanoic acid (360 mg) were added, and the mixture was stirred at room temperature for 12 hours. An aqueous sodium bicarbonate solution was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (542 mg).
¹H NMR (400 MHz, CDCl₃) δ 7.60 - 7.50 (m, 4H), 7.43 - 7.27 (m, 6H), 4.17 - 3.96 (m, 8H), 3.56 (s, 2H), 3.22 (s, 2H), 2.15-2.09 (m, 4H), 1.71 (br s, 2H),1.23 - 1.12 (m, 46H), 0.97 (s, 9H), 0.78 - 0.82 (m, 15H).

### C) Nonyl 2-(hydroxymethyl)-3-{(3-pentyloctanoyl)oxy]-2-{[(3-pentyloctanoyl)oxy]methyl}propyl propanedioate

To a mixture of nonyl 2-({[tert-butyl(diphenyl)silyl]oxy}methyl)-3-[(3-pentyloctanoyl)oxy]-2-[{(3-pentyloctanoyl)oxy]methyl}propyl propanedioate (540 mg) and THF (10 mL), a solution of acetic acid (66 mg) and TBAF in THF (1 M, 1.38 mL) was added, and the mixture was stirred at room temperature for 12 hours. An aqueous sodium bicarbonate solution was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain the title compound (200 mg).
¹H NMR (400 MHz, CDCl₃) δ 4.23 (s, 2H), 4.19 - 4.12 (m, 6H), 3.54 (s, 2H), 3.42 (s, 2H), 2.29 (d, J=6.78 Hz, 4H), 1.85 (br s, 2H), 1.37 - 1.26 (m, 46H), 0.90 (t, J=6.90 Hz, 15H).

### D) 2-({[4-(Dimethylamino)butanoyl]oxy}methyl)-3-[(3-pentyloctanoyl)oxy]-2-[{(3-pentyloctanoyl)oxy]methyl}propyl nonyl propanedioate

To a mixture of nonyl 2-(hydroxymethyl)-3-[(3-pentyloctanoyl)oxy]-2-{(3-pentyloctanoyl)oxy]methyl}propyl propanedioate (200 mg) and DMF (6 mL), EDCI (129 mg), DMAP (16.5 mg) and 4-(dimethylamino)butyric acid hydrochloride (91 mg) were added, and the mixture was stirred at room temperature for 12 hours. Water was added to the mixture, followed by extraction with ethyl acetate. The organic layer was washed with saturated brine, then dried over sodium sulfate, and concentrated under reduced pressure. The residue was purified by silica gel column chromatography (MeOH/DCM) to obtain the title compound (160 mg). ¹H NMR (400 MHz, CDCl₃) δ 4.22 (s, 2H), 4.18 - 4.09 (m, 8H), 3.40 (s, 2H), 2.42-2.40 (m, 4H), 2.33 (s, 6H), 2.27 (d, J=6.88 Hz, 4H), 1.89 - 1.76 (m, 6H), 1.38 - 1.25 (m, 44H), 0.90 (t, J=6.88 Hz, 15H).
MS: [M+H]⁺ 854.7.

### [Example 93]

### 1,1'-Diheptyl 6,6'-(2-[({4-[(2-hydroxyethyl)(methyl)amino]butanoyl}oxy)methyl]-2-{[(3-pentyloctanoyl)oxy]methyl}propane-1,3-diyl) dihexanedioate

### A) 4-(Benzyloxy)-4-oxobutanoic acid

To a mixture of succinic anhydride (30 g), benzyl alcohol (32.4 g) and DCM (800 mL), DMAP (3.7 g) and TEA (60.7 g) were added at 0°C, and the mixture was stirred for 3 hours. 1 M hydrochloric acid was added to the mixture, followed by extraction with DCM. The organic layer was dried over sodium sulfate and concentrated under reduced pressure. The residue was purified by silica gel column chromatography (ethyl acetate/petroleum ether) to obtain the title compound (54.1 g). ¹H NMR (400 MHz, CDCl₃) δ 7.47 - 7.31 (m, 5 H) 5.17 (s, 2 H) 2.80 - 2.63 (m, 4 H).

### B) Benzyl 4-hydroxybutanoate

A mixture of 4-(benzyloxy)-4-oxobutanoic acid (45 g) and THF (500 mL) was stirred, and a borane-dimethyl sulfide complex (28.1 mL) was added dropwise thereto. 15 hours later, methanol was gradually added to the mixture. 1 hour later, the mixture was concentrated under reduced pressure. The residue was purified by silica gel column chromatography (ethyl acetate/petroleum ether) to obtain the title compound (40.1 g). ¹H NMR (400 MHz, CDCl₃) δ 7.48 - 7.31 (m, 5 H), 5.15 (s, 2 H), 4.52 (t, J=6.13 Hz, 1 H), 3.71 (t, J=6.13 Hz, 2 H), 2.52 (t, J=7.13 Hz, 2 H), 1.96-1.89 (m, 2 H).

### C) Benzyl 4-[(methanesulfonyl)oxy]butanoate

To a mixture of benzyl 4-hydroxybutanoate (30 g) and THF (350 mL), TEA (20.3 g) was added, then methanesulfonyl chloride (20.7 g) was added dropwise at 0°C, and the mixture was then stirred for 30 minutes. After further stirring at room temperature for 1 hour, the mixture was filtered. The filtrate was concentrated under reduced pressure to obtain the title compound (31.1 g).
¹H NMR (400 MHz, CDCl₃) δ 7.44 - 7.30 (m, 5 H) 5.15 (s, 2 H) 4.30 (t, J=6.19 Hz, 2 H) 2.97 (s, 3 H) 2.55 (t, J=7.19 Hz, 2 H) 2.15 - 2.08 (m, 2 H).

### D) 2-{[tert-Butyl(dimethyl)silyl]oxy}-N-methylethan-1-amine

To a mixture of 2-(methylamino)ethanol (10 g) and DCM (200 mL), tert-butyldimethylchlorosilane (24.1 g) and imidazole (9.1 g) were added, and the mixture was stirred at room temperature for 12 hours. Water was added to the mixture, followed by extraction with DCM. The organic layer was washed with saturated brine, then dried over sodium sulfate, and concentrated under reduced pressure. The residue was purified by silica gel column chromatography (MeOH/DCM) to obtain the title compound (13 g).
¹H NMR (400 MHz, CDCl₃) δ 3.73 (t, J=5.27 Hz, 2 H), 2.69 (t, J=5.27 Hz, 2 H), 2.46 (s, 3 H), 1.78 (s, 1 H), 0.90 (s, 9 H), 0.07 (s, 6 H).

### E) 4-[(2-{[tert-Butyl(dimethyl)silyl]oxy}ethyl)(methyl)amino]butanoic acid

To a mixture of benzyl 4-[(methanesulfonyl)oxy]butanoate (5 g) and EtOH (20 mL), N,N-diisopropylethylamine (4.8 g) and 2-{[tert-butyl(dimethyl)silyl]oxy}-N-methylethan-1-amine (10.4 g) were added, and the mixture was stirred at 50°C for 12 hours. Water was added to the mixture, followed by extraction with ethyl acetate. The organic layer was washed with saturated brine, then dried over sodium sulfate, and concentrated under reduced pressure. The residue was purified by silica gel column chromatography (ethyl acetate/petroleum ether) to obtain benzyl 4-[(2-{[tert-butyl(dimethyl)silyl]oxy}ethyl) (methyl)amino]butanoate (340 mg). This compound was diluted with EtOH (20 mL). To the dilution, 10% Pd/C (200 mg) was added, and the mixture was stirred at room temperature for 12 hours in a hydrogen atmosphere (15 psi). The mixture was filtered and concentrated under reduced pressure. The residue was purified by silica gel column chromatography (ethyl acetate/petroleum ether) to obtain the title compound (220 mg) .
¹H NMR (400 MHz, CDCl₃) δ 3.91 - 3.89 (m, 2 H), 2.91 - 2.85 (m, 4 H), 2.65 - 2.61 (m, 2 H), 2.57 (s, 3 H), 1.82 - 1.89 (m, 2 H), 0.91 (s, 9 H), 0.10 (s, 6 H).

### F) 1,1'-Diheptyl 6,6'-(2-[({4-[(2-{[tert-butyl(dimethyl)silyl]oxy}ethyl)(methyl)amino]butanoyl}oxy)methyl ]-2-{[(3-pentyloctanoyl)oxy]methyl}propane-1,3-diyl) dihexanedioate

To a mixture of 4-[(2-{[tert-butyl(dimethyl)silyl]oxy}ethyl) (methyl)amino]butanoic acid (326 mg) and DMF (20 mL), EDCI (303 mg), DMAP (48 mg) and 1,1'-diheptyl 6,6'-[2-(hydroxymethyl)-2-{[(3-pentyloctanoyl)oxy]methyl}propane-1,3-diyl] dihexanedioate (620 mg) were added, and the mixture was stirred at room temperature for 12 hours. Water was added to the mixture, followed by extraction with ethyl acetate. The organic layer was washed with saturated brine, then dried over sodium sulfate, and concentrated under reduced pressure. The residue was purified by silica gel column chromatography (MeOH/DCM) to obtain the title compound (270 mg).
¹H NMR (400 MHz, CDCl₃) δ 4.17 - 4.02 (m, 12 H), 3.85 - 3.73 (m, 2 H), 2.62 - 2.21 (m, 19 H), 1.87 -1.76 (m, 3 H), 1.71 - 1.52 (m, 12 H), 1.38 - 1.16 (m, 32 H), 0.95 - 0.82 (m, 21 H), 0.07 (s, 6 H) .

### G) 1,1'-Diheptyl 6,6'-(2-[({4-[(2-hydroxyethyl)(methyl)amino]butanoyl}oxy)methyl]-2-{[(3-pentyloctanoyl)oxy]methyl}propane-1,3-diyl) dihexanedioate

To a mixture of 1,1'-diheptyl 6,6'-(2-[({4-[(2-{[tert-butyl(dimethyl)silyl]oxy}ethyl)(methyl)amino]butanoyl}oxy)methyl ]-2-{[(3-pentyloctanoyl)oxy]methyl}propane-1,3-diyl) dihexanedioate (270 mg) and THF (35 mL), a solution of TBAF in THF (1 M, 0.65 mL) and acetic acid (31 mg) were added, and the mixture was stirred at room temperature for 12 hours. Water was added to the mixture, followed by extraction with ethyl acetate. The organic layer was washed with an aqueous sodium bicarbonate solution and saturated brine, then dried over sodium sulfate, and concentrated under reduced pressure. The residue was purified by silica gel column chromatography (MeOH/DCM) to obtain the title compound (106 mg).
¹H NMR (400 MHz, CDCl₃) δ 4.13 (s, 8 H), 4.08 (t, J=6.79 Hz, 4 H), 3.78 -3.77 (m, 2 H), 2.90 - 2.75 (m, 4 H), 2.52 (s, 3 H), 2.47 - 2.41 (m, 2 H), 2.40 - 2.31(m, 8 H), 2.27 (d, J=6.85 Hz, 2 H), 2.02 - 1.91 (m, 3 H), 1.70 - 1.57 (m, 12 H), 1.44 - 1.22 (m, 32 H), 0.95 - 0.87 (m, 12 H).
MS: [M+H]⁺ 928.7.

### [Example 115]

### 1,1'-[2-({[4-(Dimethylamino)butanoylloxylmethyl)-2-{[(3-pentyloctanoyl)oxy]methyl}propane-1,3-diyl] 6,6'-bis(2-propylpentyl) dihexanedioate

### A) 2-Propylpentan-1-ol

To a mixture of 2-propylpentanoic acid (20 g) and THF (200 mL), borane dimethylsulfide complex (16.6 mL) was added dropwise at 0°C, and the mixture was stirred at room temperature for 2 hours. Water was added to the mixture at 0°C, followed by extraction with ethyl acetate. The organic layer was washed with saturated aqueous sodium hydrogencarbonate solution and saturated brine, then dried over sodium sulfate, and concentrated under reduced pressure. The residue was purified by silica gel column chromatography (ethyl acetate/petroleum ether) to obtain the title compound (16 g).
¹H NMR (400 MHz, CDCl₃) δ = 3.47 (d, J=5.6 Hz, 2H), 1.42 (td, J=5.6, 11.2 Hz, 1H), 1.29 - 1.17 (m, 8H), 0.88 - 0.80 (m, 6H).

### B) 6-Oxo-6-[(2-propylpentyl)oxy]hexanoic acid

To a mixture of hexanedioic acid (3 g), 2-propylpentan-1-ol (2.1 g) and DCM (50 mL), EDCI (4.7 g) and DMAP (1.3 g) were added at room temperature, and the mixture was stirred for 12 hours. 0.5M Hydrochloric acid was added to the mixture, followed by extraction with ethyl acetate. The organic layer was dried over sodium sulfate and concentrated under reduced pressure. The residue was purified by silica gel column chromatography (ethyl acetate/petroleum ether) to obtain the title compound (2.3 g).
¹H NMR (400 MHz, CDCl₃) δ 4.00 (d, J=6.0 Hz, 2H), 2.51 - 2.27 (m, 4H), 1.81 - 1.64 (m, 5H), 1.42 - 1.27 (m, 8H), 0.92 (t, J=6.8 Hz, 6H).

### C) Dihexanedioate 6,6'-bis(2-propylpentyl) 1,1'-[2-({[tert-butyl(diphenyl)silyl]oxy}methyl)-2-{[(3-pentylocta noyl)oxy]methyl}propane-1,3-diyl]

2-({[tert-butyl(diphenyl)silyl]oxy}methyl)-3-hydroxy-2-(hydroxymethyl)propyl 3-pentyl octanoate (1 g), 6-oxo-6-[(2-propyl pentyl)oxy]hexanoic acid (1.1 g) and DMF (20 mL), DMAP (107 mg) and EDCI (1.0 g) were added at room temperature, and the mixture was stirred for 12 hours. Ethyl acetate was added to the mixture, and the mixture was washed with saturated brine, then dried over sodium sulfate, and concentrated under reduced pressure. The residue was purified by silica gel column chromatography (ethyl acetate/petroleum ether) to obtain the title compound (1.7 g).
¹H NMR (400 MHz, CDCl₃) δ 7.66 - 7.59 (m, 4H), 7.49 - 7.34 (m, 6H), 4.14 (s, 6H), 3.99 (d, J=6.0 Hz, 4H), 3.64 (s, 2H), 2.30 (td, J=7.2,18.0 Hz, 8H), 2.21 (d, J=6.8 Hz, 2H), 1.88 - 1.75 (m, 1H), 1.72 - 1.62 (m, 10H), 1.42 - 1.22 (m, 32H), 1.06 (s, 9H), 0.98 - 0.84 (m, 18H).

### D) Dihexanedioate 6,6'-bis(2-propylpentyl) 1,1'-[2-(hydroxymethyl)-2-{[(3-pentyloctanoyl)oxy]methyl}propane-1,3-diyl]

To a mixture of dihexanedioate 6,6'-Bis(2-propylpentyl) 1,1'-[2-({[tert-butyl(diphenyl)silyl]oxy]methyl)-2-{[(3-pentyloctanoyl)oxy]methyl}propane-1,3-diyl] (1.7 g) and THF (40 mL), acetic acid (194 mg) and a THF solution of TBAF (1 M, 4.0 mL) were added, and the mixture was stirred at room temperature for 12 hours. Ethyl acetate was added to the mixture, and the mixture was washed with saturated brine, then dried over sodium sulfate, and concentrated under reduced pressure. The residue was purified by silica gel column chromatography (ethyl acetate/petroleum ether) to obtain the title compound (1.15 g). ¹H NMR (400 MHz, CDCl₃) δ 4.04 (s, 6H), 3.90 (d, J=6.0 Hz, 4H), 3.43 (s, 2H), 2.54 (br s, 1H), 2.34 - 2.22 (m, 8H), 2.19 (d, J=6.8 Hz, 2H), 1.82 - 1.70 (m, 1H), 1.65 - 1.54 (m, 10H), 1.30 - 1.13 (m, 32H), 0.90 - 0.72 (m, 18H).

### E) 1,1'-[2-({[4-(Dimethylamino)butanoyl]oxy}methyl)-2-{[(3-pentyloctanoyl)oxy]methyl}propane-1,3-diyl] 6,6'-bis(2-propylpentyl) dihexanedioate

To a mixture of dihexanedioate 6,6'-bis(2-propylpentyl) 1,1'-[2-(hydroxymethyl)-2-{[(3-pentyloctanoyl)oxy]methyl}propane-1,3-diyl] (400 mg), 4-(dimethylamino)butyric acid hydrochloride (107 mg) and DMF (10 mL), EDCI (151 mg) and DMAP (30 mg) were added, and the mixture was stirred at room temperature for 12 hours. Water was added to the mixture, followed by extraction with ethyl acetate. The organic layer was washed with saturated aqueous sodium hydrogencarbonate solution and saturated brine, then dried over sodium sulfate, and concentrated under reduced pressure. The residue was purified by silica gel column chromatography (MeOH/DCM) to obtain the title compound (280 mg).
¹H NMR (400 MHz, CDCl₃) δ = 4.04 (s, 8H), 3.90 (d, J=6.0 Hz, 4H), 2.33 - 2.13 (m, 20H), 1.76 - 1.66 (m, 3H), 1.60 - 1.56 (m, 10H), 1.29 -1.14 (m, 32H), 0.89 - 0.74 (m, 18H).
MS: [M+H]⁺ 926.7

### [Example 119]

### 1,1'-{2-({[4-(Dimethylamino)butanoyl]oxy}methyl)-2-[({3-oxo-3-[(2-pentylheptyl)oxy]propanoyl}oxy)methyl]propane-1,3-diyl} 3,3'-bis(2-pentylheptyl) dipropanedioate

### A) 6-Methylideneundecane

To a mixture of methyltriphenylphosphonium bromide (42.0 g) and THF (300 mL), potassium tert-butoxide (13.2 g) was gradually added at 0°C, and the mixture was stirred for 15 minutes. Undecan-6-one (10.0 g) was added thereto, and the mixture was stirred at room temperature for 12 hours. Water was added to the mixture, followed by extraction with ethyl acetate. The organic layer was washed with saturated brine, dried over sodium sulfate, and concentrated under reduced pressure. The residue was purified by silica gel column chromatography (petroleum ether) to obtain the title compound (3 g).
¹H NMR (400 MHz, CDCl₃) δ 4.72 (s, 2 H), 2.07-1.95 (m, 4H), 1.45 - 1.23 (m, 12H), 0.85 - 0.78 (m, 6H).

### B) 2-Pentylheptan-1-ol

To a mixture of 6-methylideneundecane (7.1 g) and THF (75 mL), a THF solution of borane THF complex (1 M, 169 mL) was added at 0°C, and the mixture was stirred for 1 hour and then at room temperature for 2 hours. An aqueous sodium hydroxide solution (4 M, 52.7 mL) was added to the mixture, and the mixture was stirred for 10 minutes. A 30% hydrogen peroxide solution (20.3 mL) was added at 0°C, and the mixture was stirred at room temperature for 12 hours. A saturated aqueous sodium sulfite solution (150 mL) was added to the mixture, followed by extraction with ethyl acetate. The organic layer was washed with saturated brine, dried over sodium sulfate, and concentrated under reduced pressure. The residue was purified by silica gel column chromatography (ethyl acetate/petroleum ether) to obtain the title compound (4.5 g).
¹H NMR (400 MHz, CDCl₃) δ 3.55 (d, J=5.5 Hz, 2H), 1.46 - 1.45 (m, 1H), 1.38 - 1.22 (m, 16H), 0.90 (t, J=6.8 Hz, 6H).

### C) 3-Oxo-3-[(2-pentylheptyl)oxy]propanoic acid

To a mixture of 2-pentylheptan-1-ol (3.8 g) and toluene (60 mL), 2,2-Dimethyl-1,3-dioxane-4,6-dione (3.3 g) was added, and the mixture was stirred at 120°C for 16 hours. The mixture was concentrated under reduced pressure, and the residue was poured into water, followed by extraction with petroleum ether. The organic layer was dried over sodium sulfate and concentrated under reduced pressure. The residue was purified by silica gel column chromatography (MeOH/DCM) to obtain the title compound (3.0 g).
¹H NMR (400 MHz, CDCl₃) δ 4.10 (d, J=5.8 Hz, 2H), 3.45 (s, 2H), 1.73-1.62 (m, 1H), 1.38 - 1.19 (m, 16H), 0.89 (t, J=6.9 Hz, 6H).

### D) Dipropanedioic acid 3,3'-bis(2-pentylheptyl) 1,1'-{2-({[tert-butyl(diphenyl)silyl]oxy}methyl)-2-[({3-oxo-3-[(2-pentylheptyl)oxy]propanoyl}oxy)methyl]propane-1,3-diyl}

To a mixture of 2-({[tert-butyl(diphenyl)silyl]oxy}methyl)-2-(hydroxymethyl)propane-1,3-diol (700 mg) and DCM (15 mL), 3-oxo-3 -[(2-Pentylheptyl)oxy]propanoic acid (2.0 g) and DCC (1.9 g) were added, and the mixture was stirred at room temperature for 16 hours. The mixture was filtered and the filtrate was concentrated under reduced pressure. The residue was purified by silica gel column chromatography (ethyl acetate/petroleum ether) to obtain the title compound (2.0 g).
¹H NMR (400 MHz, CDCl₃) δ 7.63 - 7.61 (m, 4H), 7.48 - 7.37 (m, 6H), 4.22 (s, 6H), 4.03 (d, J=5.8 Hz, 6H), 3.64 (s, 2H), 3.32 (s, 6H), 1.10 - 1.60 (m, 3H), 1.36 - 1.23 (m, 48H), 1.05 (s, 9H), 0.93 - 0.79 (m, 18H).

### E) Dipropanedioic acid 3,3'-bis(2-pentylheptyl) 1,1'-{2-(hydroxymethyl)-2-[({3-oxo-3-[(2-pentylheptyl)oxy]propanoyl}oxy)methyl]propane-1,3-diyl}

To a mixture of dipropanedioate 3,3'-bis(2-pentylheptyl) 1,1'-{2-({[tert-butyl(diphenyl)silyl]oxy}methyl)-2-[({3-oxo-3-[(2-pentylheptyl)oxy]propanoyl}oxy)methyl]propane-1,3-diyl} (950 mg) and THF (40 mL), acetic acid (100 mg) and THF solution of TBAF (1 M, 2.1 mL) were added, and the mixture was stirred at room temperature for 16 hours. Water was added to the mixture, followed by extraction with ethyl acetate. The organic layer was dried over sodium sulfate and concentrated under reduced pressure. The residue was purified by silica gel column chromatography (ethyl acetate/petroleum ether) to obtain the title compound (690 mg).
¹H NMR (400 MHz, CDCl₃) δ 4.23 (s, 6H), 4.06 (d, J=5.9 Hz, 6H), 3.60 (s, 2H), 3.42 (s, 6H), 1.70 -1.60 (m, 3H), 1.36 - 1.23 (m, 48H), 0.89 (t, J=6.9 Hz, 18H).

### F) 1,1'-{2-({[4-(Dimethylamino)butanoyl]oxy}methyl)-2-[({3-oxo-3-[(2-pentylheptyl)oxy]propanoyl}oxy)methyl]propane-1,3-diyl} 3,3'-bis(2-pentylheptyl) dipropanedioate

To a mixture of dipropanedioate 3,3'-bis(2-pentylheptyl) 1,1'-{2-(hydroxymethyl)-2-[({3-oxo-3-[(2-pentylheptyl)oxy]propanoyl}oxy)methyl]propane-1,3-diyl (300 mg) and DMF (3 mL), 4-(dimethylamino)butyric acid hydrochloride (100 mg), EDCI (192 mg) and DMAP (25 mg) were added, and the mixture was stirred at room temperature for 16 hours. The mixture was poured into water, followed by extraction with ethyl acetate. The organic layer was washed with a saturated aqueous sodium hydrogencarbonate solution and saturated brine, dried over sodium sulfate, and concentrated under reduced pressure. The residue was purified by silica gel column chromatography (MeOH/DCM) to obtain the title compound (279 mg).
¹H NMR (400 MHz, CDCl₃) δ 4.22 (s, 6H), 4.13 (s, 2H), 4.05 (d, J=5.8 Hz, 6H), 3.41 (s, 6H), 2.38 (m, 4H), 2.28 (s, 6H), 1.82 (quin, J=7.3 Hz, 2H), 1.65 (br s, 3H), 1.36 - 1.21 (m, 48H), 0.89 (t, J=6.8 Hz, 18H).
MS: [M+H]⁺ 1012.7.

### [Example 121]

### 1,1'-[2-({[4-(Dimethylamino)butanoyl]oxy}methyl)-2-{[(3-pentyloctanoyl)oxy]methyl}propane-1,3-diyl] 3,3'-dihexyl bis(hexylpropanedioate)

### A) 5-Hexyl-2,2-dimethyl-1,3-dioxane-4,6-dione

To a mixture of 2,2-dimethyl-1,3-dioxane-4,6-dione (1 g) and THF (15 mL), a 60% sodium hydride (305 mg) was added at 0°C over 30 minutes. After stirring for 30 minutes, 1-iodohexane (1.47 g) was added dropwise, and the mixture was stirred at room temperature for 14 hours. An aqueous ammonium chloride solution was added to the mixture, followed by extraction with ethyl acetate. The organic layer was washed with saturated brine, dried over sodium sulfate, and concentrated under reduced pressure. The residue was purified by silica gel column chromatography (ethyl acetate/petroleum ether) to obtain the title compound (610 mg).
¹H NMR (400 MHz, CDCl₃) δ 3.51 (t, J=5.0 Hz, 1H), 2.17 - 2.09 (m, 2H), 1.79 (d, J=9.4 Hz, 6H), 1.51 - 1.29 (m, 8H), 0.90 (t, J=6.7 Hz, 3H).

### B) 2-[(Hexyloxy)carbonyl]octanoic acid

To a mixture of 5-hexyl-2,2-dimethyl-1,3-dioxane-4,6-dione (500 mg) and toluene (15 mL), 1-hexanol (246 mg) was added, and the mixture was stirred at 120°C for 15 hours. The mixture was concentrated under reduced pressure, and the residue was poured into water, followed by extraction with petroleum ether. The organic layer was dried over sodium sulfate and concentrated under reduced pressure. The residue was purified by silica gel column chromatography (ethyl acetate/petroleum ether) to obtain the title compound (310 mg).
¹H NMR (400 MHz, CDCl₃) δ 4.18 (t, J=6.8 Hz, 2H), 3.40 (t, J=7.4 Hz, 1H), 2.00 - 1.87 (m, 2H), 1.73 - 1.62 (m, 2H), 1.41 - 1.27 (m, 14H), 0.99 - 0.85 (m, 6H).

### C) Bis(hexylpropanedioic acid) 3,3'-dihexyl 1,1'-[2-({[tert-butyl(diphenyl)silyl]oxy}methyl)-2-{[(3-pentyloctanoyl)oxy]methyl}propane-1,3-diyl]

To a mixture of 2-({[tert-butyl(diphenyl)silyl]oxy}methyl)-3-hydroxy-2-(hydroxymethyl)propyl 3-pentyloctanoate (1 g), 2-[(hexyloxy)carbonyl]octanoic acid (1.1 g) and DCM (20 mL), DCC (1.1 g) was added, and the mixture was stirred at room temperature for 15 hours. Water was added to the mixture, followed by extraction with ethyl acetate. The organic layer was washed with saturated brine, dried over sodium sulfate, and concentrated under reduced pressure. The residue was purified by silica gel column chromatography (ethyl acetate/petroleum ether) to obtain the title compound (1.1 g).
¹H NMR (400 MHz, CDCl₃) δ 7.64 - 7.62 (m, 4H), 7.50 - 7.37 (m, 6H), 4.24 - 4.04 (m, 10H), 3.65 (s, 2H), 3.31 -3.27 (m, 2H), 2.20 - 2.18 (m, 2H), 1.95 - 1.77 (m, 5H), 1.65 - 1.58 (m, 4H), 1.33 - 1.22 (m, 44H), 1.06 (s, 9H), 0.96 - 0.84 (m, 18H).

### D) Bis(hexylpropanedioic acid) 3,3'-[2-(hydroxymethyl)-2-{[(3-pentyloctanoyl)oxy]methyl}propane-1,3-diyl] 1,1'-dihexyl

To a mixture of bis(hexylpropanedioic acid) 3,3'-dihexyl 1,1'-[2-({[tert-butyl(diphenyl)silyl]oxy]methyl)-2-{[(3-pentyloctanoyl)oxy]methyl}propane-1,3-diyl] (550 mg) and THF (15 mL), acetic acid (61 mg) and a THF solution of TBAF (1 M, 1.2 mL) were added at room temperature, and the mixture was stirred for 12 hours. Water was added to the mixture, followed by extraction with ethyl acetate. The organic layer was washed with saturated brine, dried over sodium sulfate, and concentrated under reduced pressure. The residue was purified by silica gel column chromatography (ethyl acetate/petroleum ether) to obtain the title compound (401 mg).
¹H NMR (400 MHz, CDCl₃) δ 4.29 - 4.21 (m, 2H), 4.18 - 4.09 (m, 8H), 3.54 (d, J=6.0 Hz, 2H), 3.38 (t, J=7.4 Hz, 2H), 2.62 - 2.51 (m, 1H), 2.28 (d, J=6.8 Hz, 2H), 1.94 - 1.83 (m, 5H), 1.69 - 1.61 (m, 4H), 1.39 - 1.24 (m, 44H), 0.99 - 0.83 (m, 18H).

### E) 1,1'-[2-({[4-(Dimethylamino)butanoyl]oxy}methyl)-2-{[(3-pentyloctanoyl)oxy]methyl}propane-1,3-diyl] 3,3'-dihexyl bis(hexylpropanedioate)

To a mixture of 4-(4-(dimethylamino))butyric acid hydrochloride (64 mg), bis(hexylpropanedioic acid) 3,3'-[2-(hydroxymethyl)-2-{[(3-pentyloctanoyl)oxy]methyl}propane-1,3-diyl] 1,1'-dihexyl (160 mg) and DMF (5 mL), EDCI (91 mg) and DMAP (12 mg) were added at room temperature, and the mixture was stirred for 15 hours. Water was added to the mixture, followed by extraction with ethyl acetate. The organic layer was washed with a saturated aqueous sodium hydrogencarbonate solution and saturated brine, dried over sodium sulfate, and concentrated under reduced pressure. The residue was purified by silica gel column chromatography (MeOH/DCM) to obtain the title compound (161 mg).
¹H NMR (400 MHz, CDCl₃) δ 4.26 - 4.07 (m, 12H), 3.36 (t, J=7.5 Hz, 2H), 2.41- 2.39 (m, 4H), 2.33 (s, 6H), 2.26 (d, J=6.8 Hz, 2H), 1.94 - 1.81 (m, 7H), 1.70 - 1.58 (m, 4H), 1.37 - 1.23 (m, 44H), 0.96 - 0.85 (m, 18H).
MS: [M+H]⁺ 954.7.

### [Example 132]

### 1,1'-[2-({[4-(Dimethylamino)butanoyl]oxy}methyl)-2-{[(3-pentyloctanoyl)oxy]methyl}propane-1,3-diyl] 3,3'-dimethyl bis(octylpropanedioate)

### A) Methyl octylpropanedioate tert-butyl

To a mixture of 60% sodium hydride (2.8 g) and DMF (300 mL), 60% methyl propanedioate tert-butyl (8 g) was added at 0°C. After stirring for 1 hour, 1-iodooctane (7.7 g) was added, and the mixture was stirred at room temperature for 12 hours. Water was added to the mixture, followed by extraction with ethyl acetate. The organic layer was washed with saturated brine, dried over sodium sulfate, and concentrated under reduced pressure. The residue was purified by silica gel column chromatography (ethyl acetate/petroleum ether) to obtain the title compound (2.1 g).
¹H NMR (400 MHz, CDCl₃) δ 3.70 (s, 3H), 3.26 (t, J=7.6 Hz, 1H), 1.86 (d, J=7.0 Hz, 2H), 1.52 - 1.44 (m, 9H), 1.36 - 1.25 (m, 12H), 0.93 - 0.87 (m, 3H).

### B) 2-(Methoxycarbonyl)decanoic acid

To a mixture of methyl octylpropanedioate tert-butyl (2.1 g) and DCM (20 mL), trifluoroacetic acid (4 mL) was added at room temperature. After stirring for 12 hours, the mixture was concentrated under reduced pressure. Water was added to the residue, followed by extraction with ethyl acetate. The organic layer was washed with saturated brine, dried over sodium sulfate, and concentrated under reduced pressure to obtain the title compound (6.5 g).
¹H NMR (400 MHz, CDCl₃) δ 3.70 (s, 3H), 3.33 (t, J=7.4 Hz, 1H), 1.95 - 1.77 (m, 2H), 1.31 - 1.14 (m, 12H), 0.81 (t, J=6.8 Hz, 3H) .

### C) Bis(octylpropanedioic acid) 3,3'-dimethyl 1,1'-[2-({[tert-butyl(diphenyl)silyl]oxy}methyl)-2-{[(3-pentyloctanoyl)oxy]methyl}propane-1,3-diyl]

To a mixture of 2-({[tert-butyl(diphenyl)silyl]oxy}methyl)-3-hydroxy-2-(hydroxymethyl)propyl 3-pentyloctanoate (500 mg) and DCM (10 mL), DCC (452 mg), 2-(methoxycarbonyl)decanoic acid (464 mg) and DMAP (21 mg) were added, and the mixture was stirred at room temperature for 12 hours. Water was added to the mixture. Followed by extractioon with ethyl acetate. The organic layer was washed with saturated brine, dried over sodium sulfate, and concentrated under reduced pressure. The residue was purified by silica gel column chromatography (ethyl acetate/petroleum ether) to obtain the title compound (680 mg).
¹H NMR (400 MHz, CDCl₃) δ 7.63 (dd, J=1.4, 7.8 Hz, 4H), 7.53 - 7.35 (m, 6H), 4.28 - 4.06 (m, 6H), 3.71 - 3.57 (m, 8H), 3.31 (dt, J=1.9, 7.5 Hz, 2H), 2.20 (td, J=2.3, 6.8 Hz, 2H), 1.95 - 1.75 (m, 5H), 1.34 - 1.22 (m, 40H), 1.07 (s, 9H), 0.92 - 0.87 (m, 12H).

### D) Bis(octylpropanedioic acid) 3,3'-dimethyl 1,1'-[2-(hydroxymethyl)-2-{[(3-pentyloctanoyl)oxy]methyl}propane-1,3-diyl ]

To a mixture of bis(octylpropanedioic acid) 3,3'-dimethyl 1,1'-[2-({[tert-butyl(diphenyl)silyl]oxy]methyl)-2-{[(3-pentyloctanoyl)oxy]methyl}propane-1,3-diyl] (680 mg) and THF (30 mL), acetic acid (82 mg) and a THF solution of TBAF (1 M, 1.71 mL) were added at room temperature, and the mixture was stirred for 12 hours. Water was added to the mixture, followed by extraction with ethyl acetate. The organic layer was washed with saturated brine, dried over sodium sulfate, and concentrated under reduced pressure. The residue was purified by silica gel column chromatography (ethyl acetate/petroleum ether) to obtain the title compound (430 mg).
¹H NMR (400 MHz, CDCl₃) δ 4.36 - 4.07 (m, 6H), 3.76 (s, 6H), 3.54 (s, 2H), 3.39 (t, J=7.5 Hz, 2H), 2.51 (br s, 1H), 2.28 (d, J=6.9 Hz, 2H), 2.00 - 1.80 (m, 5H), 1.36 - 1.23 (m, 40H), 0.90 (dt, J=2.3, 6.8 Hz, 12H).

### E) 1,1'-[2-({[4-(Dimethylamino)butanoyl]oxy}methyl)-2-{[(3-pentyloctanoyl)oxy]methyl}propane-1,3-diyl] 3,3'-dimethyl bis(octylpropanedioate)

To a mixture of bis(octylpropanedioic acid) 3,3'-dimethyl 1,1'-[2-(hydroxymethyl)-2-{[(3-pentyloctanoyl)oxy]methyl}propane-1,3-diyl] (210 mg) and DMF (10 mL), EDCI (160 mg), 4-(dimethylamino)butyric acid hydrochloride (94 mg) and DMAP (17 mg) were added at room temperature, and the mixture was stirred for 12 hours. Water was added to the mixture, followed by extraction with ethyl acetate. The organic layer was washed with a saturated aqueous sodium hydrogencarbonate solution and saturated brine, dried over sodium sulfate, and concentrated under reduced pressure. The residue was purified by silica gel column chromatography (MeOH/DCM) to obtain the title compound (200 mg).
¹H NMR (400 MHz, CDCl₃) δ 4.30 - 4.03 (m, 8H), 3.75 (s, 6H), 3.37 (t, J=7.5 Hz, 2H), 2.45 - 2.25 (m, 12H), 1.95 - 1.78 (m, 7H), 1.35 - 1.23 (m, 40H), 0.90 (dt, J=2.3, 6.9 Hz, 12H).
MS: [M+H]⁺ 870.6.

### [Example 173]

### N,N,N-trimethyl-4-oxo-4-{3-({3-oxo-3-[(2-pentylheptyl)oxy]propanoyl}oxy)-2,2-bis[({3-oxo-3-[(2-pentylheptyl)oxy]propanoyl}oxy)methyl]propoxy}butane-1-aminium

To a mixture of 1,1'-{2-({[4-(dimethylamino)butanoyl]oxy}methyl)-2-[({3-oxo-3-[(2-pentylheptyl)oxy]propanoyl}oxy)methyl]propane-1,3-diyl} 3,3'-bis(2-pentylheptyl) dipropanedioate (1 g) and DCM (10 mL), iodomethane (1.4 g) was added, and the mixture was stirred at room temperature for 5 hours. The mixture was concentrated under reduced pressure to obtain the title compound (1.16 g). ¹H NMR (400 MHz, CDCl₃) δ 4.21 (s, 6H), 4.13 (s, 2H), 4.03 (d, J=5.8 Hz, 6H), 3.69 - 3.60 (m, 2H), 3.44 (s, 9H), 3.40 (s, 6H), 2.55 (t, J=6.4 Hz, 2H), 2.20 - 2.06 (m, 2H), 1.70 - 1.63 (m, 3H), 1.34 - 1.22 (m, 48H), 0.88 (t, J=6.9 Hz, 18H).
MS: [M+H]⁺ 1026.9.

### [Production Example] Production example of DNA-encapsulated lipid nanoparticle

A lipid mixture containing a cationic lipid of the Examples shown in Table 2 (cationic lipid:DPPC:cholesterol:GM-020 = 60:10.6:28:1.4, molar ratio) was dissolved in 90% of EtOH and 10% of water to obtain a 15.0 mg/ml lipid solution. Luciferase (luc) DNA (manufactured by Nature Technology Corp.) was dissolved in a 10 mM MES buffer solution (pH 5.5) to obtain a 0.3 mg/ml nucleic acid solution. The obtained lipid solution and nucleic acid solution were mixed at a flow rate ratio of 3 ml/min : 6 ml/min at room temperature using NanoAssemblr (manufactured by Precision NanoSystems Inc.) to obtain a dispersion containing Luc DNA-encapsulated lipid nanoparticles. The obtained dispersion was dialyzed against water at room temperature for 1 hour and against PBS at 4°C for 23 hours using Slyde-A-Lyzer (molecular weight cutoff: 20 K, Thermo Fischer Scientific Inc.). Subsequently, the dialysate was filtered through a 0.2 µm syringe filter (Iwaki). A 60% solution of sucrose in PBS was added thereto, and the mixture was preserved as a 20% solution of sucrose in PBS at -80°C. Analysis results of particle size and rate of encapsulation of the Luc DNA-encapsulated lipid nanoparticles are also shown in Table 2.

### [Test Example 1] Test example of mouse bioluminescence imaging evaluation

The Luc DNA-encapsulated lipid nanopatricles obtained in the aforesaid Production Example were intravenously administered to the tails of ICrl:CD1 (ICR) mice (10 mL/kg i.v.). After administration of the lipid particles, luciferin (15 mg/mL) was administered to the mice (10 mL/kg i.p.), which were loaded in an IVIS (*in vivo* imaging system) apparatus (manufactured by PerkinElmer, Inc.) under isoflurane inhalation anesthesia. Luminescence images of the mice were taken ventrally (in a supine position) 15 minutes after luciferin administration. The luminescence was digitized with the software of IVIS, followed by evaluation using the value of total flux. Higher luminescence means strong expression of the protein encoded by the DNA encapsulate in the lipid particle. Measurement results are also shown in Table 2. In Table 2, higher luminescence is indicated on a scale of A, B and C in order. A represents luminescence of 3.0E+07 or more, B represents luminescence of 1.0E+0.7 or more and less than 3.0E+07, and C represents luminescence of less than 1.0E+07. High luminescence was observed for all the cationic lipids of Examples used.

**[Table 2-1]**

| Cationic lipid | Particle size (nm) | Rate of encapsulation | Luminescence |
|---|---|---|---|
| Example 12 | 89.5 | 95.7 | C |
| Example 13 | 136.5 | 96.3 | A |
| Example 14 | 145.0 | 94.7 | A |
| Example 15 | 89.5 | 95.9 | C |
| Example 16 | 98.4 | 97.0 | B |
| Example 17 | 120.7 | 96.2 | B |
| Example 18 | 138.9 | 96.6 | A |
| Example 19 | 89.8 | 95.4 | B |
| Example 20 | 104.2 | 97.2 | B |
| Example 21 | 131.4 | 96.2 | B |
| Example 28 | 137.3 | 94.9 | A |
| Example 31 | 97.9 | 97.1 | A |
| Example 33 | 145.7 | 89.9 | B |
| Example 34 | 184.3 | 52.7 | C |
| Example 35 | 95.1 | 96.1 | B |
| Example 36 | 127.9 | 95.1 | B |
| Example 37 | 124.1 | 97.0 | A |
| Example 38 | 131.3 | 95.2 | A |
| Example 39 | 135.2 | 98.5 | B |
| Example 41 | 138.0 | 80.3 | B |
| Example 42 | 114.5 | 86.2 | C |
| Example 43 | 126.7 | 96.0 | A |
| Example 44 | 132.3 | 96.3 | A |

**[Table 2-2]**

| | | | |
|---|---|---|---|
| Example 45 | 119.0 | 95.6 | A |
| Example 49 | 112.8 | 96.0 | B |
| Example 50 | 86.5 | 98.2 | B |
| Example 51 | 83.9 | 98.2 | B |
| Example 55 | 186.2 | 79.7 | C |
| Example 60 | 143.9 | 77.6 | A |
| Example 63 | 90.9 | 98.2 | C |
| Example 71 | 97.2 | 92.6 | A |
| Example 77 | 128.7 | 73.5 | B |
| Example 83 | 148.7 | 92.9 | B |
| Example 84 | 172.6 | 93.5 | B |
| Example 85 | 148.3 | 95.4 | B |
| Example 91 | 111.9 | 96.5 | B |
| Example 92 | 125.7 | 94.1 | B |
| Example 93 | 145.6 | 94.8 | B |
| Example 94 | 129.4 | 93.0 | C |
| Example 95 | 82.6 | 99.1 | B |
| Example 96 | 74.2 | 98.8 | B |
| Example 97 | 118.7 | 97.8 | A |
| Example 98 | 140.5 | 96.3 | A |
| Example 99 | 153.8 | 92.4 | A |
| Example 100 | 141.8 | 94.9 | B |
| Example 101 | 137.2 | 93.2 | A |
| Example 102 | 139.8 | 91.8 | B |

**[Table 2-3]**

| | | | |
|---|---|---|---|
| Example 115 | 109.6 | 95.7 | A |
| Example 116 | 122.0 | 97.0 | B |
| Example 117 | 161.0 | 82.0 | B |
| Example 119 | 116.5 | 96.4 | A |
| Example 120 | 77.4 | 97.8 | B |
| Example 121 | 78.7 | 97.7 | A |
| Example 122 | 152.1 | 96.0 | B |
| Example 125 | 88.4 | 98.1 | A |
| Example 129 | 144.9 | 92.6 | A |
| Example 132 | 129.2 | 96.5 | A |
| Example 133 | 133.4 | 95.3 | C |
| Example 134 | 103.0 | 94.5 | A |
| Example 135 | 80.1 | 97.1 | A |

### [Test Example 2] Evaluation of viability using Huh-7 cells

Huh-7 cells were seeded in a 96-well plate, and PBS, the Luc DNA-encapsulated lipid nanoparticles obtained in the aforesaid Production Example by using the cationic lipids of the Examples shown in Table 3, or Luc DNA-encapsulated lipid nanoparticles for control which were obtained in the same manner as in the aforesaid Production Example except that a known lipid, MC3 (DLin-MC3-DMA) or ALC-0315, was used instead of the cationic lipids of the Examples were added thereto (final concentration: 0.1 µg/mL and 0.3 µg/mL), followed by evaluation of viability (CellTiter-Fluor Cell Viability Assay, Promega Corporation). Taking the viability of dead cell group as 0% and the viability of the PBS group as 100%, the viability of each of the lipid particle groups was calculated. Higher viability indicates higher safety of the lipid particles. The measurement results are also shown in Table 3. When the cationic lipids of the Examples were used, an improvement in safety compared to the control groups of MC3 and ALC-0315 was observed.

**[Table 3]**

| Cationic lipid | Viability 0.1 µg/ml (%) | Viability 0.3 µg/ml (%) |
|---|---|---|
| MC3 | 97 | 49 |
| ALC-0315 | 89 | 68 |
| Example 13 | 112 | 105 |
| Example 18 | 110 | 106 |
| Example 28 | 113 | 110 |
| Example 37 | 111 | 98 |
| Example 39 | 113 | 94 |
| Example 41 | 111 | 101 |
| Example 45 | 108 | 103 |
| Example 51 | 104 | 78 |
| Example 93 | 105 | 101 |
| Example 115 | 110 | 100 |
| Example 119 | 95 | 95 |
| Example 121 | 99 | 98 |
| Example 132 | 95 | 94 |

### Industrial Applicability

The compound, the lipid particle or the composition of the present invention is capable of efficiently transferring a nucleic acid to various cells, tissues or organs. Thus, the compound, the lipid particle or the composition of the present invention can be utilized as a DDS technique for nucleic acid medicaments. Furthermore, the compound, the lipid particle or the composition of the present invention can also be utilized as a nucleic acid transfer reagent for research.

## Claims

1. A compound represented by the following formula (I): 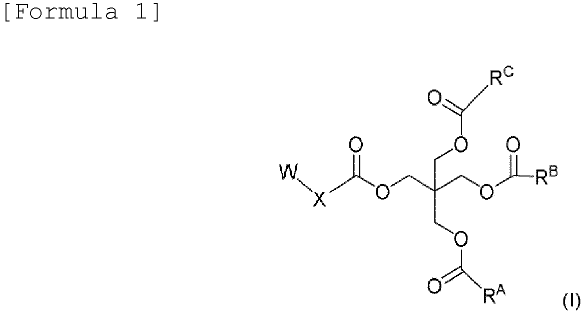 wherein
W represents -NR¹R² or -N⁺R¹¹R¹²R¹³ (Z⁻),
R¹ and R² each independently represent H or an optionally substituted C₁₋₅ alkyl group,
R¹¹, R¹² and R¹³ each independently represent an optionally substituted C₁₋₅ alkyl group,
Z⁻ represents an anion,
X represents an optionally substituted C₂₋₆ alkylene group,
R^{A} and R^{B} each independently represent an optionally substituted C₁₋₁₇ alkyl group, an optionally substituted C₃₋₁₇ alkenyl group, an optionally substituted C₁₅₋₁₇ alkadienyl group, -R³-C (O) O-R⁴ or -R³-OC(O)-R⁴,
R^{C} represents -R³-C (O) O-R⁴ or -R³-OC(O)-R⁴,
R³ represents an optionally substituted C₁₋₁₆ alkylene group, an optionally substituted C₄₋₁₆ alkenylene group or an optionally substituted C₇₋₁₆ alkadienylene group, and
R⁴ represents H, an optionally substituted C₁₋₁₈ alkyl group, an optionally substituted C₃₋₁₈ alkenyl group or an optionally substituted C₁₅₋₁₈ alkadienyl group,
or a salt thereof.

2. The compound according to claim 1 or a salt thereof, wherein the compound is represented by the following formula (II) : 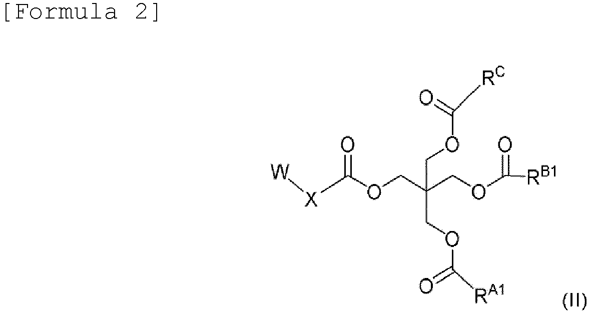 wherein
W represents -NR¹R² or -N⁺R¹¹R¹²R¹³ (Z⁻),
R¹ and R² each independently represent H or an optionally substituted C₁₋₅ alkyl group,
R¹¹, R¹² and R¹³ each independently represent an optionally substituted C₁₋₅ alkyl group,
Z⁻ represents an anion,
X represents an optionally substituted C₂₋₆ alkylene group,
R^{A1} and R^{B1} each independently represent an optionally substituted C₁₋₁₇ alkyl group, an optionally substituted C₃₋₁₇ alkenyl group, or an optionally substituted C₁₅₋₁₇ alkadienyl group,
R^{C} represents -R³-C (O) O-R⁴ or -R³-OC(O)-R⁴,
R³ represents an optionally substituted C₁₋₁₆ alkylene group, an optionally substituted C₄₋₁₆ alkenylene group or an optionally substituted C₇₋₁₆ alkadienylene group, and
R⁴ represents H, an optionally substituted C₁₋₁₈ alkyl group, an optionally substituted C₃₋₁₈ alkenyl group or an optionally substituted C₁₅₋₁₈ alkadienyl group.

3. The compound according to claim 1 or a salt thereof, wherein the compound is represented by the following formula (III): 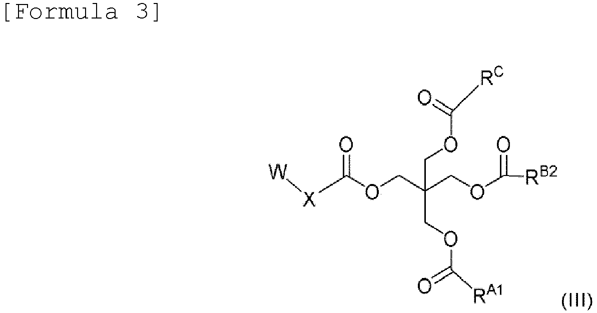 wherein
W represents -NR¹R² or -N⁺R¹¹R¹²R¹³ (Z⁻),
R¹ and R² each independently represent H or an optionally substituted C₁₋₅ alkyl group,
R¹¹, R¹² and R¹³ each independently represent an optionally substituted C₁₋₅ alkyl group,
Z⁻ represents an anion,
X represents an optionally substituted C₂₋₆ alkylene group,
R^{A1} represents an optionally substituted C₁₋₁₇ alkyl group, an optionally substituted C₃₋₁₇ alkenyl group, or an optionally substituted C₁₅₋₁₇ alkadienyl group,
R^{B2} and R^{C} each independently represent -R³-C (O) O-R⁴ or -R³-OC(O)-R⁴,
R³ represents an optionally substituted C₁₋₁₆ alkylene group, an optionally substituted C₄₋₁₆ alkenylene group or an optionally substituted C₇₋₁₆ alkadienylene group, and
R⁴ represents H, an optionally substituted C₁₋₁₈ alkyl group, an optionally substituted C₃₋₁₈ alkenyl group or an optionally substituted C₁₅₋₁₈ alkadienyl group.

4. The compound according to claim 1 or a salt thereof, wherein the compound is represented by the following formula (IV) : 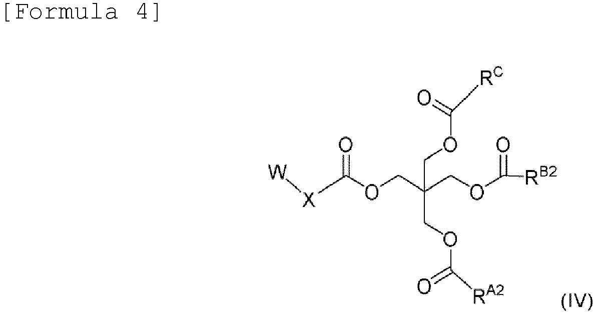 wherein
W represents -NR¹R² or -N⁺R¹¹R¹²R¹³ (Z⁻),
R¹ and R² each independently represent H or an optionally substituted C₁₋₅ alkyl group,
R¹¹, R¹² and R¹³ each independently represent an optionally substituted C₁₋₅ alkyl group,
Z⁻ represents an anion,
X represents an optionally substituted C₂₋₆ alkylene group,
R^{A2}, R^{B2} and R^{C} each independently represent -R³-C (O) O-R⁴ or -R³-OC(O)-R⁴,
R³ represents an optionally substituted C₁₋₁₆ alkylene group, an optionally substituted C₄₋₁₆ alkenylene group or an optionally substituted C₇₋₁₆ alkadienylene group, and
R⁴ represents H, an optionally substituted C₁₋₁₈ alkyl group, an optionally substituted C₃₋₁₈ alkenyl group or an optionally substituted C₁₅₋₁₈ alkadienyl group.

5. 1,1'-[2-({[4-(Dimethylamino)butanoyl]oxy}methyl)-2-{[(3-pentyloctanoyl)oxy]methyl}propane-1,3-diyl] 6,6'-diheptyl dihexanedioate.

6. 1,1'-{2-({[4-(Dimethylamino)butanoyl]oxy}methyl)-2-[({4-oxo-4-[(undecan-6-yl)oxy]butanoyl}oxy)methyl]propane-1,3-diyl} 4,4'-diundecan-6-yl dibutanedioate.

7. Decyl 2-({[4-(dimethylamino)butanoyl]oxy}methyl)-3-[(3-pentyloctanoyl)oxy]-2-{[(3-pentyloctanoyl)oxy]methyl}propyl hexanedioate.

8. 1,1'-[2-({[4-(Dimethylamino)butanoyl]oxy}methyl)-2-{[(3-pentyloctanoyl)oxy]methyl}propane-1,3-diyl] 6,6'-bis(2-propylpentyl) dihexanedioate.

9. 1,1'-{2-({[4-(Dimethylamino)butanoyl]oxy}methyl)-2-[({3-oxo-3-[(2-pentylheptyl)oxy]propanoyl}oxy)methyl]propane-1,3-diyl} 3,3'-bis(2-pentylheptyl) dipropanedioate.

10. 1,1'-[2-({[4-(Dimethylamino)butanoyl]oxy}methyl)-2-{[(3-pentyloctanoyl)oxy]methyl}propane-1,3-diyl] 3,3'-dihexyl bis(hexylpropanedioate).

11. A lipid particle comprising a compound according to claim 1 or a salt thereof.

12. A composition for nucleic acid transfer comprising a nucleic acid and a lipid particle according to claim 11.

13. The composition according to claim 12, wherein the nucleic acid is DNA or RNA.
